# EUROPEAN PATENT SPECIFICATION

(11) **EP 2 718 264 B1**
(45) Date of publication and mention of the grant of the patent: **05.08.2015**
(21) Application number: 12725741.8
(22) Date of filing: 04.06.2012
(51) Int. Cl.: C07D 213/34, C07D 213/71, C07C 311/20, C07C 311/29, A61P 11/00, A61K 31/18, A61K 31/44

(54) **BENZOCYCLOHEPTENE ACETIC ACIDS**
BENZOCYCLOHEPTEN-ESSIGSÄUREN
ACIDE ACÉTIQUE DE BENZOCYLCOHEPTÈNE

(30) Priority: 06.06.2011 US 201161493603 P
(43) Date of publication of application: 16.04.2014
(73) Proprietor: F.Hoffmann-La Roche AG, 4070 Basel (CH)
(72) Inventor: FIROOZNIA, Fariborz, Florham Park New Jersey 07932 (US); GILLESPIE, Paul, Westfield New Jersey 07090 (US); LIN, Tai-An, Pequannock New Jersey 07440 (US); SO, Sung-Sau, Verona New Jersey 07044 (US)
(74) Representative: Schirlin, Julien
(86) International application number: PCT/EP2012/060455
(87) International publication number: WO 2012/168162

(56) References cited:
- EP-A1- 0 861 836
- WO-A2-2010/018112
- WO-A2-2010/018113

## Description

The present invention relates to organic compounds useful for therapy and/or prophylaxis in a mammal of an inflammatory disease or disorder, and in particular to arylsulfonylamino-6,7,8,9-tetrahydro-5H-benzocyclohepten-1-yloxy-acetic acids, their manufacture, pharmaceutical compositions containing them and their use as CRTH2 antagonists.

Prostaglandin D₂ (PGD2) is the major prostanoid produced by activated mast cells and has been implicated in the pathogenesis of allergic diseases such as allergic asthma and atopic dermatitis. Chemoattractant Receptor-homologous molecule expressed on T-helper type cells (CRTH2) is one of the prostaglandin D₂ receptors and is expressed on the effector cells involved in allergic inflammation such as T helper type 2 (Th2) cells, eosinophils, and basophils (Nagata, K. et al. FEBS Lett. 1999, 459, 195-199). It has been shown to mediate PGD2-stimulated chemotaxis of Th2 cells, eosinophils, and basophils (Hirai, H. et al. J. Exp. Med. 2001, 193, 255-261). Moreover, CRTH2 mediates the respiratory burst and degranulation of eosinophils (Gervais, F. G. J. Allergy Clin. Immunol. 2001, 108, 982-988), induces the production of proinflammatory cytokines in Th2 cells (Xue, L. et al. J. Immunol. 2005, 175, 6531-6536), and enhances the release of histamine from basophils (Yoshimura-Uchiyama, C. et al. Clin. Exp. Allergy 2004, 34, 1283-1290). Sequence variants of the gene encoding CRTH2, which differentially influence its mRNA stability, are shown to be associated with asthma (Huang, J.-L. et al. Hum. Mol. Genet. 2004, 13, 2691-2697). Increased numbers of circulating T cells expressing CRTH2 have also been correlated with severity of atopic dermatitis (Cosmi, L. et al. Eur J Immunol 2000, 30, 2972-2979). These findings suggest that CRTH2 plays a proinflammatory role in allergic diseases. Therefore, antagonists of CRTH2 are believed to be useful for treating disorders such as asthma, allergic inflammation, chronic obstructive pulmonary disease (COPD), allergic rhinitis, and atopic dermatitis.

Monoaryl aminotetralines which are antagonists at the CRTH2 receptor are disclosed in Blanc, J.-B. et al. US 20100041760.
Bi-aryl aminotetralines which are antagonists at the CRTH2 receptor are disclosed in Blanc, J.-B. et al. US 20100041714. >

In an embodiment of the present invention, provided are compounds of general formula (I): 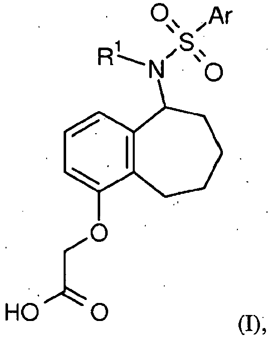 wherein:
Ar is: -phenyl, unsubstituted or mono- or bi-substituted independently with halogen, lower alkyl, -CF₃, -SO₂CH₃, alkoxy, -C(O)CH₃, unsubstituted heteroaryl or heteroaryl substituted with lower alkyl;
   - biphenyl, unsubstituted or mono- or bi-substituted independently with -OH, lower alkyl, -SCH₃ or -SO₂CH₃; or
   - pyridine, unsubstituted or substituted independently with unsubstituted phenyl or phenyl mono- or bi-substituted independently with lower alkyl, -CF₃ or - CH₂CH₂OH; and
R¹ is hydrogen or lower alkyl,
   or a pharmaceutically acceptable salt thereof.

In a further embodiment of the invention, provided is a pharmaceutical composition comprising a therapeutically effective amount of a compound according to formula (I) and a therapeutically inert carrier.

Also disclosed is a method for the treatment or prophylaxis of asthma or COPD, which method comprises the step of administering a therapeutically effective amount of a compound according to formula (I) to a patient in need thereof.

Unless otherwise indicated, the following specific terms and phrases used in the description and claims are defined as follows:
As used herein, the term "alkyl", alone or in combination with other groups, refers to a branched or straight-chain monovalent saturated aliphatic hydrocarbon radical of one to twenty carbon atoms, preferably one to sixteen carbon atoms, more preferably one to ten carbon atoms.

The term "lower alkyl", alone or in combination with other groups, refers to a branched or straight-chain alkyl radical of one to nine carbon atoms, preferably one to six carbon atoms, more preferably one to four carbon atoms. This term is further exemplified by radicals such as methyl, ethyl, *n*-propyl, isopropyl, *n*-butyl, s-butyl, isobutyl, *t*-butyl, *n*-pentyl, 3-methylbutyl, *n*-hexyl, 2-ethylbutyl and the like.

The term "cycloalkyl" refers to a monovalent mono- or polycarbocyclic radical of three to ten, preferably three to six carbon atoms. This term is further exemplified by radicals such as cyclopropyl, cyclobutyl, cyclopentyl, cyclohexyl, cycloheptyl, norbornyl, adamantyl and the like. In a preferred embodiment, the "cycloalkyl" moieties can optionally be substituted with one, two, three or four substituents, with the understanding that said substituents are not, in turn, substituted further. Each substituent can independently be, alkyl, alkoxy, halogen, amino, hydroxyl or oxygen (O=) unless otherwise specifically indicated. Examples of cycloalkyl moieties include, but are not limited to, optionally substituted cyclopropyl, optionally substituted cyclobutyl, optionally substituted cyclopentyl, optionally substituted cyclopentenyl, optionally substituted cyclohexyl, optionally substituted cyclohexylene, optionally substituted cycloheptyl, and the like or those which are specifically exemplified herein.

The term "heterocycloalkyl" denotes a mono- or polycyclic alkyl ring, wherein one, two or three of the carbon ring atoms is replaced by a heteroatom such as N, O or S. Examples of heterocycloalkyl groups include, but are not limited to, morpholinyl, thiomorpholinyl, piperazinyl, piperidinyl, pyrrolidinyl, tetrahydropyranyl, tetrahydrofuranyl, 1,3-dioxanyl and the like. The heterocycloalkyl groups may be unsubstituted or substituted and attachment may be through their carbon frame or through their heteroatom(s) where appropriate, with the understanding that said substituents are not, in turn, substituted further.

The term "aryl" refers to an aromatic mono- or polycarbocyclic radical of 6 to 12 carbon atoms having at least one aromatic ring. Examples of such groups include, but are not limited to, phenyl, naphthyl, 1,2,3,4-tetrahydronaphthalene, 1,2-dihydronaphthalene, indanyl, 1H-indenyl and the like.

The term "heteroaryl," refers to an aromatic mono- or polycyclic radical of 5 to 12 atoms having at least one aromatic ring containing one, two, or three ring heteroatoms selected from N, O, and S, with the remaining ring atoms being C. Examples of such groups include, but are not limited to, pyridine, thiazole and pyranyl.

The alkyl, lower alkyl, aryl and heteroaryl groups described above may be substituted independently with one, two, or three substituents, with the understanding that said substituents are not, in turn, substituted further. Substituents may include, for example, halogen, lower alkyl, -CF₃, -SO₂CH₃, alkoxy, -C(O)CH₃, -OH, -SCH₃ and-CH₂CH₂OH.

As used herein, the term "alkoxy" means alkyl-O-; and "alkoyl" means alkyl-CO-. Alkoxy substituent groups or alkoxy-containing substituent groups may be substituted by, for example, one or more alkyl groups, with the understanding that said substituents are not, in turn, substituted further.

As used herein, the term "halogen" means a fluorine, chlorine, bromine or iodine radical, preferably a fluorine, chlorine or bromine radical, and more preferably a fluorine or chlorine radical.

Compounds of formula I can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates or mixtures of diastereoisomeric racemates. The optically active forms can be obtained for example by resolution of the racemates, by asymmetric synthesis or asymmetric chromatography (chromatography with a chiral adsorbents or eluant). The invention embraces all of these forms.

As used herein, the term "pharmaceutically acceptable salt" means any pharmaceutically acceptable salt of the compound of formula (I). Salts may be prepared from pharmaceutically acceptable non-toxic acids and bases including inorganic and organic acids and bases. Such acids include, for example, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfuric, tartaric, oxalic, p-toluenesulfonic and the like. Particularly preferred are fumaric, hydrochloric, hydrobromic, phosphoric, succinic, sulfuric and methanesulfonic acids. Acceptable base salts include alkali metal (e.g. sodium, potassium), alkaline earth metal (e.g. calcium, magnesium) and aluminum salts.

In the practice of the method herein described, an effective amount of any one of the compounds of this invention or a combination of any of the compounds of this invention or a pharmaceutically acceptable salt thereof, is administered via any of the usual and acceptable methods known in the art, either singly or in combination. The compounds or compositions can thus be administered orally (e.g., buccal cavity), sublingually, parenterally (e.g., intramuscularly, intravenously, or subcutaneously), rectally (e.g., by suppositories or washings), transdermally (e.g., skin electroporation) or by inhalation (e.g., by aerosol), and in the form or solid, liquid or gaseous dosages, including tablets and suspensions. The administration can be conducted in a single unit dosage form with continuous therapy or in a single dose therapy ad libitum. The therapeutic composition can also be in the form of an oil emulsion or dispersion in conjunction with a lipophilic salt such as pamoic acid, or in the form of a biodegradable sustained-release composition for subcutaneous or intramuscular administration.

Useful pharmaceutical carriers for the preparation of the compositions hereof, can be solids, liquids or gases. Thus, the compositions can take the form of tablets, pills, capsules, suppositories, powders, enterically coated or other protected formulations (e.g. binding on ion-exchange resins or packaging in lipid-protein vesicles), sustained release formulations, solutions, suspensions, elixirs, aerosols, and the like. The carrier can be selected from the various oils including those of petroleum, animal, vegetable or synthetic origin, e.g., peanut oil, soybean oil, mineral oil, sesame oil, and the like. Water, saline, aqueous dextrose, and glycols are preferred liquid carriers, particularly (when isotonic with the blood) for injectable solutions. For example, formulations for intravenous administration comprise sterile aqueous solutions of the active ingredient(s) which are prepared by dissolving solid active ingredient(s) in water to produce an aqueous solution, and rendering the solution sterile. Suitable pharmaceutical excipients include starch, cellulose, talc, glucose, lactose, talc, gelatin, malt, rice, flour, chalk, silica, magnesium stearate, sodium stearate, glycerol monostearate, sodium chloride, dried skim milk, glycerol, propylene glycol, water, ethanol, and the like. The compositions may be subjected to conventional pharmaceutical additives such as preservatives, stabilizing agents, wetting or emulsifying agents, salts for adjusting osmotic pressure, buffers and the like. Suitable pharmaceutical carriers and their formulation are described in **Remington's Pharmaceutical Sciences** by E. W. Martin. Such compositions will, in any event, contain an effective amount of the active compound together with a suitable carrier so as to prepare the proper dosage form for proper administration to the recipient.

The dose of a compound of the present invention depends on a number of factors, such as, for example, the manner of administration, the age and the body weight of the subject, and the condition of the subject to be treated, and ultimately will be decided by the attending physician or veterinarian. Such an amount of the active compound as determined by the attending physician or veterinarian is referred to herein, and in the claims, as a "therapeutically effective amount". For example, the dose of a compound of the present invention is typically in the range of about 1 to about 1000 mg per day. Preferably, the therapeutically effective amount is in an amount of from about 1 mg to about 500 mg per day.

The present invention provides compounds having the general formula (I): 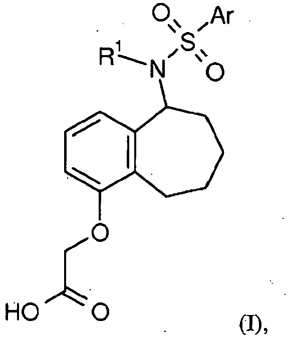 wherein:
Ar is: -phenyl, unsubstituted or mono- or bi-substituted independently with halogen, lower alkyl, -CF₃, -SO₂CH₃, alkoxy, -C(O)CH₃, unsubstituted heteroaryl or heteroaryl substituted with lower alkyl;
   - biphenyl, unsubstituted or mono- or bi-substituted independently with -OH, lower alkyl, -SCH₃ or -SO₂CH₃; or
   - pyridine, unsubstituted or substituted independently with unsubstituted phenyl or phenyl mono- or bi-substituted independently with lower alkyl, -CF₃ or - CH₂CH₂OH; and
R¹ is hydrogen or lower alkyl,
   or a pharmaceutically acceptable salt thereof.

In another embodiment of the present invention, provided is a compound of formula (I) wherein Ar is phenyl, unsubstituted or mono- or bi-substituted independently with fluorine, chlorine, bromine, -CH(CH₃)₂, -CF₃, -SO₂CH₃, -OCH3, -C(O)CH₃ or-pyridine-methyl.

In another embodiment of the present invention, provided is a compound of formula (I) wherein Ar is biphenyl, unsubstituted or mono- or bi-substituted independently with-CH₃,

-CH(CH₃)₂, -C(CH₃)₃, -OH, -SCH₃ or -SO₂CH₃.

In another embodiment of the present invention, provided is a compound of formula (I) wherein Ar is pyridine substituted independently with unsubstituted phenyl or phenyl mono- or bi-substituted independently with -CH₃, -CH(CH₃)₂, -C(CH₃)₃, -CF₃ or-CH₂CH₂OH.

In another embodiment of the present invention, provided is a compound of formula (I) wherein R¹ is hydrogen.

In another embodiment of the present invention, provided is a compound of formula (I) wherein R¹ is methyl.

Particular compounds of formula (I) include the following:
[5-(3,5-Bis-trifluoromethyl-benzenesulfonylamino)-6,7,8,9-tetrahydro-5H-benzocyclohepten-1-yloxy]-acetic acid;
[5-(3,5-Dichloro-benzenesulfonylamino)-6,7,8,9-tetrahydro-5H-benzocyclohepten-1-yloxy]-acetic acid;
[5-(Biphenyl-3-sulfonylamino)-6,7,8,9-tetrahydro-5H-benzocyclohepten-1-yloxy]-acetic acid;
[5-(Biphenyl-4-sulfonylamino)-6,7,8,9-tetrahydro-5H-benzocyclohepten-1-yloxy]-acetic acid;
[5-(3-Methanesulfonyl-benzenesulfonylamino)-6,7,8,9-tetrahydro-5H-benzocyclohepten-1-yloxy]-acetic acid;
[5-(3-Fluoro-5-trifluoromethyl-benzenesulfonylamino)-6,7,8,9-tetrahydro-5H-benzocyclohepten-1-yloxy]-acetic acid;
{5-[(3-Fluoro-5-trifluoromethyl-benzenesulfonyl)-methyl-amino]-6,7,8,9-tetrahydro-5H-benzocyclohepten-1-yloxy}-acetic acid;
[5-(3-Bromo-5-trifluoromethyl-benzenesulfonylamino)-6,7,8,9-tetrahydro-5H-benzocyclohepten-1-yloxy]-acetic acid;
{5-[(3-Bromo-5-trifluoromethyl-benzenesulfonyl)-methyl-amino]-6,7,8,9-tetrahydro-5H-benzocyclohepten-1-yloxy}-acetic acid;
[5-(3,5-Bis-methanesulfonyl-benzenesulfonylamino)-6,7,8,9-tetrahydro-5H-benzocyclohepten-1-yloxy]-acetic acid;
{5-[(3,5-Bis-methanesulfonyl-benzenesulfonyl)-methyl-amino]-6,7,8,9-tetrahydro-5H-benzocyclohepten-1-yloxy}-acetic acid;
{5-[(Biphenyl-4-sulfonyl)-methyl-amino]-6,7,8,9-tetrahydro-5H-benzocyclohepten-1-yloxy}-acetic acid;
[5-(3-Methoxy-5-trifluoromethyl-benzenesulfonylamino)-6,7,8,9-tetrahydro-5H-benzocyclohepten-1-yloxy]-acetic acid;
{5-[(3-Methoxy-5-trifluoromethyl-benzenesulfonyl)-methyl-amino]-6,7,8,9-tetrahydro-5H-benzocyclohepten-1-yloxy}-acetic acid;
[5-(3-Acetyl-5-trifluoromethyl-benzenesulfonylamino)-6,7,8,9-tetrahydro-5H-benzocyclohepten-1-yloxy]-acetic acid;
{5-[(3-Acetyl-5-trifluoromethyl-benzenesulfonyl)-methyl-amino]-6,7,8,9-tetrahydro-5H-benzocyclohepten-1-yloxy}-acetic acid;
[5-(3-Methanesulfonyl-5-trifluoromethyl-benzenesulfonylamino)-6,7,8,9-tetrahydro-5H-benzocyclohepten-1-yloxy]-acetic acid;
{5-[(3-Methanesulfonyl-5-trifluoromethyl-benzenesulfonyl)-methyl-amino)-6,7,8,9-tetrahydro-5H-benzocyclohepten-1-yloxy}-acetic acid;
[5-(3'-Isopropyl-biphenyl-4-sulfonylamino)-6,7,8,9-tetrahydro-5H-benzocyclohepten-1-yloxy]-acetic acid;
{5-[(3'-Isopropyl-biphenyl-4-sulfonyl)-methyl-amino]-6,7,8,9-tetrahydro-5H-benzocyclohepten-1-yloxy}-acetic acid;
[5-(3'-tert-Butyl-5'-methyl-biphenyl-4-sulfonylamino)-6,7,8,9-tetrahydro-5H-benzocyclohepten-1-yloxy]-acetic acid;
{5-[(3'-tert-Butyl-5'-methyl-biphenyl-4-sulfonyl)-methyl-amino]-6,7,8,9-tetrahydno-5H-benzocyclohepten-1-yloxy}-acetic acid;
[5-(4'-Hydroxy-biphenyl-4-sulfonylamino)-6,7,8,9-tetrahydro-5H-benzocyclohepten-1-yloxy]-acetic acid;
{5-[(4'-Hydroxy-biphenyl-4-sulfonyl)-methyl-amino]-6,7,8,9-tetrahydro-5H-benzocyclohepten-1-yloxy}-acetic acid;
{5-[4-(5-Methyl-pyridin-3-yl)-benzenesulfonylamino]-6,7,8,9-tetrahydro-5H-benzocyclohepten-1-yloxy}-acetic acid;
(5-{Methyl-[4-(5-methyl-pyridin-3-yl)-benzenesulfonyl]-amino}-6,7,8,9-tetrahydro-5H-benzocyclohepten-1-yloxy)-acetic acid;
[5-(3'-Methylsulfanyl-biphenyl-4-sulfonylamino)-6,7,8,9-tetrahydro-5H-benzocyclohepten-1-yloxy]-acetic acid;
{5-[Methyl-(3'-methylsulfanyl-biphenyl-4-sulfonyl)-amino]-6,7,8,9-tetrahydro-5H-benzocyclohepten-1-yloxy}-acetic acid;
[5-(3'-Methanesulfonyl-biphenyl-4-sulfonylamino)-6,7,8,9-tetrahydro-5H-benzocyclohepten-1-yloxy]-acetic acid;
{5-[(3'-Methanesulfonyl-biphenyl-4-sulfonyl)-methyl-amino]-6,7,8,9-tetrahydro-5H-benzocyclohepten-1-yloxy} -acetic acid;
{5-[5-(3-Isopropyl-phenyl)-pyridine-2-sulfonylamino]-6,7,8,9-tetrahydro-5H-benzocyclohepten-1-yloxy}-acetic acid;
(5-{[5-(3-Isopropyl-phenyl)-pyridine-2-sulfonyl]-methyl-amino}-6,7,8,9-tetrahydro-5H-benzocyclohepten-1-yloxy)-acetic acid;
{5-[5-(3-Trifluoromethyl-phenyl)-pyridine-2-sulfonylamino]-6,7,8,9-tetrahydro-5H-benzocyclohepten-1-yloxy}-acetic acid;
(5-{Methyl-[5-(3-trifluoromethyl-phenyl)-pyridine-2-sulfonyl]-amino}-6,7,8,9-tetrahydro-5H-benzocyclohepten-1-yloxy)-acetic acid;
{5-[5-(3-tert-Butyl-5-methyl-phenyl)-pyridine-2-sulfonylamino]-6,7,8,9-tetrahydro-5H-benzocyclohepten-1-yloxy}-acetic acid;
(5-{[5-(3-tert-Butyl-5-methyl-phenyl)-pyridine-2-sulfonyl]-methyl-amino}-6,7,8,9-tetrahydro-5H-benzocyclohepten-1-yloxy)-acetic acid;
(5-{5-[3-(2-Hydroxy-ethyl)-phenyl]-pyridine-2-sulfonylamino}-6,7,8,9-tetrahydro-5H-benzocyclohepten-1-yloxy)-acetic acid;
[5-({5-[3-(2-Hydroxy-ethyl)-phenyl]-pyridine-2-sulfonyl}-methyl-amino)-6,7,8,9-tetrahydro-5H-benzocyclohepten-1-yloxy]-acetic acid;
[5-(4'-Methyl-biphenyl-3-sulfonylamino)-6,7,8,9-tetrahydro-5H-benzocyclohepten-1-yloxy]-acetic acid;
{5-[Methyl-(4'-methyl-biphenyl-3-sulfonyl)-amino]-6,7,8,9-tetrahydro-5H-benzocyclohepten-1-yloxy}-acetic acid;
[5-(3'-Isopropyl-biphenyl-3-sulfonylamino)-6,7,8,9-tetrahydro-5H-benzocyclohepten-1-yloxy]-acetic acid;
{5-[(3'-Isopropyl-biphenyl-3-sulfonyl)-methyl-amino]-6,7,8,9-tetrahydro-5H-benzocyclohepten-1-yloxy}-acetic acid;
[5-(3-Isopropyl-5-trifluoromethyl-benzenesulfonylamino)-6,7,8,9-tetrahydro-5H-benzocyclohepten-1-yloxy]-acetic acid;
{5-[(3-Isopropyl-5-trifluoromethyl-benzenesulfonyl)-methyl-amino]-6,7,8,9-tetrahydro-5H-benzocyclohepten-1-yloxy}-acetic acid; and
[5-(3,5-Bis-trifluoromethyl-benzenesulfonylamino)-6,7,8,9-tetrahydro-5H-benzocyclohepten-1-yloxy]-acetic acid.

In another embodiment of the invention, provided is a compound of formula (I) for use as a therapeutically active substance.

In another embodiment of the invention, provided is pharmaceutical composition comprising a therapeutically effective amount of a compound of formula (I) and a therapeutically inert carrier.

In another embodiment of the invention, provided is a use of a compound according to formula (I) for the preparation of a medicament for the treatment or prophylaxis of asthma or COPD.

In another embodiment of the invention, provided is a compound according to formula (I) for the treatment or prophylaxis of asthma or COPD.

In another embodiment of the invention, provided is compound according formula (I), when manufactured according to a process below.

It will be appreciated, that the compounds of general formula I in this invention may be derivatized at functional groups to provide derivatives which are capable of conversion back to the parent compound *in vivo.* Physiologically acceptable and metabolically labile derivatives, which are capable of producing the parent compounds of general formula I *in vivo* may thus be prepared.

Compounds of the present invention can be prepared beginning with commercially available starting materials, or utilizing general synthetic techniques and procedures known to those skilled in the art. Chemicals may be purchased from companies such as for example Aldrich, Argonaut Technologies, VWR, Lancaster, Princeton, Alfa, Oakwood, TCI, Fluorochem, Apollo, Matrix, Maybridge or Meinoah. Chromatography supplies and equipment may be purchased from such companies as for example AnaLogix, Inc, Burlington, WI; Biotage AB, Charlottesville, VA; Analytical Sales and Services, Inc., Pompton Plains, NJ; Teledyne Isco, Lincoln, NE; VWR International, Bridgeport, NJ; Varian Inc., Palo Alto, CA, and Multigram II Mettler Toledo Instrument Newark, DE. Biotage, ISCO and Analogix columns are pre-packed silica gel columns used in standard chromatography. Final compounds and intermediates were named using the AutoNom2000 feature in the MDL ISIS Draw application.

Also described the administration of a therapeutically effective amount of a compound of formula I in combination or association with other drugs or active agents for the treatment of inflammatory or allergic diseases and disorders. In one embodiment, the present invention relates to a method for the treatment and/or prevention of such diseases or disorders comprising administering to a human or animal simultaneously, sequentially, or separately, a therapeutically effective amount of a compound of formula I and another drug or active agent (such as another anti-inflammatory or anti-allergic drug or agent). These other drugs or active agents may have the same, similar, or a completely different mode of action. Suitable other drugs or active agents may include, but are not limited to: Beta2-adrenergic agonists such as albuterol or salmeterol; corticosteroids such as dexamethasone or fluticasone; antihistamines such as loratidine; leukotriene antagonists such as montelukast or zafirlukast; anti-IgE antibody therapies such as omalizumab; anti-infectives such as fusidic acid (particularly for the treatment of atopic dermatitis); anti-fungals such as clotrimazole (particularly for the treatment of atopic dermatitis); immunosuppressants such as tacrolimus and pimecrolimus; other antagonists of PGD2 acting at other receptors such as DP antagonists; inhibitors of phosphodiesterase type 4 such as cilomilast; drugs that modulate cytokine production such as inhibitors of TNF-alpha converting enzyme (TACE); drugs that modulate the activity of Th2 cytokines IL-4 and IL-5 such as blocking monoclonal antibodies and soluble receptors; PPAR-gamma agonists such as rosiglitazone; and 5-lipoxygenase inhibitors such as zileuton.

Preferably, the compounds of formula I, can be prepared by the following general reaction scheme.

### General Synthesis

The compounds of the present invention can be prepared by any conventional means. Suitable processes for synthesizing these compounds are provided in the examples. Generally, compounds of formula I can be prepared according to the schemes illustrated below. For example, certain compounds of the invention may be made using the approach outlined in Scheme 1.

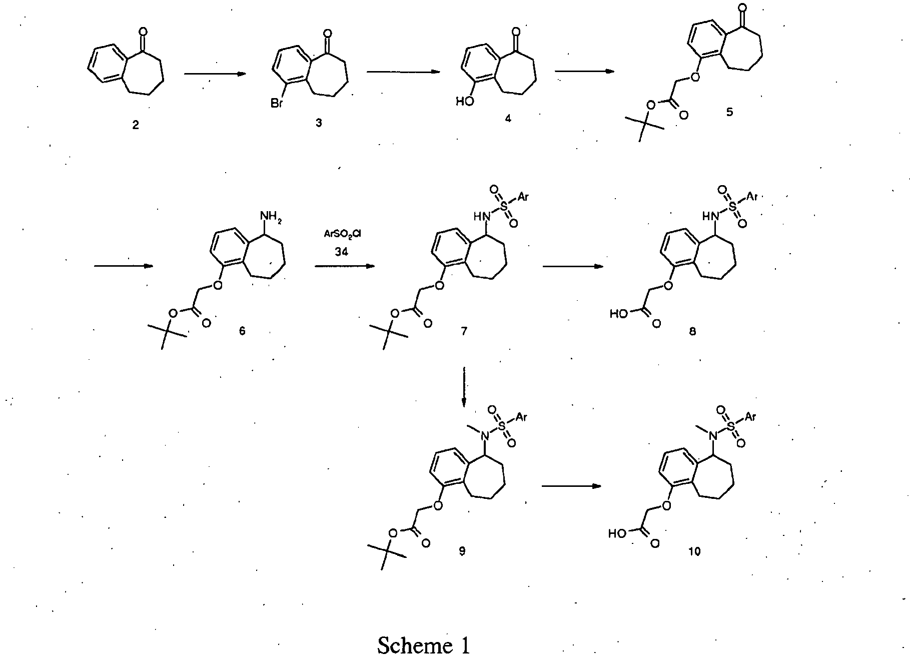

According to Scheme 1, benzosuberone, the compound of formula **2** (which may be purchased from suppliers such as Aldrich Chemical Company, Inc., 1001 West Saint Paul Avenue, Milwaukee, WI 53233, USA and TCI America, 9211 N. Harborgate Street, Portland, OR 97203, USA) is brominated to give the bromo-derivative of formula **3.** The compound of formula **3** is then subjected to a palladium-catalyzed hydroxylation reaction to give the compound of formula **4** which is alkylated to give the compound of formula **5**. Reductive amination of the ketone then gives the amine of formula **6,** which is reacted with an aryl-sulfonyl chloride of formula **34** to give the compound of formula **7.** Removal of the tert-butyl protective group then gives the compound of the invention of formula **8.** Alternatively, the compound of formula **7** may be methylated to give the compound of formula **9,** followed by removal of the tert-butyl protective group to give the compound of the invention of formula **10.**

The bromination of the compound of benzosuberone (the compound of formula **2)** is a known reaction and the reaction may be carried out using the conditions reported in the literature, or using such modifications of these conditions as are obvious to one skilled in the art of organic synthesis. For example, the reaction may be carried out by heating the compound of formula **2** with bromine in the presence of aluminum chloride at a temperature of about 75-80 °C to give a mixture of the desired compound, 1-bromo-6,7,8,9-tetrahydro-benzocyclohepten-5-one; the isomeric compound, 3-bromo-6,7,8,9-tetrahydro-benzocyclohepten-5-one; and the disubstituted compound, 1,3-dibromo-6,7,8,9-tetrahydro-benzocyclohepten-5-one. Examples of conditions suitable for carrying out this reaction may be found in the literature, for example in McCormick, K. D. et al. WO 2010042475; in Cornelius, L. A. M. and Combs, D. W. Synth. Commun. 1994, 24, 2777-2788; or else in the Examples below. The mixture of products may be separated by distillation, or preferably by supercritical fluid chromatography, as described in the experimental section below.

Alternatively, the compound of formula **3** may be prepared by the cyclization of 5-(2-bromo-phenyl)-pentanoic acid using polyphosphoric acid at a temperature of about 150 °C as described by Gruber, R. et al. Bull. Chem. Soc. France 1983, 96-104.

The conversion of the compound of formula **3** to the phenol of formula **4** may conveniently be carried out using a palladium-catalyzed hydroxylation reaction. The reaction may be carried out by heating the compound of formula **3** with potassium hydroxide in the presence of tris(dibenzylideneacetone)dipalladium(0) (which may be purchased from suppliers such as Aldrich Chemical Company, Inc., 1001 West Saint Paul Avenue, Milwaukee, WI 53233, USA; Alfa Aesar, 26 Partridge Road, Ward Hill, MA 01835, USA; and TCI America, 9211 N. Harborgate Street, Portland, OR 97203, USA) and 2-di-tert-butyl-phosphino-2',4',6'-triisopropylbiphenyl (which may be purchased from suppliers such as Aldrich Chemical Company, Inc., 1001 West Saint Paul Avenue, Milwaukee, WI 53233, USA; and Strem Chemicals, Inc., 7 Mulliken Way, Dexter Industrial Park, Newburyport, MA, USA) in a mixture of water and dioxane at a temperature between about 80 °C and about 100 °C. An example of conditions useful for this reaction may be found in the literature, in Anderson, K. et al. J. Am. Chem. Soc. 2006,128, 10694-10695.

Alternatively, the compound of formula **3** may be made using the procedure described in Ito, F. et al. JP 2006063064 where 1-methoxy-6,7,8,9-tetrahydrobenzocyclohepten-5-one may be hydrolyzed by heating in a mixture of acetic acid and 48% aqueous hydrobromic acid at reflux. The multi-step preparation of 1-methoxy-6,7,8,9-tetrahydrobenzocyclohepten-5-one is described in da Conceicao, C. M. M. et al. J. Chem. Res. 1995, 347.

The alkylation of the phenol of formula **4** may be carried out using any conventional means. For example, the reaction may conveniently be effected by treating the phenol with tert-butyl bromoacetate in the presence of an inorganic base such as Cs₂CO₃ or K₂CO₃ in an inert solvent such as DMF or CH₃CN or acetone or 2-butanone at a temperature between about 20 °C and about 80 °C. Examples of appropriate conditions may be found in the literature, for example in Blanc, J.-B. et al. US 20100041714; in Firooznia, F. et al. US 20100041713; in Isaad, J. et al. Eur. J. Org. Chem. 2009, 2748-2764; in Matsumoto, T. et al. Chem. Lett. 1988, 1399-1402; in Akahane, A. et al. US 6,355,640; or in Ohkawa, S. et al. US 6,248,766.

The reductive amination of the ketone of formula **5** may be carried out using any of a number of reactions that are familiar to one of average skill in the art of organic synthesis. For example, the reaction may be conveniently carried out by treating the ketone of formula **5** with an acid addition salt of ammonia such as ammonium acetate in the presence of a reducing agent such as sodium triacetoxyborohydride or sodium cyanoborohydride in an inert solvent such as methanol or ethanol at about room temperature. Examples of specific conditions useful for this reaction may be found in the literature, for example in Blanc, J.-B. et al. US 20100041760; in Chow, K. et al. WO 2009023757; and in Gross, M. F. et al. Bioorg. Med. Chem. Lett. 2009, 19, 3063-3066. Alternatively, the ketone of formula **5** may be converted to the corresponding oxime by heating with hydroxylamine hydrochloride in MeOH or EtOH at reflux in the presence of an organic base such as triethylamine or diisopropylethylamine or sodium acetate followed by treatment of the oxime under dissolving metal conditions such as by treatment with sodium metal in propanol at reflux to give the amine of formula **6.** Examples of specific conditions useful for this reaction may be found in the literature, for example in Bhandari, K. et al. Bioorg. Med. Chem. 2004, 12, 4189-4196. Alternatively, the ketone of formula **5** may be treated with O-methylhydroxylamine hydrochloride in MeOH at room temperature, followed by treatment of the resulting oxime ether with borane-THF complex in THF at about 60 °C to give the amine of formula **6.** Examples of specific conditions useful for this reaction may be found in the literature, for example in Sørensen, U. S. et al. US 7,737,167.

The sulfonylation of a compound of formula **6** can be effected using procedures that are well known in the field of organic synthesis. For example, the compound of formula **6** may be treated with an arylsulfonyl chloride in the presence of an appropriate base for example pyridine, which may also be used as solvent. The reaction may also be performed by using a tertiary amine as the base, in the presence of an inert solvent such as tetrahydrofuran or dichloromethane; or in aqueous solution using an alkali metal hydroxide such as sodium hydroxide as the base. The reaction is conveniently carried out at a temperature of between about room temperature and about 80 °C, preferably at about room temperature. Examples of conditions suitable for carrying out this reaction may be found in the literature, for example in Gillespie, J. et al. WO 2009147167; in Allerton, C. M. M. WO 2007057775; in Firooznia, F. et al. US 20100041713; and in Guianvarc'h, D. et al. J. Med. Chem. 2004, 47, 2365-2374.

Removal of the tert-butyl group from a compound of formula **7** to give a compound of the invention of formula **8** may be accomplished using a variety of conditions that are well known to one of average skill in the art of organic synthesis. For example, many conditions for effecting such a transformation are outlined in "Protective Groups in Organic Synthesis" [T. W. Greene and P. G. M. Wuts, 2nd Edition, John Wiley & Sons, N.Y. 1991]. For example, the compound of formula **7** may be treated with a strong organic acid (preferably trifluoroacetic acid) in an inert solvent such as a halogenated hydrocarbon (preferably dichloromethane or chloroform) at a temperature about room temperature. Exact conditions for such a reaction may be found in the literature, for example in Bartel, S. et al. US 20100029772; in Thompson, T. and Willis, P. US 20080146612; in Ford, R. et al. US 20080153850; and in Hirashima, S. et al. J. Med. Chem. 2006, 49, 4721-4736. Alternatively, the reaction may be affected by heating the compound of formula 7 in 2,2,2-trifluoroethanol or hexafluoroisopropanol at a temperature between about 100 °C and about 150 °C with or without microwave irradiation (for an example of conditions, see Choi, J. C.-C. et al. US 20090203910). Most preferably, the reaction can be accomplished by treating the compound of formula **7** with an excess of an alkali metal hydroxide such as lithium hydroxide or preferably sodium hydroxide in an inert solvent such as a mixture of tetrahydrofuran and water at about room temperature.

Methylation of a compound of formula **7** to give a compound of formula **9** may be accomplished using any conventional means. For example, the reaction may be conveniently carried out by treating the compound of formula **7** with a methylating agent such as methyl iodide or dimethyl sulfate in the presence of a base such as potassium carbonate or cesium carbonate or sodium hydride in an inert solvent such as DMF or tetrahydrofuran at a temperature between about 0 °C and about room temperature, preferably at about room temperature. Examples of conditions for such a reaction may be found in the literature, for example in Yates, C. M. et al. J. Med. Chem. 2010, 53, 4531-4544; in Blanc, J.-B. et al. US 20100041760; in Kremoser, C. et al. US 20100184809; and in Mailyan, A. K. et al. J. Org. Chem. 2009, 74, 8444-8447.

Removal of the tert-butyl group from a compound of formula **9** to give a compound of the invention of formula **10** may be accomplished using any of the conditions described above for the removal of the tert-butyl group from a compound of formula **7** to give a compound of the invention of formula **8.**

Additionally, certain compounds of the invention may be made as shown in Scheme 2

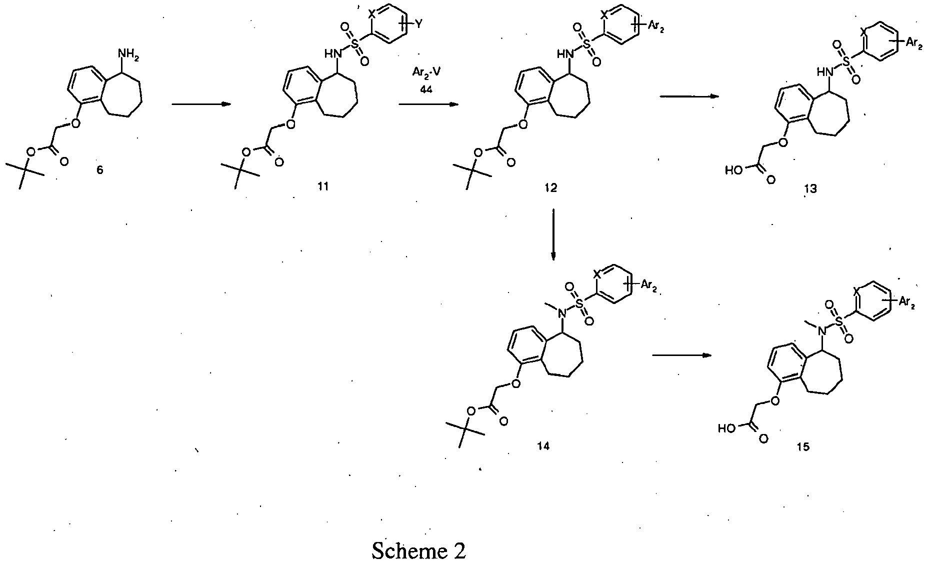

According to Scheme 2, the compound of formula **6,** which may be prepared as outlined in Scheme 1, is reacted with an aryl-sulfonyl chloride to give the compound of formula **11,** in which Y represents a group such as bromo or iodo that can act as a leaving group in a noble metal-catalyzed coupling reaction such as a Suzuki reaction, a Stille reaction, or a Negishi reaction. The compound of formula **11** then undergoes a noble metal-catalyzed reaction to give a biaryl of formula **12,** which may be hydrolyzed to give the compound of the invention of formula **13,** or methylated and then hydrolyzed to give the compound of the invention of formula **15.**

The sulfonylation of a compound of formula **6** to give a compound of formula **11** where X is N or CH and Y is a group which is commonly used in a Suzuki, Stille, or Negishi reaction, such as bromine, iodine, or trifluoromethanesulfonyl, may be effected using the conditions described above for the sulfonylation of a compound of formula **6** to give a compound of formula **7.**

The reaction of a compound of formula **11** to give a biaryl derivative of formula **12** may be accomplished using reactions that are well known to one of average skill in the art of organic synthesis. For example, the reaction may be accomplished using one of a set of reactions which use noble metal catalysis, and which include the Suzuki reaction, the Stille reaction, and the Negishi reaction. For example, the reaction can be conveniently carried out by reacting a compound of formula **11** with a compound of formula **44** where V represents B(OH)₂ or the pinacol ester thereof, in a convenient inert solvent such as a polar aprotic solvent (e.g., N,N-dimethylformamide) or an ether (e.g., dioxane) or water, or indeed in a mixture of such solvents, in the presence of a catalytic amount of a compound that can be reduced in situ to give palladium(0) (for example, palladium(II) acetate or bis(triphenylphosphine)palladium(II) chloride), in the optional additional presence of a catalytic amount of a phosphine ligand, for example tri-o-tolylphosphine or tri-tert-butylphosphine, or alternatively in the presence of a preformed complex of palladium(0) with a phosphine ligand such as bis(tri-cyclohexylphosphine)palladium, tetrakis(triphenylphosphine)-palladium(0) or [1,1-bis(diphenylphosphino)ferrocene]dichloropalladium(II)), and also in the presence of an inorganic base, for example, an alkali metal carbonate, bicarbonate or phosphate (e.g., potassium phosphate or sodium carbonate) at a temperature between about room temperature and about 100 degrees, and preferably between about room temperature and about 50 degrees. The Suzuki reaction is familiar to one of ordinary skill in the art of organic synthesis, and has been reviewed several times, notably in Miyaura, N.; Suzuki, A. Chem. Rev. 1995, 95, 2457-2483 and, more recently, in Alonso, F.; Beletskaya, I. P.; Yus, M. Tetrahedron 2008, 64, 3047-3101. Examples of specific conditions useful for Suzuki coupling may be found in many references in the literature including: Tiede, S. et al. Angew. Chem. Intl. Edn. 2010, 49, 3972-3975; Schmidt, A. and Rahimi, A. Chem. Commun. 2010, 46, 2995-2997; Lee, S. H. et al. US 20100063281; and Tobisu, M. et al. J. Org. Chem. 2010, 75, 4835-4840 (Supporting Information). Stille coupling is well known to one of average skill in the field of organic synthesis, and may be used as an alternative to the Suzuki coupling, examples of conditions for which have been provided above. Stille coupling has been reviewed, including in Farina, V. et al. Org. Reactions 1997, 50, 1-652. Examples of specific conditions that have been used for Stille coupling may be found in the literature, for example in Littke, A. F. et al. J. Am. Chem. Soc. 2002, 124, 5343-6348; in Alberati-Giani, D. et al. US 7,462,617; and in Robl, J. A. US 5,072,023.

Removal of the tert-butyl group from a compound of formula **12** to give a compound of the invention of formula **13** may be accomplished using any of the conditions described above for the removal of the tert-butyl group from a compound of formula **7** to give a compound of the invention of formula **8.**

Methylation of a compound of formula **12** to give a compound of formula **14** may be accomplished using the conditions outlined above for the conversion of a compound of formula **7** to a compound of formula **9.**

Removal of the tert-butyl group from a compound of formula **14** to give a compound of the invention of formula **15** may be accomplished using any of the conditions described above for the removal of the tert-butyl group from a compound of formula **7** to give a compound of the invention of formula **8.**

Additionally, certain compounds of the invention may be made as shown in Scheme 3.

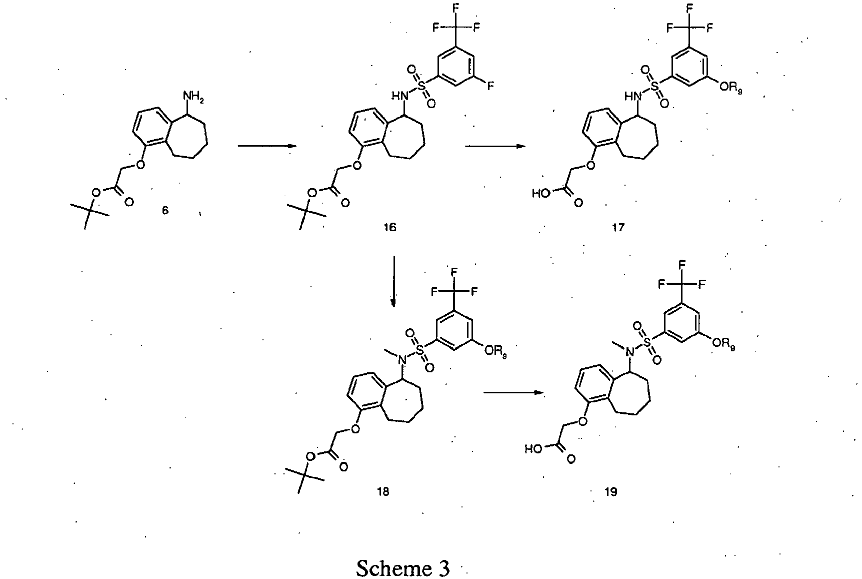

According to Scheme 3, the compound of formula **6,** which may be prepared as outlined in Scheme 1, is reacted with 3-fluoro-5-(trifluoromethyl)benzenesulfonyl chloride (which may be purchased from Alfa Aesar, 26 Parkridge Road, Ward Hill, MA 01835, USA) to give the sulfonamide of formula **16.** This compound may be reacted with the sodium salt of a lower alcohol to effect simultaneous substitution of the fluorine and hydrolysis of the tert-butyl protective group to give the compound of the invention of formula **17.** Alternatively, the sulfonamide of formula **16** may be methylated to give the compound of formula **18,** and then reacted with the sodium salt of a lower alcohol to effect simultaneous substitution of the fluorine and hydrolysis of the tert-butyl protective group to give the compound of the invention of formula **19.**

The sulfonylation of a compound of formula **6** to give a compound of formula **16** may be effected using the conditions described above for the sulfonylation of a compound of formula **6** to give a compound of formula **7.**

The conversion of a compound of formula **16** to give a compound of formula **17** may be effected by treating the compound of formula **16** with the sodium salt of a lower alcohol in an inert solvent such as the same lower alcohol or a mixture of the lower alcohol and DMF at a temperature between about the reflux temperature of the solvent and about 150 °C, with or without microwave irradiation. Depending on the conditions used, the tert-butyl protective group may be deprotected during the course of the reaction giving directly the compound of the invention of formula **17.** However, if the tert-butyl group is not cleaved during the nucleophilic substitution reaction, the it can be removed using any of the conditions described above for the removal of the tert-butyl group from a compound of formula **7** to give a compound of the invention of formula **8.**

Methylation of a compound of formula **16** to give a compound of formula **18** may be accomplished using the conditions outlined above for the conversion of a compound of formula **7** to a compound of formula **9.**

The conversion of a compound of formula **18** to give a compound of the invention of formula **19** may be effected using the conditions outlined above for the conversion of a compound of formula **16** to a compound of formula **17.**

Additionally, certain compounds of the invention may be made as shown in Scheme 4.

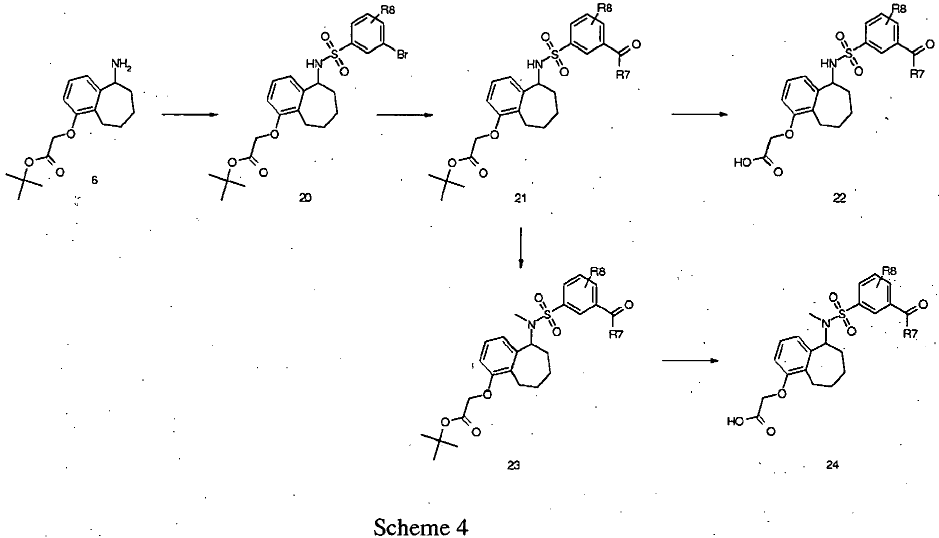

According to Scheme 4, the compound of formula **6,** which may be prepared as outlined in Scheme 1, is reacted with a 3-bromo-substituted benzenesulfonyl chloride to give the sulfonamide of formula **20.** This compound may be reacted with a tributyl(1-ethoxyvinyl)tin derivative under Stille coupling conditions to give the ketone of formula **21,** which may be hydrolyzed to give the compound of the invention of formula **22,** or methylated and then hydrolyzed to give the compound of the invention of formula **24.**

The sulfonylation of a compound of formula **6** to give a compound of formula **20** may be effected using the conditions described above for the sulfonylation of a compound of formula **6** to give a compound of formula **7.**

The conversion of a compound of formula **20** to give a compound of formula **21** can be conveniently carried out by subjecting the compound of formula **20** to a Stille coupling reaction to give a vinyl ether which is then hydrolyzed to give the ketone. Accordingly, the compound of formula **20** is treated with a tributyl(1-ethoxyvinyl)tin derivative in the presence of a catalytic amount of a compound that can be reduced in situ to give palladium(0) (for example, palladium(II) acetate or bis(triphenylphosphine)palladium(II) chloride), in the optional additional presence of a catalytic amount of a phosphine ligand, for example tri-o-tolylphosphine or tri-tert-butylphosphine or triphenylarsine, or alternatively in the presence of a preformed complex of palladium(0) with a phosphine ligand such as bis(tricyclohexylphosphine)palladium, tetrakis(ti-iphenylphosphine)-palladium(0) or [1,1-bis(diphenylphosphino)fenocene]dichloropalladium(II)), in an inert solvent such as DMF or dioxane at a temperature between about room temperature and about 100 °C, and preferably at about 100 °C. Examples of specific conditions that may be used for this reaction can be found in the literature, for example in Qian, X. et al. ACS Med. Chem. Lett. 2010, 1, 30-34; in Clawson, R. W., Jr. et al. Tetrahedron 2009, 65, 8786-8793; and in Fotouhi, N. et al. US 7,579,368. The intermediate vinyl ether may then be hydrolyzed, without or without isolation and purification, to give the desired ketone of formula **21.** The hydrolysis is conveniently carried out by treating the vinyl ether with aqueous hydrochloric acid in an inert solvent such as the solvent used for the Stille coupling reaction, or DMF or dioxane at about room temperature.

Removal of the tert-butyl group from a compound of formula **21** to give a compound of the invention of formula **22** may be accomplished using any of the conditions described above for the removal of the tert-butyl group from a compound of formula **7** to give a compound of the invention of formula **8.**

Methylation of a compound of formula **21** to give a compound of formula **23** may be accomplished using the conditions outlined above for the conversion of a compound of formula **7** to a compound of formula **9.**

Removal of the tert-butyl group from a compound of formula **23** to give a compound of the invention of formula **24** may be accomplished using any of the conditions described above for the removal of the tert-butyl group from a compound of formula **7** to give a compound of the invention of formula **8.**

Additionally, certain compounds of the invention may be made as shown in Scheme **5.**

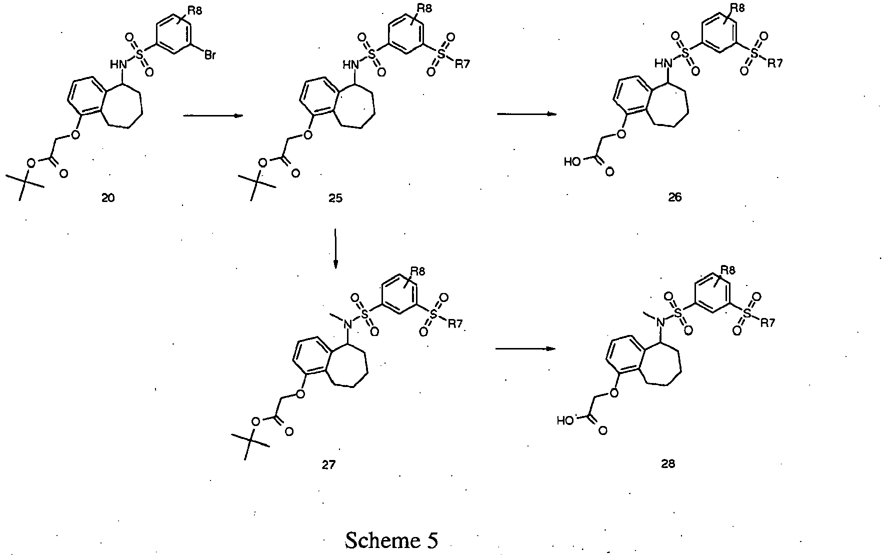

According to Scheme 5, the compound of formula **20,** which may be prepared as outlined in Scheme 4, is reacted with a lower alkylsulfinic acid to give the sulfone of formula **25.** This compound may be hydrolyzed to give the compound of the invention of formula **26,** or methylated and then hydrolyzed to give the compound of the invention of formula **28.**

The conversion of the compound of formula **20** to the compound of formula **25** may be conveniently carried out by treating the compound of formula **20** with a lower alkanesulfinate of formula R₇S(=O)OH in the presence of a copper catalyst such as copper(I) iodide in an inert solvent such as DMF or N-methylpyrrolidone at a temperature between about 100 °C and about 150 °C. Examples of specific conditions that may be used for this reaction can be found in the literature, for example in Chesworth, R. et al. WO 2009158467; in Ivachtchenko, A. V. et al. Eur. J. Med. Chem. 2010, 45, 782-789; in Qin, Z. et al. J. Med. Chem. 2007, 50, 2682-2692; and in Sturino, C. F. et al. J. Med. Chem. 2007, 50, 794-806.

Removal of the tert-butyl group from a compound of formula **25** to give a compound of the invention of formula **26** may be accomplished using any of the conditions described above for the removal of the tert-butyl group from a compound of formula **7** to give a compound of the invention of formula **8.**

Methylation of a compound of formula **25** to give a compound of formula **27** may be accomplished using the conditions outlined above for the conversion of a compound of formula **7** to a compound of formula **9.**

Removal of the tert-butyl group from a compound of formula **27** to give a compound of the invention of formula **28** may be accomplished using any of the conditions described above for the removal of the tert-butyl group from a compound of formula **7** to give a compound of the invention of formula **8.**

Additionally, certain compounds of the invention may be made as shown in Scheme 6. 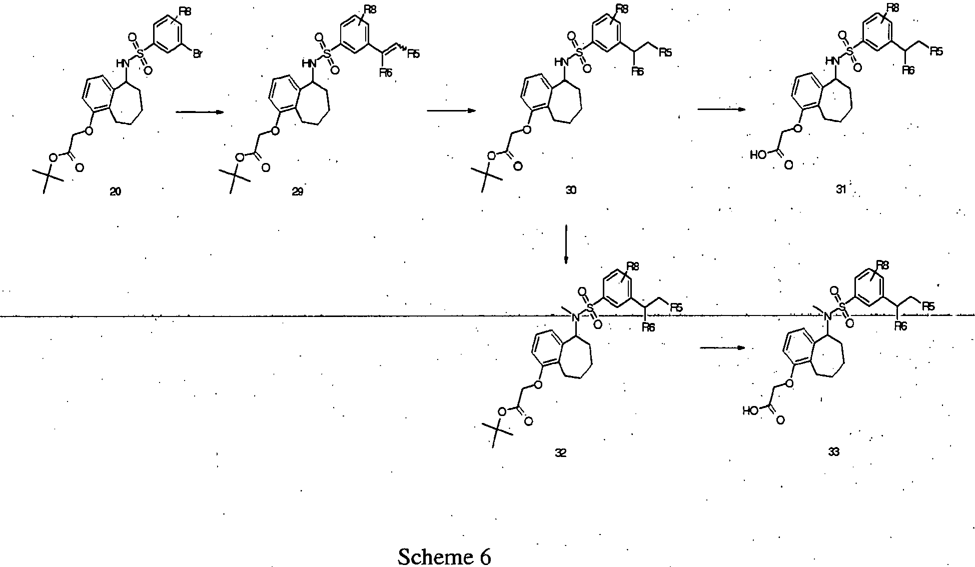 According to Scheme 6, the compound of formula **20,** which may be prepared as outlined in Scheme 4, is reacted with a vinylboronic acid to give the olefin of formula **29.** This compound may be hydrogenated to give the compound of formula **30.** Removal of the tert-butyl group then gives the compound of the invention of formula **31.** Alternatively, the compound of formula **30** may be methylated and then hydrolyzed to give the compound of the invention of formula **33.**

The conversion of the compound of formula **20** to the compound of formula **29** may be conveniently carried out by subjecting the compound of formula **20** to a Suzuki coupling reaction with a vinylboronic acid or the ester of a vinylboronic acid in a convenient inert solvent such as a polar aprotic solvent (e.g., N,N-dimethylformamide) or an ether (e.g., dioxane) or water, or indeed in a mixture of such solvents, in the presence of a catalytic amount of a compound that can be reduced in situ to give palladium(0) (for example, palladium(II) acetate or bis(triphenylphosphine)palladium(II) chloride), in the optional additional presence of a catalytic amount of a phosphine ligand, for example tri-o-tolylphosphine or tri-tert-butylphosphine, or alternatively in the presence of a preformed complex of palladium(0) with a phosphine ligand such as bis(tri-cyclohexylphosphine)palladium, tetrakis(triphenylphosphine)-palladium(0) or [1,1-bis(diphenylphosphino)ferrocene]dichloropalladium(II)), and also in the presence of an inorganic base, for example, an alkali metal carbonate, bicarbonate or phosphate (e.g., potassium phosphate or sodium carbonate) at a temperature between about room temperature and about 100 degrees, and preferably between about room temperature and about 50 degrees. The Suzuki reaction is familiar to one of ordinary skill in the art of organic synthesis, and has been reviewed several times, notably in Miyaura, N.; Suzuki, A. Chem. Rev. 1995, 95, 2457-2483 and, more recently, in Alonso, F.; Beletskaya, I. P.; Yus, M. Tetrahedron 2008, 64, 3047-3101. Examples of specific conditions useful for Suzuki coupling may be found in many references in the literature including: Chessari, G. et al. US 7,700,625; Beck, H. et al. WO 2008030520; Grove, S. J. A. et al. US 20100210680; Wang, X. et al. Org. Lett. 2009, 11, 5490-5493; and Beckett, R. P. et al. WO 2008157751.

The conversion of the compound of formula **29** to the compound of formula **30** may be carried out by treating the compound of formula **29** with hydrogen gas at ambient pressure or at a pressure of up to approximately 34.5 × 10⁴ Pa (50 pounds per square inch) in an inert solvent such as ethanol or ethyl acetate at about room temperature.

Removal of the tert-butyl group from a compound of formula **30** to give a compound of the invention of formula **31** may be accomplished using any of the conditions described above for the removal of the tert-butyl group from a compound of formula **7** to give a compound of the invention of formula **8.**

Methylation of a compound of formula **30** to give a compound of formula **32** may be accomplished using the conditions outlined above for the conversion of a compound of formula **7** to a compound of formula **9.**

Removal of the tert-butyl group from a compound of formula **32** to give a compound of the invention of formula **33** may be accomplished using any of the conditions described above for the removal of the tert-butyl group from a compound of formula **7** to give a compound of the invention of formula **8.**

Many arylsulfonyl chlorides of formula **34** useful for the conversion of a compound of formula **6** to a compound of formula **7** are commercially available; many others are known in the scientific literature and may be synthesized using procedures that are known in the art; and yet others, although not yet reported, may be made using procedures that are obvious to one of average skill in the art of organic synthesis.

For example, the following compounds are available from the suppliers indicated below.

From Aldrich Chemical Company, Inc., 1001 West Saint Paul Avenue, Milwaukee, WI 53233, USA: 2,3,4-trichlorobenzenesulfonyl chloride; 2,3,4-trifluorobenzenesulfonyl chloride; 2,3-dichlorobenzenesulfonyl chloride; 2,4,5-trichlorobenzenesulfonyl chloride; 2,5-bis(trifluoromethyl)benzenesulfonyl chloride; 2,5-dichlorobenzenesulfonyl chloride; 2,5-dimethoxybenzenesulfonyl chloride; 2-bromo-5-(trifluoromethyl)benzenesulfonyl chloride; 2-methyl-5-nitrobenzenesulfonyl chloride; 3-(trifluoromethoxy)benzenesulfonyl chloride; 3-(trifluoromethyl)benzenesulfonyl chloride; 3,4,5-trifluorobenzenesulfonyl chloride; 3,4-dichlorobenzenesulfonyl chloride; 3,4-dimethoxybenzenesulfonyl chloride; 3,5-bis(trifluoromethyl)benzenesulfonyl chloride; 3,5-dichlorobenzenesulfonyl chloride; 3,5-difluorobenzenesulfonyl chloride; 3,5-dimethylbenzenesulfonyl chloride; 3-bromo-5-(trifluoromethyl)benzenesulfonyl chloride; 3-chloro-4-fluoroberizenesulfonyl chloride; 3-chlorobenzenesulfonyl chloride; 3-cyano-4-fluorobenzenesulfonyl chloride; 3-cyanobenzenesulfonyl chloride; 3-fluoro-4-methylbenzenesulfonyl chloride; 3-fluorobenzenesulfonyl chloride; 3-nitrobenzenesulfonyl chloride; 4-biphenylsulfonyl chloride; 4-bromo-2,5-difluorobenzenesulfonyl chloride; 4-bromo-3-(trifluoromethyl)benzenesulfonyl chloride; 4-bromo-3-fluorobenzenesulfonyl chloride; 4-methyl-3-nitrobenzenesulfonyl chloride; 4-nitro-3-(trifluoromethyl)benzenesulfonyl chloride; 5-bromo-2-methoxybenzenesulfonyl chloride; m-toluenesulfonyl chloride; and pentamethylbenzenesulfonyl chloride.

From Alfa Aesar, 26 Parkridge Road, Ward Hill, MA 01835, USA: 2,3,5,6-tetramethylbenzenesulfonyl chloride; 2,4-dichloro-5-methylbenzenesulfonyl chloride; 2,5-dibromo-3,6-difluorobenzenesulfonyl chloride; 2,5-dimethylbenzenesulfonyl chloride; 2-chloro-5-(trifluoromethyl)benzenesulfonyl chloride; 2-fluoro-5-(trifluoromethyl)benzenesulphonyl chloride; 3-fluoro-5-(tiifluoromethyl)benzenesulfonyl chloride; 4-chloro-2,5-dimethylbenzenesulfonyl chloride; 4-fluoro-3-(trifluoromethyl)benzenesulfonyl chloride; 4-methoxy-3-(trifluoromethyl)benzenesulfonyl chloride; and 4'-methylbiphenyl-4-sulfonyl chloride.

From Apollo Scientific Ltd., Whitefield Road, Bredbury, Stockport, Cheshire SK6 2QR, United Kingdom: 2,4-dichloro-5-fluorobenzenesulphonyl chloride; 2-bromo-3-(trifluoromethyl)benzenesulphonyl chloride; and 4,5-dichloro-2-fluorobenzenesulphonyl chloride.

From Butt Park Ltd., Braysdown Works, Peasedown St. John, Bath, BA2 8LL, United Kingdom: 3,5-dichloro-4-methoxybenzene-1-sulfonyl chloride; 3-bromo-2-fluoro-5-(methylsulfonyl)benzenesulfonyl chloride; 3-iodobenzene-1-sulfonyl chloride; 5-(trifluoromethyl)-2-methoxybenzene-1-sulfonyl chloride; 2-methyl-5-(propane-2-sulfonyl)-benzenesulfonyl chloride; 5-ethanesulfonyl-2-methyl-benzenesulfonyl chloride; and 2-methyl-5-(propane-1-sulfonyl)-benzenesulfonyl chloride

From Chem-Impex International, Inc., 935 Dillon Drive, Wood Dale, IL 60191, USA: (3-[3,5-bis(trifluoromethyl)phenyl]phenyl)sulfonylchloride; (3-[4-(trifluoromethyl)phenyl]phenyl)sulfonylchloride; (4-[4-(trifluoromethyl)phenyl]phenyl)sulfonylchloride; [3-(3,5-dichlorophenyl)phenyl]sulphonyl chloride; 3-(3,4-dichlorophenyl)benzenesulfonyl chloride; 3',4'-dichloro[1,1'-biphenyl]-4-sulfonyl chloride; 3',5'-dichloro[1,1'-biphenyl]-4-sulfonyl chloride; 4-[3,5-bis(trifluoromethyl)phenyl]benzenesulphonyl chloride; and 4'-chloro[1,1'-biphenyl]-4-sulfonyl chloride.

From Enamine, 23A Motrosova Street, Kiev 01103, Ukraine: 4-fluoro-3-methanesulfonylbenzene-1-sulfonyl chloride; and 4-methyl-3-(trifluoromethyl)benzene-1-sulfonyl chloride.

From Maybridge, Trevillet, PL34 OHW Tintagel, Cornwall, United Kingdom: 3-(1-methyl-1H-pyrazol-3-yl)benzenesulfonyl chloride; 3-(1-methyl-1H-pyrazol-5-yl)benzenesulfonyl chloride; 3-pyrimidin-2-ylbenzenesulfonyl chloride; 4-(1-methyl-1H-pyrazol-3-yl)benzenesulfonyl chloride; and 4-pyrimidin-2-ylbenzenesulfonyl chloride.

From Oakwood Products, Inc., 1741 Old Dunbar Road, West Columbia, SC 29172, USA: 2',4'-difluoro-biphenyl-4-sulfonyl chloride; 2',4'-dimethoxy-biphenyl-3-sulfonyl chloride; 2'-4'-dimethoxy-biphenyl-4-sulfonyl chloride; 2-chloro-4,5-difluorobenzenesulfonyl chloride; 2'-chloro-biphenyl-4-sulfonyl chloride; 2'-fluoro-biphenyl-3-sulfonyl chloride; 2'-fluoro-biphenyl-4-sulfonyl chloride; 2'-methoxy-biphenyl-3-sulfonyl chloride; 2'-methoxy-biphenyl-4-sulfonyl chloride; 2'-methyl-biphenyl-3-sulfonyl chloride; 2'-methyl-biphenyl-4-sulfonyl chloride; 3-(4-fluorophenyl)benzenesulfonyl chloride; 3-(methylsulfonyl)benzenesulfonyl chloride; 3,5-bis(methylsulfonyl)benzenesulfonyl chloride; 3,5-dichloro-4-fluorobenzenesulfonyl chloride; 3'-4'-dimethoxy-biphenyl-4-sulfonyl chloride; 3'-fluoro-biphenyl-3-sulfonyl chloride; 3'-fluoro-biphenyl-4-sulfonyl chloride; 3'-methoxy-biphenyl-3-sulfonyl chloride; 3'-methoxy-biphenyl-4-sulfonyl chloride; 3'-methyl-biphenyl-3-sulfonyl chloride; 3'-methyl-biphenyl-4-sulfonyl chloride; 3-phenylbenzenesulfonyl chloride; 3-tert-butyl benzenesulfonyl chloride; 3-tert-butyl-4-methoxy-benzenesulfonyl chloride; 4'-bronio-2'-fluorobiphenyl-4-sulfonyl chloride; 4-bromo-3,5-dichlorobenzenesulfonyl chloride; 4'-bromobiphenyl-4-sulfonyl chloride; 4-chloro-2,5-difluorobenzenesulfonyl chloride; 4-chloro-3-(trifluoromethyl)benzenesulfonyl chloride; 4'-chloro-biphenyl-3-sulfonyl chloride; 4'-fluoro[1,1'-biphenyl]-4-sulfonyl chloride; 4'-methoxy-biphenyl-3-sulfonyl chloride; 4'-methoxy-biphenyl-4-sulfonyl chloride; 4'-methyl-biphenyl-3-sulfonyl chloride; 4'-nitrobiphenyl-4-sulfonyl chloride; 5-isopropyl-2-methoxy-benzenesulfonyl chloride; 5-methanesulfonyl-2-methylbenzene-1-sulfonyl chloride; and 5-tert-butyl-2-methyl-benzenesulfonyl chloride.

Sulfonyl chlorides of formula **34** can also be made by reactions that are well known in the field of organic synthesis, such as those outlined below.

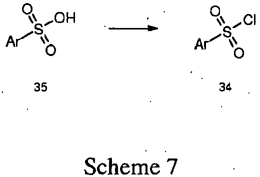

For example, a sulfonyl chloride of formula **34** can be made from a sulfonic acid of formula **35** as shown in Scheme 7. The chlorination of an arylsulfonic acid, or a salt thereof, of formula **35** can be accomplished conveniently by treating it with a chlorinating agent such as thionyl chloride or phosphorus oxychloride or phosphorus pentachloride, in the optional additional presence of a catalytic amount of N,N-dimethylformamide, at a temperature between about 0 °C and about 120 °C depending on the reactivity of the chlorinating agent. Examples of specific conditions useful for this reaction may be found in the literature, for example in Morikawa, A. et al. J. Med. Chem. 1989, 32, 42-46; in Baucherel, X. and Sheldon, R. A. US 7,019,175; in Sandanayaka, V. P. et al. US 20020099035; in Kunda, S. A. et al. US 6,140,505; and in Wu, C. J. Org. Chem. 1998, 63, 2348-2350.

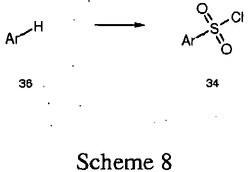

Sulfonyl chlorides of formula **34** can be made by electrophilic aromatic substitution of an aromatic compound of formula **36** as shown in Scheme 8. As is known to one of average skill in the art, this process is suitable for the preparation of arylsulfonyl chlorides with particular substitution patterns, such as for example where there is an ortho/para directing substituent in a benzene ring ortho or para to the site of introduction of the sulfonyl group. The reaction is conveniently carried out by treating the aromatic compound of formula **36** with chlorosulfonic acid in the absence of solvent and then heating the mixture at a temperature between about 70 °C and about 100 °C. Examples of specific conditions useful for this reaction may be found in the literature, for example in Arduini, A. et al. Tetrahedron Lett. 2003, 44, 5755-5757; in Derdau, V. et al. J. Org. Chem. 2003, 68, 5168-5173; in Wischnat, R. and Rudolf, J. WO 2003002546; in Lima, L. M. et al. Bioorg. Med. Chem. 2002, 10, 3067-3073; in Aboud-Gharbia, M. A. US 4,857,644; in Christidis, Y. US 4,948,827; in Pal, M. et al. J. Med. Chem. 2003, 46, 3975-3984; in Dollings, P. J. et al. US 6,103,708; and in Clark, B. P. US 6,482,824.

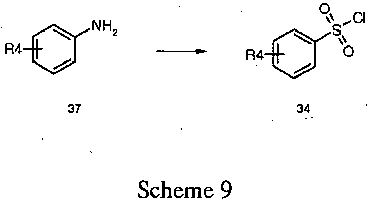

Sulfonyl chlorides of formula **34** can also be made from anilines of formula **37** by a diazotization/sulfonylation reaction sequence as shown in Scheme 9. The diazotization reaction is conveniently carried out by treating the aniline of formula **37** or an acid addition salt thereof (such as the hydrochloride salt) in aqueous solution in the presence of a mineral acid such as hydrochloric acid or sulfuric acid with an alkali metal nitrite salt such as sodium nitrite at a temperature less than 10 °C, preferably around 0 °C. The diazonium salt obtained in this way can be converted directly to the sulfonyl chloride using a variety of reagents and conditions which are known in the field of organic synthesis. Examples of suitable reagents include sulfur dioxide and copper(I) chloride or copper(II) chloride in acetic acid/water, or thionyl chloride and copper(I) chloride or copper(II) chloride in water, according to the procedure of P. J. Hogan (US 6,531,605). For example, the sulfonylation reaction can be carried out by adding the solution of the diazonium salt, prepared as described above, to a mixture of sulfur dioxide and copper(II) chloride in a suitable inert solvent, such as glacial acetic acid, at a temperature around 0 °C. Examples of specific conditions useful for this reaction may be found in the literature, for example in N. Ikemoto, N. et al. Tetrahedron 2003, 59, 1317-1325; in C. Binisti, C. et al. Eur. J. Med. Chem. 2001, 36, 809-828; in Gonzalez, M. A. and Otterbacher, E. W. US 6,433,169; in Gwaltney, S. L. et al. Bioorg. Med. Chem. Lett. 2001, 11, 871-874; in Meier, M. and Wagner, R. US 5,436,370; in Cherney, R. J. et al. J. Med. Chem. 2003, 46, 1811-1823; in Wagman, A. S. et al. J. Org. Chem. 2000, 65, 9103-9113.

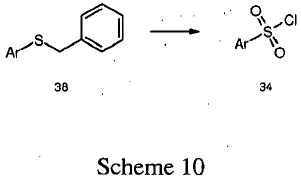

A sulfonyl chloride of formula **34** can also be made from an aryl benzyl sulfide of formula **38** by an oxidative chlorination reaction as shown in Scheme 10. The reaction is conveniently carried out by bubbling chlorine gas into a solution or suspension of the aryl benzyl sulfide of formula **38** in a suitable inert solvent such as a mixture of acetic acid and water at a temperature around room temperature. Examples of specific conditions useful for this reaction may be found in the literature, for example in Andrews, S. P. and Ladlow, M. J. Org. Chem. 2003, 68, 5525-5533; in Baker, R. H. et al. J. Am. Chem. Soc. 1946, 68, 2636-2639; in Hay, J. V. et al. US 4,521,241; in Howbert, J. J. and Crowell, T. A. Synthetic Common. 1990, 20, 3193-3195; in Barry, W. J. and Finar, I. L. J. Chem. Soc. 1954, 138-140; in Baum, J. C. et al. Can. J. Chem. 1990, 68, 1450-1455.

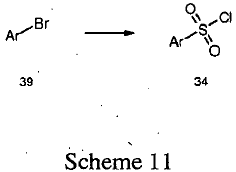

Sulfonyl chlorides of formula **34** can also be made as shown in Scheme 11 from an aryl bromide of formula **39** by metal-halogen exchange, followed by reaction of the organometallic intermediate with sulfur dioxide to give an arylsulfonate salt, followed by reaction with sulfuryl chloride to give the arylsulfonyl chloride. The reaction can be carried out by treating the aryl bromide with an organometallic reagent such as n-butyl lithium or preferably see-butyl lithium, in the optional additional presence of tetramethylethylenediamine (TMEDA) in a suitable inert solvent such as tetrahydrofuran (THF) or diethyl ether at low temperature (for example, around - 78 °C) to give the aryllithium intermediate. This can then be reacted, without isolation, with a mixture of sulfur dioxide and a solvent such as diethyl ether, again at low temperature, such as for example between about -78 °C and about -60 °C. The resulting arylsulfonate salt can then be converted to the arylsulfonyl chloride, again without isolation of the intermediate, by treatment with sulfuryl chloride at a temperature around 0 °C. Examples of specific conditions useful for this reaction may be found in the literature, for example in Chan, M. F. et al. Bioorg. Med. Chem. 1998, 6, 2301-2316; in Ewing, W. R. et al. J. Med. Chem. 1999, 42, 3557-3571; in Tamura, Y. et al. J. Med. Chem. 1998, 41, 640-649; in Raju, B. et al. Bioorg. Med. Chem. Lett. 1997, 7, 939-944; and in Hamada, T. and Yonemitsu, O. Synthesis 1986, 852-854. 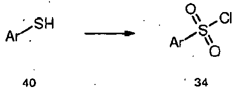 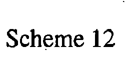 Sulfonyl chlorides of formula **34** can be made from an aryl thiol of formula **40** by oxidation using chlorine as shown in Scheme 12. For example, the reaction can be carried out by treating the aryl thiol of formula **40** with a solution of chlorine in an inert solvent such as glacial acetic acid at a temperature around 0 °C; or by treating the aryl thiol of formula **40** with N-chlorosuccinimide in a mixture of aqueous hydrochloric acid and acetonitrile at a temperature below about 20 °C. Examples of specific conditions useful for this reaction may be found in the literature, for example in Curran, W. V. et al. US 3,932,440; in Crich, D. and Sharma, I. Angew. Chem. Intl. Edn. 2009, 121, 7727-7730; in Malecha, J. W. et al. US 20070027184; in Vedejs, E. et al. J. Org. Chem. 2000, 65, 2309-2318; in Shankar, R. B. US 4,937,350; and in Lepone, G. E. US 4,454,135. One example of an aryl thiol of formula **40** that is particularly useful for the preparation of certain compounds of the invention is 5-bromo-pyridine-2-thiol. This compound is commercially available from a number of different vendors including Combi-Blocks Inc., 7949 Silverton Avenue, Suite 915, San Diego, CA 92126, USA; Aldrich Chemical Company, Inc., 1001 West Saint Paul Avenue, Milwaukee, WI 53233, USA; and Enamine, 23 A. Motrosova Street, Kiev 01103, Ukraine. 5-Bromo-pyridine-2-thiol may also be synthesized according to the procedure disclosed in Fuchss, T. et al. WO 2007039578 or according to the procedure disclosed in Raeth, C. Liebigs Ann. Chem. 1931, 487, 105-119.

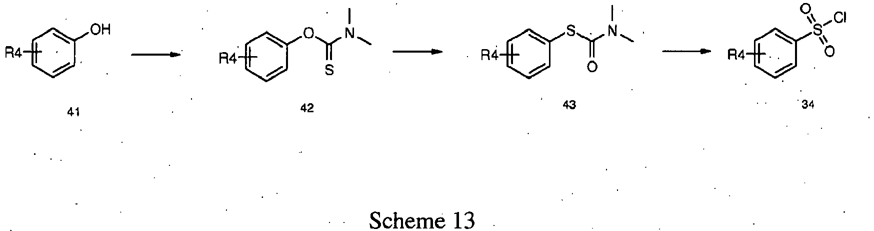

Sulfonyl chlorides of formula **34** can be made from phenols of formula **41** through a sequence of reactions outlined in Scheme 13. The phenol of formula **41** can be converted to the O-aryl-N,N'-dialkylthiocarbamate of formula **42** by reaction with an N,N'-dialkylthiocarbamoyl chloride in an inert solvent in the presence of a base. The resulting O-aryl-N,N'-dialkylthiocarbamate of formula **42** can be rearranged to the S-aryl-N,N'-dialkylthiocarbamate of formula **43** by heating neat at high temperature such as at around 250 °C. The S-aryl-N,N'-dialkylthiocarbamate of formula **43** can then be converted to the sulfonyl chloride of formula **34** by oxidation using chlorine in a suitable inert solvent such as a mixture of formic acid and water at a temperature around 0 °C. Examples of specific conditions useful for this reaction may be found in the literature, for example in Percec, V. et al. J. Org. Chem. 2001, 66, 2104-2117; in Allison, B. D. et al. WO 2008124524; and in Deng, X. et al. US 7,288,651.

The following sulfonyl chlorides of formula **34** which are particularly useful for the preparation of sulfonamides of formula 11 in Scheme 2 are commercially available: 4-iodobenzenesulfonyl chloride (available from Aldrich Chemical Company, Inc., 1001 West Saint Paul Avenue, Milwaukee, WI 53233, USA; Alfa Aesar, 26 Parkridge Road, Ward Hill, MA 01835, USA; and TCI America, 9211 N. Harborgate Street, Portland, OR 97203, USA); and 3-bromobenzenesulfonyl chloride (available from Aldrich Chemical Company, Inc., 1001 West Saint Paul Avenue, Milwaukee, WI 53233, USA; Alfa Aesar, 26 Parkridge Road, Ward Hill, MA 01835, USA; and Combi-Blocks Inc., 7949 Silverton Avenue, Suite 915, San Diego, CA 92126, USA).

Many boronic acids of formula **44** where V represents -B(OH)₂ useful for the conversion of a compound of formula **11** to a compound of formula **12** (see Scheme 2) are commercially available; many others are known in the scientific literature and may be synthesized using procedures that are known in the art; and yet others, although not yet reported, may be made using procedures that are obvious to one of average skill in the art of organic synthesis.

For example, the following compounds are available from the suppliers indicated below. These examples of commercially available compounds are provided for the purposes of illustration and are not intended to limit the invention. The suppliers indicated are not necessarily the only suppliers of these reagents, and these and other suppliers also provide other building blocks useful for the preparation of compounds of the invention.

From Aldrich Chemical Company, Inc., 1001 West Saint Paul Avenue, Milwaukee, WI 53233, USA: 2,3,4,5-tetrafluorophenylboronic acid; 2,3,4,6-tetrafluorophenylboronic acid; 2,3,5,6-tetramethylphenylboronic acid; 2,4,5-trimethylphenylboronic acid; 2,5-difluorophenylboronic acid; 2,5-dimethoxyphenylboronic acid; 2-chloro-6-fluoro-3-methylphenylboronic acid; 2-chloro-6-fluoro-5-methylphenylboronic acid; 2-methoxy-5-methylphenylboronic acid; 3-(methylthio)phenylboronic acid; 3,4-dimethoxyphenylboronic acid; 3,5-difluorophenylboronic acid; 3-acetyl-2-fluorophenylboronic acid; 3-acetylphenylboronic acid; 3-chloro-4-fluorophenylboronic acid; 3-chloro-4-methylphenylboronic acid; 3-chlorophenylboronic acid; 3-ethoxy-2-fluorophenylboronic acid; 3-fluoro-4-methoxyphenylboronic acid; 3-propoxyphenylboronic acid; 3-(trifluoromethyl)phenylboronic acid; 3-trimethylsilylphenylboronic acid; 4-ethoxy-3-fluorophenylboronic acid; 4-hydroxyphenylboronic acid; 4-methoxy-3-methylphenylboronic acid; 4-methyl-3-nitrophenylboronic acid; 4-methylphenylboronic acid; 5-chloro-2-fluoro-3-methylphenylboronic acid; 5-ethoxy-2-fluorophenylboronic acid; and 5-fluoro-2-methylphenylboronic acid.

From Alfa Aesar, 26 Parkridge Road, Ward Hill, MA 01835, USA: 2,3,5,6-tetrafluorophenylboronic acid; 2,5-dichlorophenylboronic acid; 2,5-dichloropyridine-3-boronic acid; 2-fluoro-5-hydroxymethylphenylboronic acid; 3,4-difluorophenylboronic acid; 3,5-dichlorophenylboronic acid; 3-acetamidophenylboronic acid; 3-chloro-5-fluorophenylboronic acid; 3-isopropylphenylboronic acid; 3-methylphenylboronic acid; 5-chloro-2,4-difluorophenylboronic acid; and 5-chloropyridine-3-boronic acid.

From ChemBridge Corporation, 16981 Via Tazon, Suite G, San Diego, CA 92127, USA: [3-(1-methoxyethyl)phenyl]boronic acid; [4-fluoro-3-(hydroxymethyl)phenyl]boronic acid; and 4-chloro-3-fluorophenylboronic acid.

From Combi-Blocks, Inc., 7949 Silverton Avenue, Suite 915, San Diego, CA 92126, USA: 5-chloro-2-methoxypyridine-3-boronic acid; (2-aminomethyl-5-fluoro)phenylboronic acid hydrochloride; (2-chloro-3-methylphenyl)boronic acid; (2-methyl-5-nitrophenyl)boronic acid; (3-aminomethylphenyl)boronic acid hydrochloride; (3-carbamothioyl)benzeneboronic acid; (3-chloro-2-cyanophenyl)boronic acid; (3-chloro-2-methylphenyl)boronic acid; (3-fluoro-5-methylphenyl)boronic acid; (4-aminocarbonyl-3-chloro)benzeneboronic acid; (4-chloro-3-ethoxyphenyl)boronic acid; [3-(3-hydroxypropyl)phenyl]boronic acid; 2,3,4-trichlorophenylboronic acid; 2,3,5-trichlorophenylboronic acid; 2,3,5-trifluorophenylboronic acid; 2,3,6-trifluorophenylboronic acid; 2,3-dichlorophenylboronic acid; 2,3-dichloropyridine-5-boronic acid; 2,3-difluoro-4-methoxyphenylboronic acid; 2,3-difluoro-6-methoxyphenylboronic acid; 2,3-dimethylphenylboronic acid; 2,4,5-trifluorophenylboronic acid; 2,4-dichloro-3-cyanophenylboronic acid; 2,4-dichloro-3-methoxyphenylboronic acid; 2,4-dichloro-5-methoxyphenylboronic acid; 2,5-difluoro-4-methoxyphenylboronic acid; 2,5-dimethyl-4-methoxybenzeneboronic acid; 2,6-dichloro-3-methylphenylboronic acid; 2,6-difluoro-3-methoxyphenylboronic acid; 2-chloro-3-ethoxyphenylboronic acid; 2-chloro-3-fluorophenylboronic acid; 2-chloro-3-fluoropyridine-5-boronic acid; 2-chloro-3-methoxyphenylboronic acid; 2-chloro-3-methylpyridine-5-boronic acid; 2-chloro-4-fluoro-5-methoxy-benzeneboronic acid; 2-chloro-5-boronobenzamide; 2-chloro-5-cyanophenylboronic acid; 2-chloro-5-cyanopyridine-3-boronic acid; 2-chloro-5-ethoxybenzeneboronic acid; 2-chloro-5-fluorophenylboronic acid; 2-chloro-5-fluoropyridine-3-boronic acid; 2-chloro-5-hydroxymethylphenylboronic acid; 2-chloro-5-methoxyphenylboronic acid; 2-chloro-5-methoxypyridine-3-boronic acid; 2-chloro-5-methylphenylboronic acid; 2-chloro-5-methylpyridine-3-boronic acid; 2-chloro-5-nitrophenylboronic acid; 2-chloro-6-fluoro-3-methoxyphenylboronic acid; 2-ethoxy-5-fluorophenylboronic acid; 2-ethoxy-5-methylphenylboronic acid; 2-fluoro-3-methylphenylboronic acid; 2-fluoro-3-methylpyridine-5-boronic acid; 2-fluoro-3-nitrophenylboronic acid; 2-fluoro-5-isopropylphenylboronic acid; 2-fluoro-5-methoxyphenylboronic acid; 2-fluoro-5-methylphenylboronic acid; 2-fluoro-5-methylpyridine-3-boronic acid; 2-fluoro-5-nitrophenylboronic acid; 2-methoxy-3-methylphenyl boronic acid; 2-methyl-3-nitrophenylboronic acid; 3-(1-hydroxyethyl)phenylboronic acid; 3-(2-cyanoethyl)phenylboronic acid; 3-(2-hydroxyethyl)phenylboronic acid; 3-(3-boronophenyl)acrylonitrile; 3-(aminocarbonyl)-4-fluorobenzeneboronic acid; 3-(aminocarbonyl)-5-fluorobenzeneboronic acid; 3-(aminomethyl)-2-fluorophenylboronic acid, hydrochloride salt; 3-(chloromethyl)benzeneboronic acid; 3-(difluoromethoxy)-benzeneboronic acid; 3-(hydrazinecarbonyl)benzeneboronic acid; 3-(methoxymethoxy)phenylboronic acid; 3-(methylsulfonyl)phenylboronic acid; 3-(N,N-dimethylamino)phenylboronic acid; 3-(N-methylaminocarbonyl)phenylboronic acid; 3,4,5-trichlorophenylboronic acid; 3,4-dichloro-2-methylbenzeneboronic acid; 3,4-difluoro-2-methoxyphenylboronic acid; 3,4-difluoro-5-methoxybenzeneboronic acid; 3,4-dimethylphenylboronic acid; 3,5-dichloro-2-methylphenylboronic acid; 3,5-dichloro-4-methoxyphenylboronic acid; 3,5-difluoro-2-methoxyphenylboronic acid; 3,5-difluoro-4-(hydroxymethyl)phenylboronic acid; 3,5-dimethoxyphenylboronic acid; 3,5-dimethyl-4-chlorophenylboronic acid; 3,5-dimethyl-4-methoxyphenylboronic acid; 3,5-dimethylphenylboronic acid; 3-allyloxyphenylboronic acid; 3-borono-5-chlorobenzamide; 3-boronobenzenesulfonamide; 3-chloro-2,6-difluorophenylboronic acid; 3-chloro-2-methoxyphenylboronic acid; 3-chloro-2-methoxypyridine-5-boronic acid; 3-chloro-4-cyanophenylboronic acid; 3-chloro-4-ethoxyphenylboronic acid; 3-chloro-4-methoxyphenylboronic acid; 3-chloro-5-ethoxyphenylboronic acid; 3-chloro-5-methoxyphenylboronic acid; 3-chloro-5-methylphenylboronic acid; 3-cyano-2-fluorophenylboronic acid; 3-cyano-4-fluorophenylboronic acid; 3-cyano-4-methylphenylboronic acid; 3-cyanomethoxyphenylboronic acid; 3-cyanomethylphenylboronic acid; 3-dimethylaminophenylboronic acid hydrochloride salt; 3-ethoxy-4-fluorophenylboronic acid; 3-ethoxy-5-fluorophenylboronic acid; 3-ethoxy-5-methylphenylboronic acid; 3-ethylphenylboronic acid; 3-ethylsulfinylphenylboronic acid; 3-ethylthiophenylboronic acid; 3-fluoro-2-methoxyphenylboronic acid; 3-fluoro-2-methoxypyridine-5-boronic acid; 3-fluoro-2-methylphenylboronic acid; 3-fluoro-4-(methylthio)phenylboronic acid; 3-fluoro-4-methylphenylboronic acid; 3-fluoro-5-methoxyphenylboronic acid; 3-isopropoxyphenylboronic acid; 3-isopropylphenylboronic acid; 3-mercaptophenylboronic acid; 3-methoxy-4-methylphenylboronic acid; 3-methoxy-5-methylphenyl boronic acid; 3-methoxycarbonylphenylboronic acid; 3-methoxymethylphenylboronic acid; 3-methylsulfinylphenylboronic acid; 3-nitrophenylboronic acid; (3-t-butyl-5-methylphenyl)boronic acid; 3-vinylphenylboronic acid; 4-(aminomethyl)-3-fluorophenylboronic acid, hydrochloride salt; 4,5-difluoro-2-methoxyphenylboronic acid; 4-acetyl-3-fluorophenylboronic acid; 4-carbamoyl-3-fluorophenylboronic acid; 4-chloro-2-fluoro-3-methoxyphenylboronic acid; 4-chloro-3-cyanophenylboromc acid; 4-chloro-3-ethylphenylboronic acid; 4-chloro-3-methoxyphenylboronic acid; 4-chloro-3-methylphenylboronic acid; 4-chloro-3-nitrophenylboronic acid; 4-cyano-3-fluorophenylboronic acid; 4-cyano-3-methoxyphenylboronic acid; 4-ethoxy-3-methylphenylboronic acid; 4-fluoro-2,3-dimethylphenylboronic acid; 4-fluoro-2,5-dimethylphenylboronic acid; 4-fluoro-3-methylphenylboronic acid; 4-fluoro-3-nitrophenylboronic acid; 4-hydroxymethyl-3-methylphenylboronic acid; 5-(aminomethyl)-2-fluorophenylboronic acid, hydrochloride salt; 5-(methylsulphonyl)pyridine-3-boronic acid; 5-(methylthio)pyridine-3-boronic acid; 5-acetyl-2-chlorophenylboronic acid; 5-acetyl-2-fluorophenylboronic acid; 5-carbamoyl-2-chlorophenylboronic acid; 5-carbamoyl-2-fluorobenzeneboronic acid; 5-chloro-2-cyanophenylboronic acid; 5-chloro-2-ethoxyphenylboronic acid; 5-chloro-2-ethoxypyridine-3-boronic acid; 5-chloro-2-fluoro-4-methoxyphenylboronic acid; 5-chloro-2-fluoro-4-methylphenylboronic acid; 5-chloro-2-fluoropyridine-3-boronic acid; 5-chloro-2-methoxyphenylboronic acid; 5-chloro-2-nitrophenylboronic acid; 5-chloro-4-methoxy-2-methylphenylboronic acid; 5-chloro-6-ethoxypyridine-3-boronic acid; 5-cyano-2-fluorobenzeneboronic acid; 5-cyano-2-methoxyphenylboronic acid; 5-cyano-2-methylphenylboronic acid; 5-cyano-3-pyridinyl boronic acid; 5-fluoro-2-(methylthio)phenylboronic acid; 5-fluoro-2-methoxypyridine-3-boronic acid; 5-fluoropyridine-3-boronic acid; 5-methoxypyridine-3-boronic acid; 5-methylpyridine-3-boronic acid; 5-trifluoromethyl-pyridine-3-boronic acid; methyl 5-borononicotinate; and N,N,2-trimethylaniline-4-boronic acid.

From Matrix Scientific, P.O. Box 25067, Columbia, SC 29224-5067, USA: 2-fluoro-3-methoxyphenylboronic acid; 3-(hydroxymethyl)phenylboronic acid; 3,4,5-trifluorophenylboronic acid; 3,4-dichlorophenylboronic acid; 3-aminocarbonylphenylboronic acid; 4-fluoro-3-methoxyphenylboronic acid; 5-chloro-2-methylphenylboronic acid; 5-fluoro-2-methoxyphenylboronic acid; and 5-fluoro-2-nitrobenzeneboronic acid.

From Oakwood Products, Inc., 1741 Old Dunbar Road, West Columbia, SC 29172, USA: 3-(trifluoromethyl)phenylboronic acid; 3,6-difluoro-2-methoxybenzeneboronic acid; 3-chloro-2,4-difluorobenzeneboronic acid; 3-chloro-2-fluorophenylboronic acid; 3-fluorophenylboronic acid; 3-methoxyphenylboronic acid; 5-chloro-2-fluorophenylboronic acid; 6-chloro-2,3-difluorophenylboronic acid; 6-chloro-2-fluoro-3-methoxyphenylboronic acid; and m-t-butylphenylboronic acid.

From TCI America, 9211 N. Harborgate Street, Portland, OR 97203, USA: 2,3,4-trifluorophenylboronic acid; 2,3-difluorophenylboronic acid; 2,3-dimethoxyphenylboronic acid; 2,4-dimethylphenylboronic acid; 2,5-dimethylphenylboronic acid; 2-fluoro-4-methylphenylboronic acid; 3-cyanophenylboronic acid; 3-ethoxyphenylboronic acid; 4-acetylphenylboronic acid; 4-chlorophenylboronic acid; 4-cyanophenylboronic acid; 4-ethoxyphenylboronic acid; 4-ethylphenylboronic acid; 4-(hydroxymethyl)phenylboronic acid; 4-isopropylphenylboronic acid; 4-methoxyphenylboronic acid; 4-(methylthio)phenylboronic acid; 4-(methylsulfonyl)phenylboronic acid; 4-tert-butylphenylboronic acid; 4-(trifluoromethyl)phenylboronic acid; 4-(trifluoromethoxy)phenylboronic acid.

Compounds of formula **44** where V represents -B(OH)₂, that is compounds of formula **46,** may be synthesized by procedures that are well known to one skilled in the art of organic synthesis.

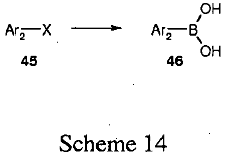

For example, a compound of this type can conveniently be synthesized according to Scheme 14 from a compound of formula **45,** in which X represents bromine or iodine, by treatment with an alkyllithium (e.g., n-butyllithium) or magnesium (to form the Grignard reagent) in a suitable inert solvent such as an ether (such as tetrahydrofuran or diethyl ether) at a temperature appropriate for the reaction (for example, at approximately -78 degrees for reaction with an alkyllithium, or at approximately room temperature for reaction with magnesium), followed by treatment with a trialkyl borate and then with dilute acid to form the compound of formula **46.** Examples of specific conditions that may be used for this reaction can be found in the literature, for example in Gimeno, N. et al. Angew. Chem. Int. Edn. 2004, 43, 5235-5238 Supporting Information; in Erickson-Miller, C. J. et al. US 20040019190; in Sundermann, B. et al. US 20020198251; in Hirano, M. et al. US 7,001,917; and in Zbruyev, A. I. et al. Tetrahedron 2007, 63, 4297-4303.

Compounds of formula **44** where V represents 4,4,5,5-tetramethyl-[1,3,2]dioxaborolane, that is compounds of formula **47,** may be synthesized by procedures that are well known to one skilled in the art of organic synthesis.

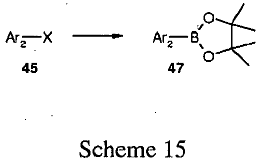

For example, a compound of this type can conveniently be synthesized according to Scheme 15 from a compound of formula **45,** in which X represents bromine or iodine or trifluoromethanesulfonate. A compound of formula **47** may be conveniently prepared according to this procedure by treating the compound of formula **45** with bis(pinacolato)diboron (which is commercially available from many vendors including Aldrich Chemical Company, Inc., 1001 West Saint Paul Avenue, Milwaukee, WI 53233, USA) in the presence of a palladium catalyst such as dichloro[1,1'-bis(diphenylphosphino)ferrocene]-palladium(II) or the dichloromethane adduct thereof in the presence of a base such as potassium acetate in an inert solvent such as 1,4-dioxane or dimethylsulfoxide or N,N-dimethylformamide at a temperature between about 80 °C and about 100 °C. The reaction may be advantageously carried out under an inert atmosphere. Examples of specific conditions that may be used for this reaction can be found in the literature, for example in Goodacre, S. C. et al. J. Med. Chem. 2006, 49, 35-38 Supporting Information; in Bouillot, A. M. J. et al. WO 2009071504; and in Ahmad, S. WO 2010104818.

A compound of formula **47** may also be conveniently prepared according to Scheme 15 by treating the compound of formula **45** with pinacolborane (which is commercially available from several vendors including Aldrich Chemical Company, Inc., 1001 West Saint Paul Avenue, Milwaukee, WI 53233, USA) in the presence of a base such as triethylamine, a palladium catalyst such as dichloro[1,1'-bis(diphenylphosphino)ferrocene]-palladium(II) or the dichloromethane adduct thereof or dichlorobis(triphenylphosphine)palladium(II), or else a mixture of a palladium catalyst such as palladium(II) acetate in the presence of a ligand such as 2-dicyclohexylphosphino-1,1'-biphenyl, in an inert solvent such as 1,4-dioxane at a temperature between about 80 °C and about 100 °C. Examples of specific conditions that may be used for this reaction can be found in the literature, for example in Baudoin, O. et al. J. Org. Chem. 2000, 65, 9268-9271; Mizojiri, R. et al. US 7,659,263; in Dodic, N. and Gellibert, F. US 20050234029; and in Wager, T. T. et al T. US 20050043354.

Many arylstannanes of formula **44** where V represents -SnMe₃ or -SnBu₃ useful for the conversion of a compound of formula **11** to a compound of formula **12** (see Scheme 2) are commercially available; many others are known in the scientific literature and may be synthesized using procedures that are known in the art; and yet others, although not yet reported, may be made using procedures that are analogous to reported procedures or obvious to one of average skill in the art of organic synthesis. For example, the following compounds are available from the suppliers indicated below.

From Aldrich Chemical Company, Inc., 1001 West Saint Paul Avenue, Milwaukee, WI 53233, USA: (4-benzyloxyphenyl)tributylstannane; 2-bromo-5-(tributylstannyl)pyridine; 2-fluoro-3-(tributylstannyl)pyridine; tributyl(4-chloro)phenylstannane; tributylphenyltin; and trimethyl(phenyl)tin.

From Alfa Aesar, 26 Parkridge Road, Ward Hill, MA 01835, USA: 2-methoxy-3-(tributylstannyl)pyridine; 2-methylthio-5-(tributylstannyl)pyridine; 2-morpholino-5-(tributylstannyl)pyridine; and 3-(tributylstannyl)pyridine.

From Maybridge, Trevillet, PL34 OHW Tintagel, Cornwall, United Kingdom: tributyl(4-fluorophenyl)stannane and tributyl[3-(trifluoromethyl)phenyl]stannane.

Compounds of formula **44** where V represents -SnMe₃ or -SnBu₃, that is compounds of formula **48**, may be synthesized by procedures that are well known to one skilled in the art of organic synthesis.

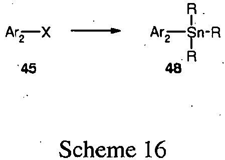

For example, a compound of this type can conveniently be synthesized according to Scheme 16 from a compound of formula **45,** in which X represents bromine or iodine. A compound of formula **47** may be conveniently prepared according to this procedure by treating the compound of formula **45** with tert-butyllithium or n-butyllithium in an inert solvent such as tetrahydrofuran or diethyl ether at -78 °C, adding trimethyltin chloride or tributyltin chloride, and allowing the reaction to proceed at room temperature. Examples of specific conditions that may be used for this reaction can be found in the literature, for example in John, V. et al. US 20090270367; in Halbert, S. M. et al. WO 2010059943; and in Pellicciari, R. et al. WO 2006013085.

A compound of formula **48** may also be conveniently prepared according to Scheme 16 by treating the compound of formula **45** with hexamethyldistannane (which is commercially available from several vendors including Aldrich Chemical Company, Inc., 1001 West Saint Paul Avenue, Milwaukee, WI 53233, USA) or hexabutyldistannane (which is commercially available from several vendors including Aldrich Chemical Company, Inc., 1001 West Saint Paul Avenue, Milwaukee, WI 53233, USA) (to give the compounds of formula **48** where R represents methyl or butyl, respectively) in the optional presence of lithium chloride, a palladium catalyst such as tetrakis(triphenylphosphine)palladium(0) (which is commercially available from several vendors including Aldrich Chemical Company, Inc., 1001 West Saint Paul Avenue, Milwaukee, WI 53233, USA) in an inert solvent such as 1,4-dioxane or toluene at a temperature about 100 °C or alternatively at a higher temperature such as at about 150 °C under microwave irradiation. Examples of specific conditions that may be used for this reaction can be found in the literature, for example in Ritter, T. et al. WO 2010059943; in Ronen, Sabrina M. et al. US 20100062465; and in Dimagno, S. WO 2010048170.

### EXAMPLES

Although certain exemplary embodiments are depicted and described herein, the compounds of the present invention can be prepared using appropriate starting materials according to the methods described generally herein and/or by methods available to one of ordinary skill in the art.

### Materials and Instrumentation In General

Intermediates and final compounds were purified by either flash chromatography and/or preparative HPLC (high performance liquid chromatography). Flash chromatography was performed using (1) the Biotage SP1™ system and the Quad 12/25 Cartridge module from Biotage AB) or (2) the ISCO CombiFlash^{®} chromatography instrument (from Teledyne Isco, Inc.); unless otherwise noted. The silica gel brand and pore size utilized were: (1) KP-SIL™ 60 Å, particle size: 40-60 micron (from Biotage AB); (2) Silica Gel CAS registry No: 63231-67-4, particle size: 47-60 micron; or (3) ZCX from Qingdao Haiyang Chemical Co., Ltd, pore size: 200-300 mesh or 300-400 mesh. Preparative HPLC was performed on a reversed phase column using an Xbridge™ Prep C₁₈ (5 m, OBD™ 30 × 100 mm) column (from Waters Corporation), a SunFire™ Prep C₁₈ (5 m, OBD™ 30 × 100 mm) column (from Waters Corporation), or a Varian Pursuit^{®} C-18 column 20 X 150 mm (from Varian, Inc.).

Mass spectrometry (MS) or high resolution mass spectrometry (HRMS) was performed using a Waters^{®} ZQ™ 4000 (from Waters Corporation), a Waters^{®} Alliance^{®} 2795-ZQ™2000 (from Waters Corporation), a Waters^{®} Quattro micro™ API (from Waters Corporation), or an MDS Sciex™ API-2000™n API (from MDS Inc.). Mass spectra data generally only indicates the parent ions unless otherwise stated. MS or HRMS data is provided for a particular intermediate or compound where indicated.

Nuclear magnetic resonance spectroscopy (NMR) was performed using a Varian^{®} Mercury300 NMR spectrometer (for the HNMR spectrum acquired at 300 MHz) and a Varian^{®} Inova400 NMR spectrometer (for the HNMR spectrum acquired at 400 MHz) both from Varian Inc. NMR data is provided for a particular intermediate or compound where indicated.

Microwave assisted reactions were carried out in a Biotage Initiator™ Sixty (or earlier models) (from Biotage AB) or by a CEM Discover^{®} model (with gas addition accessory) (from CEM Corporation).

Chiral separation was performed by supercritical fluid chromatography (SFC) using a Multigram^{®} III instrument (from Thar Technologies, Inc.).

All reactions involving air-sensitive reagents were performed under an inert atmosphere. Reagents were used as received from commercial suppliers unless otherwise noted.

### PART I: GENERAL PROCEDURES

### General Procedure 1

### Sulfonylation of (5-Amino-6,7,8,9-tetrahydro-5H-benzocyclohepten-1-yloxy)-acetic acid tert-butyl ester

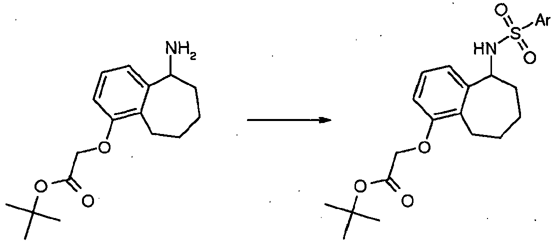

Diisopropylethylamine (1.2 equivalents) was added to a solution of (5-amino-6,7,8,9-tetrahydro-5H-benzocyclohepten-1-yloxy)-acetic acid tert-butyl ester (which may be prepared as described for Intermediate 1.04; 1 equivalent) and the sulfonyl chloride (1.2 equivalents) in anhydrous CH₂Cl₂ at 0 °C. The mixture was stirred at room temperature for 12 h, and then concentrated under reduced pressure. Water (5 mL) was added and the mixture was extracted with ethyl acetate (3 x 10 mL). The organic extracts were combined, washed with water (2 x 3 mL), dried over anhydrous Na₂SO₄, filtered, and evaporated. The residue was purified by silica gel chromatography, using 10-30% EtOAc/hexanes as eluent, to give the product.

### General Procedure 2

### Sulfonylation of (5-Amino-6,7,8,9-tetrahydro-5H-benzocyclohepten-1-yloxy)-acetic acid tert-butyl ester

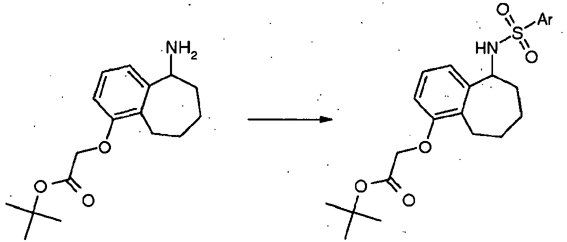

Diisopropylethylamine (2 equivalents) and the sulfonyl chloride (1 equivalent) were added to a solution of (5-amino-6,7,8,9-tetrahydro-5H-benzocyclohepten-1-yloxy)-acetic acid tert-butyl ester (which may be prepared as described for Intermediate 1.04; 1 equivalent) in THF (10 mL/mmol) at room temperature under nitrogen. The mixture was stirred at room temperature for 3 h, and then concentrated under reduced pressure. The residue was purified by silica gel chromatography, using 15-25% EtOAc/hexanes as eluent, to give the product.

### General Procedure 3

### Methylation of 5-Arylsulfonylamino-6,7,8,9-tetrahydro-5H-benzocyclohepten-1-yloxy]-acetic Acid tert-Butyl Ester

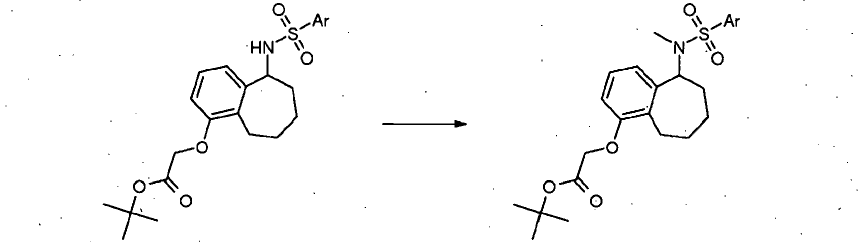

Methyl iodide (2 equivalents) was added to a mixture of the 5-arylsulfonylamino-6,7,8,9-tetrahydro-5H-benzocyclohepten-1-yloxy]-acetic acid tert-butyl ester (1 equivalent) and K₂CO₃ (2 equivalents) in DMF (20 mL/mmol) at room temperature under nitrogen. The mixture was stirred overnight at room temperature, then diluted with water and extracted with ethyl acetate (3 x 50 mL/mmol). The organic extracts were combined, dried over anhydrous Na₂SO₄, filtered, and evaporated to give the [5-(arylsulfonyl-methyl-amino)-6,7,8,9-tetrahydro-5H-benzocyclohepten-1-yloxy]-acetic acid tert-butyl ester which was used directly in the next step without further purification.

### General Procedure 4

### Suzuki Reaction

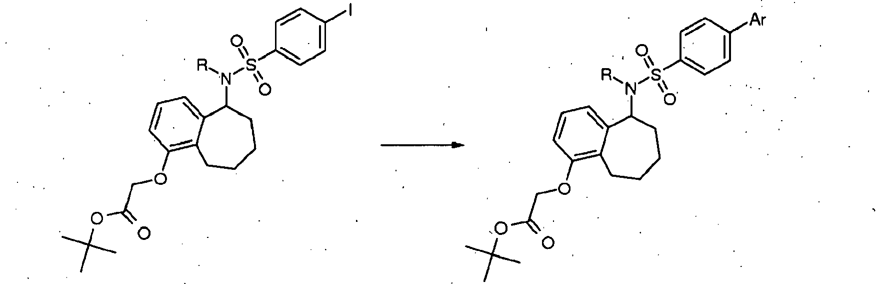

The arylboronic acid (1.2 equivalents), Pd(PPh₃)₄ (0.05 equivalents), and an aqueous solution of K₂CO₃ (1 M; 3 equivalents) were added to a degassed solution of the iodobenzenesulfonamide (1 equivalent) in dioxane (30 mL/mmol) at room temperature under argon. The mixture was heated at reflux for 4 h, then cooled and concentrated under reduced pressure. Ethyl acetate (150 mL/mmol) was added and the mixture was washed with water (2 x 30 mL/mmol), dried over anhydrous Na₂SO₄, filtered, and evaporated. The residue was purified by silica gel column chromatography, using 10-25% ethyl acetate/hexanes as eluent, to give the product.

### General Procedure 5

### Suzuki Reaction

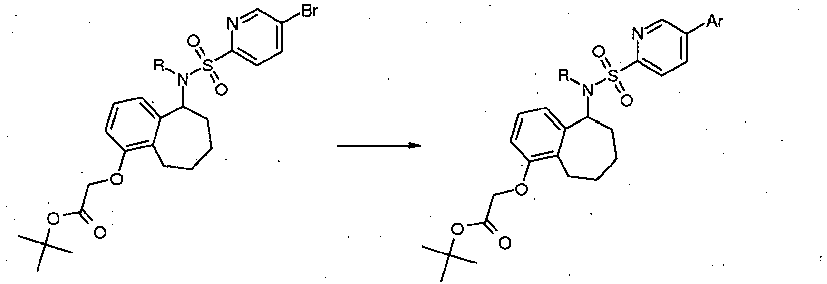

The arylboronic acid (1.2 equivalents), Pd(PPh₃)₄ (0.05 equivalents), and an aqueous solution of K₂CO₃ (1 M; 3 equivalents) were added to a degassed solution of the bromopyridinesulfonamide (1 equivalent) in dioxane (30 mL/mmol) at room temperature under argon. The mixture was heated at reflux for 4 h, then cooled and concentrated under reduced pressure. Ethyl acetate (150 mL/mmol) was added and the mixture was washed with water (2 x 30 mL/mmol), dried over anhydrous Na₂SO₄, filtered, and evaporated. The residue was purified by silica gel column chromatography, using 10-25% ethyl acetate/hexanes as eluent, to give the product.

### General Procedure 6

### Suzuki Reaction

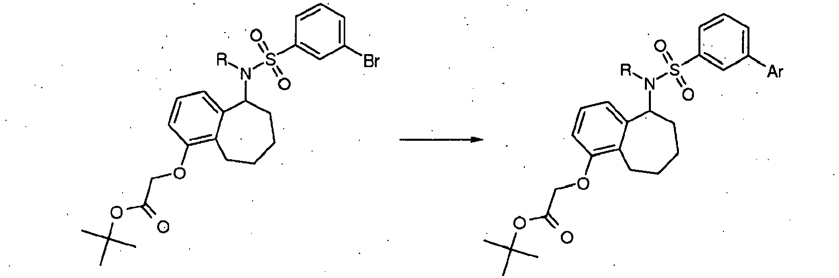

The arylboronic acid (1.2 equivalents), Pd(PPh₃)₄ (0.05 equivalents), and an aqueous solution of K₂CO₃ (1 M; 3 equivalents) were added to a degassed solution of the bromobenzenesulfonamide (1 equivalent) in dioxane (30 mL/mmol) at room temperature under argon. The mixture was heated at reflux for 4 h, then cooled and concentrated under reduced pressure. Ethyl acetate (50 mL/mmol) was added and the mixture was washed with water (2 x 30 mL/mmol), dried over anhydrous Na₂SO₄, filtered, and evaporated. The residue was purified by silica gel column chromatography, using 10-25% ethyl acetate/hexanes as eluent, to give the product.

### General Procedure 7

### Hydrolysis of 5-Arylsulfonylamino-6,7,8,9-tetrahydro-5H-benzocyclohepten-1-yloxy]-acetic Acid tert-Butyl Esters

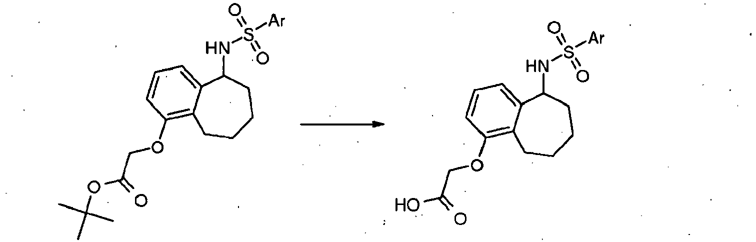

2 N Aqueous sodium hydroxide (20 equivalents) was added to a solution of the 5-arylsulfonylamino-6,7,8,9-tetrahydro-5H-benzocyclohepten-1-yloxy]-acetic acid tert-butyl ester (1 equivalent) in THF/H₂O (5:1; 18 mL/mmol). The reaction mixture was stirred at room temperature overnight. THF was removed from the mixture under reduced pressure, and the pH was adjusted to approximately 7 by the dropwise addition of 1 N HCl. The mixture was extracted with ethyl acetate (100 mL/mmol), and the organic extract was washed with water (3 x 20 mL/mmol), dried over anhydrous Na₂SO₄, filtered, and evaporated. The residue was purified by acid-base purification, by washing with organic solvent, or by preparative HPLC.

### PART II: PREPARATION OF PREFERRED INTERMEDIATES

### Intermediate 1.01 Bromo-6,7,8,9-tetrahydro-benzocyclohepten-5-one

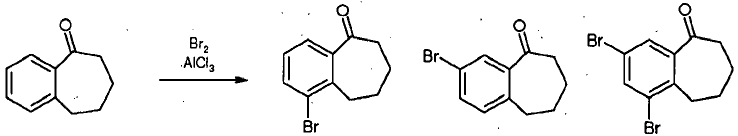

The titled compound was prepared using conditions similar to those described in Cornelius, L. A. M. and Combs, D. W. Synth. Commun. 1994, 24, 2777-2788. Aluminum chloride (17.18 g, 0.129 mol) was placed in a 250 mL, three-necked round-bottomed flask under argon. The flask was fitted with a condenser, overhead stirrer, and rubber septum; and 1-benzosuberone (available from Aldrich Chemical Company, Inc., 1001 West Saint Paul Avenue, Milwaukee, WI 53233, USA; approx. 7.6 mL; approx. 0.05 inol) was added slowly over 3 min. The mixture was stirred for 5 min, and then bromine (approx. 3.1 mL, approx. 0.06 mol) was added slowly over 9 min. The reaction vessel was placed in an oil bath at 80 °C and stirred for 5 min. The reaction mixture was then poured over a mixture of ice (100 g) and HCl (20 mL). Vigorous gas evolution occurred. The flask was rinsed with water and the combined rinsings and diluted reaction mixture were stirred for about 7 min. The mixture was extracted twice with ether. The combined extracts were washed with water and brine, dried (MgSO₄), filtered, and evaporated to give 12.71 g of crude material. Unsuccessful attempts were made to purify this material by chromatography (using a mixture of THF and hexanes as eluent) and also by distillation. The material was finally purified in two batches (of 6 g and 4.9 g) by supercritical fluid chromatography using a Daicel AD 5 x 25 cm column, and eluting with 20% MeOH/CO₂. The cycle time was 8.2 min, and the material was purified using 500 mg injections. 12 runs were made to purify the 6 g batch, and 10 runs were used to purify the 4.9 g batch.

Purification of the 6 g batch gave 2.77 g of 1-bromo-6,7,8,9-tetrahydro-benzocyclohepten-5-one as an orange oil [¹H NMR (300 MHz, DMSO-d₆) δ: 7.79 (dd, J=8.0, 1.1 Hz, 1 H), 7.46 (dd, J=7.5, 0.9 Hz, 1 H), 7.17-7.32 (m, 1 H), 3.03 (t, J=6.3 Hz, 2 H), 2.60-2.72 (m, 2 H), 1.54-1.83 (m, 4 H)]; 2.17 g of 3-bromo-6,7,8,9-tetrahydro-benzocyclohepten-5-one [¹H NMR (300 MHz, DMSO-d₆) δ: 7.62-7.69 (m, 2 H), 7.27 (d, J=8.2 Hz, 1 H), 2.88 (t, J=6.2 Hz, 2 H), 2.64-2.72 (m, 2 H), 1.61-1.82 (m, 4 H)]; and 0.44 g of 1,3-dibromo-6,7,8,9-tetrahydro-benzocyclohepten-5-one [¹H NMR (300 MHz, DMSO-d₆) δ: 8.05 (d, J=2.1 Hz, 1 H), 7.55 (d, J=1.8 Hz, 1 H), 2.99 (t, J=6.2 Hz, 2 H), 2.60-2.72 (m, 2 H), 1.57-1.80 (m, 4 H)].

Purification of the 4.9 g batch gave 1.19 g of 1-bromo-6,7,8,9-tetrahydro-benzocyclohepten-5-one [¹H NMR (300 MHz, DMSO-d₆) δ: 7.79 (dd, J=7.8, 0.9 Hz, 1 H), 7.46 (dd, J=7.7, 1.1 Hz, 1 H), 7.20-7.30 (m, 1 H), 2.94-3.15 (m, 2 H), 2.56-2.79 (m, 2 H), 1.50-1.86 (m, 4 H)]; 2.10 g of 3-bromo-6,7,8,9-tetrahydro-benzocyclohepten-5-one [¹H NMR (300 MHz, DMSO-d₆) δ: 7.62-7.68 (m, 2 H), 7.27 (d, J=7.8 Hz, 1 H), 2.88 (t, J=6.0 Hz, 2 H), 2.65-2.71 (m, 2 H), 1.62-1.81 (m, 4 H)]; and 1.38 g of 1,3-dibromo-6,7,8,9-tetrahydro-benzocyclohepten-5-one [¹H NMR (300 MHz, DMSO-d₆) δ: 8.05 (d, J=2.1 Hz, 1 H), 7.55 (d, J=1.8 Hz, 1 H), 2.99 (t, J=6.2 Hz, 2 H), 2.63-2.69 (m, 2 H), 1.56-1.80 (m, 4 H)].

The total yield of 1-bromo-6,7,8,9-tetrahydro-benzocyclohepten-5-one was 3.96 g (approx. 33%).

### Intermediate 1.02

### Hydroxy-6,7,8,9-tetrahydro-benzocyclohepten-5-one

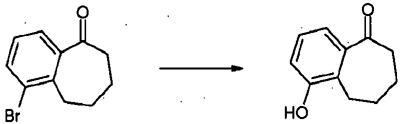

KOH pellets (632 mg, 11.3 mmol) were placed in a 25 mL round-bottomed flask and tris(dibenzylideneacetone)dipalladium(0) (available from Aldrich Chemical Company, Inc., 1001 West Saint Paul Avenue, Milwaukee, WI 53233, USA; 101 mg, 0.11 mmol) and 2-di-tert-butyl-phosphino-2',4',6'-triisopropylbiphenyl (available from Strem Chemicals, Inc., 7 Mulliken Way, Dexter Industrial Park, Newburyport, MA, USA; 383 mg, 0.9 mmol) were added. The flask was evacuated and filled with argon. Degassed water (2.5 mL) and a mixture of 1-bromo-6,7,8,9-tetrahydro-benzocyclohepten-5-one (from the second SFC purification described in the description of the preparation of Intermediate 1.01; 1.19 g, 5.0 mmol) in dioxane (2.5 mL) were added. The round-bottomed flask was placed in a preheated oil bath at 80 °C and heated overnight. The mixture was cooled to room temperature and 1 M HCl (5 mL) was added. The mixture was extracted three times with ethyl acetate, and the combined organic solutions were washed with brine, dried over MgSO₄, filtered and evaporated. The resulting crude material was purified using an Analogix Intelliflash 280 system, with a 24 g column. The mixture was eluted at 40 mL/min for 3 min with hexanes, then with a gradient of 0-25% ethyl acetate/hexanes for 10 min, and finally with 25% ethyl acetate/hexanes for 5 min. Fractions containing the product were evaporated to give 1-hydroxy-6,7,8,9-tetrahydro-benzocyclohepten-5-one (0.69 g, 79%) as a yellow solid. ¹H NMR (300 MHz, DMSO-d₆) δ: 9.64 (s, 1 H), 7.04-7.12 (m, 1 H), 6.93-7.00 (m, 2 H), 2.86 (t, J=5.7 Hz, 2 H), 2.61 (t, J=5.9 Hz, 2 H), 1.62-1.73 (m, 4H).

### Intermediate 1.03

### (5-Oxo-6,7,8,9-tetrahydro-5H-benzocyclohepten-1-yloxy)-acetic acid tert-butyl ester

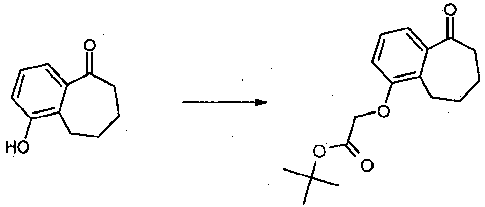

tert-Butyl bromoacetate (available from Aldrich Chemical Company, Inc., 1001 West Saint Paul Avenue, Milwaukee, WI 53233, USA; 2.76 g, 14.2 mmol) was added to a stirred mixture of 1-hydroxy-6,7,8,9-tetrahydro-benzocyclohepten-5-one (which may be prepared as described for Intermediate 1.02; 1.0 g, 5.67 mmol) and Cs₂CO₃ (5.52 g, 17.0 mmol) in CH₃CN (30 mL) at room temperature under nitrogen. The mixture was stirred at room temperature overnight and then filtered through celite. The celite was washed with EtOAc (10 mL) and the combined filtrates were concentrated under reduced pressure to give a gum. EtOAc (50 mL) was added and the mixture was washed with water (3 x 20 mL). The organic solution was dried over Na₂SO₄, filtered, and evaporated. The residue was purified by silica gel chromatography, using 5-10% EtOAc/hexanes as eluent, to give (5-oxo-6,7,8,9-tetrahydro-5H-benzocyclohepten-1-yloxy)-acetic acid tert-butyl ester (1.5 g, 91%) as a light yellow sticky material. ¹H NMR (300 MHz, DMSO-d₆) δ: 7.25 (t, J = 6.0 Hz, 1 H), 7.13 (d, J = 5.6 Hz, 1 H), 7.06 (d, J = 6.1 Hz, 1 H), 4.73 (s, 2 H), 2.96-2.98 (m, 2 H), 2.64-2.67 (m, 2 H), 1.66-1.74 (m, 4 H), 1.42 (s, 9 H).

### Intermediate 1.04

### (5-Amino-6,7,8,9-tetrahydro-5H-benzocyclohepten-1-yloxy)-acetic acid tert-butyl ester

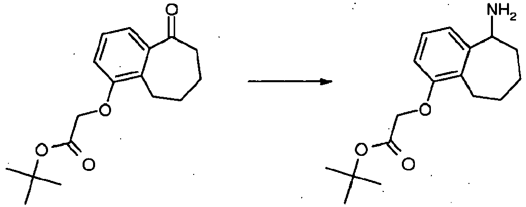

Ammonium acetate (13.27 g, 172.4 mmol) was added to a solution of (5-oxo-6,7,8,9-tetrahydro-5H-benzocyclohepten-1-yloxy)-acetic acid tert-butyl ester (which may be prepared as described for Intermediate 1.03; 2.5 g, 8.62 mmol) in MeOH (30 mL) at room temperature under nitrogen. The reaction mixture was stirred for 4 h at room temperature, and then cooled to ~0 °C. Sodium cyanoborohydride (1.35 g, 21.6 mmol) was added, and the reaction mixture was allowed to stir at room temperature for 24 h. The reaction mixture was concentrated, and the pH was adjusted to ~7 by the addition of aqueous saturated Na₂CO₃. The mixture was extracted with EtOAc (3 x 100 mL). The combined organic extracts were dried over anhydrous Na₂SO₄, filtered, and evaporated. The residue was purified by silica gel chromatography, using 2-5% MeOH/CH₂Cl₂ as eluent, to give (5-amino-6,7,8,9-tetrahydro-5H-benzocyclohepten-1-yloxy)-acetic acid tert-butyl ester (1.45 g, 58%) as a colorless semi-solid. ¹H NMR (300 MHz, DMSO-d₆) δ: 7.18-7.22 (t, J = 6.0 Hz, 1 H), 6.77-6.84 (m, 2 H), 4.68 (s, 2 H), 4.51-4.54 (m, 1 H), 2.42-2.45 (m, 1 H), 1.75-1.99 (m, 5 H), 1.49-1.51 (m, 1 H), 1.42 (s, 9 H), 1.12-1.23 (m, 3 H).

### Intermediate 1.05

### 5-Bromo-pyridine-2-thiol

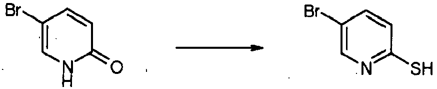

Lawesson's reagent (available from Aldrich Chemical Company, Inc., 1001 West Saint Paul Avenue, Milwaukee, WI 53233, USA; 2.32 g, 5.74 mmol) was added in portions to a suspension of 5-bromo-2(1H)-pyridone (available from Aldrich Chemical Company, Inc., 1001 West Saint Paul Avenue, Milwaukee, WI 53233, USA; 2 g, 11.5 mmol) in dry toluene (50 mL) under nitrogen. The reaction mixture was heated at reflux for 1 h and then cooled to room temperature. The product that precipitated on cooling was collected by filtration and dried under high vacuum to give 5-bromo-pyridine-2-thiol (2.1 g, 96%), which was used directly in the next step without further purification.

### Intermediate 1.06

### 5-Bromo-pyridine-2-sulfonyl chloride

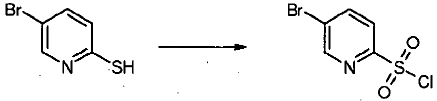

A suspension of 5-bromo-pyridine-2-thiol (which may be prepared as described for Intermediate 1.05; 2 g, 10.5 mmol) in carbon tetrachloride (40 mL) and water (8 mL) was cooled to ∼0 °C using an ice-bath. Chlorine gas was bubbled through the reaction mixture for 20 min and then CH₂Cl₂ (100 mL) was added. The mixture was washed with brine. The organic layer was dried over anhydrous Na₂SO₄, filtered, and evaporated under reduced pressure to give 5-bromo-pyridine-2-sulfonyl chloride (1.92 g, 71%) as a light yellow solid which was used directly in the next step without further purification. ¹H NMR (400 MHz, DMSO-*d*₆) δ: 8.63 (d, *J*=1.5 Hz, 3 H), 8.07 (dd, *J*=8.3, 2.2 Hz, 3 H), 7.68 (d, *J*=8.3 Hz, 3 H).

### Intermediate 1.07

### [5-(3-Isopropenyl-5-trifluoromethyl-benzenesulfonylamino)-6,7,8,9-tetrahydro-5H-benzocyclohepten-1-yloxy]-acetic acid tert-butyl ester

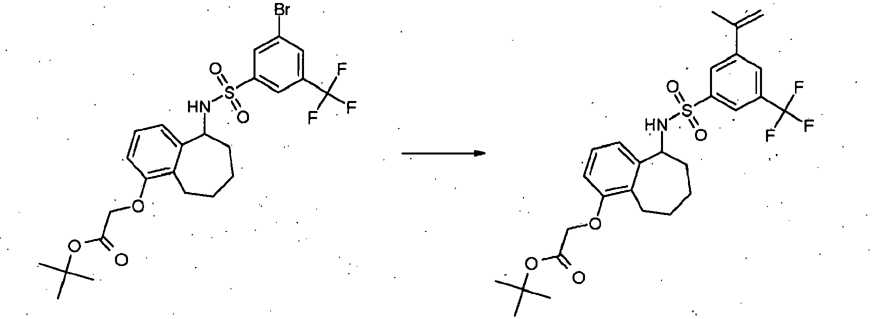

1 M K₂CO₃ (0.5 mL, 0.5 mmol), Pd(PPh₃)₄ (available from Aldrich Chemical Company, Inc., 1001 West Saint Paul Avenue, Milwaukee, WI 53233, USA; 60 mg, 0.05 mmol), and isopropenylboronic acid pinacol ester (available from Aldrich Chemical Company, Inc., 1001 West Saint Paul Avenue, Milwaukee, WI 53233, USA; 171 mg, 1.02 mmol) were added to a solution of [5-(3-bromo-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.07; 200 mg, 0.34 mmol) in dioxane (8 mL) with continuous purging with argon. The tube was sealed and the mixture was heated at 100 °C for 16 h. The mixture was cooled to room temperature and partitioned between water and ethyl acetate. The organic layer was dried over anhydrous Na₂SO₄, filtered, and evaporated to give [5-(3-isopropenyl-5-trifluoromethyl-benzenesulfonylamino)-6,7,8,9-tetrahydro-5H-benzocyclohepten-1-yloxy]-acetic acid tert-butyl ester (200 mg, crude yield: 107%) as a white solid. ¹H NMR (400 MHz, DMSO-*d*₆) δ: 8.38 (br. s., 1 H), 8.02 (s, 1 H), 7.95 (s, 1 H), 7.87 (s, 1 H), 6.90 (t, *J*=7.9 Hz, 1 H), 6.72 (d, *J*=7.3 Hz, 1 H), 6.60 (d, *J*=7.9 Hz, 1 H), 5.59 (s, 1 H), 5.32 (s, 1 H), 4.45-4.59 (m, 3 H), 2.97-3.07 (m, 1 H), 2.62-2.70 (m, 1 H), 2.13 (s, 3 H), 1.47-1.86 (m, 4 H), 1.41 (s, 9 H).

### Intermediate 1.08

### [5-(4-Iodo-benzenesulfonylamino)-6,7,8,9-tetrahydro-5H-benzocyclohepten-1-yloxy]-acetic acid tert-butyl ester

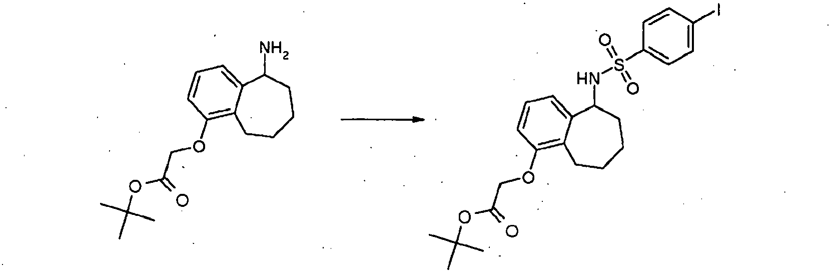

A mixture of (5-amino-6,7,8,9-tetrahydro-5H-benzocyclohepten-1-yloxy)-acetic acid tert-butyl ester (which may be prepared as described for Intermediate 1.04; 400 mg, 1.4 mmol), diisopropylethylamine (0.46 mL, 2.6 mmol), and 4-iodobenzenesulfonyl chloride (available from Aldrich Chemical Company, Inc., 1001 West Saint Paul Avenue, Milwaukee, WI 53233, USA; 414.4 mg, 1.4 mmol) in THF (10 mL) was stirred at room temperature for 14 h. The reaction mixture was concentrated under reduced pressure and the residue was purified by silica gel chromatography, using 9% EtOAc/hexane as eluent, to give [5-(4-iodo-benzenesulfonylamino)-6,7,8,9-tetrahydro-5H-benzocyclohepten-1-yloxy]-acetic acid tert-butyl ester (500 mg, 65%). ¹H NMR (400 MHz, DMSO-*d*₆) δ: 8.26 (d, *J*=7.6 Hz, 1 H), 7.91 (d, *J*=8.1 Hz, 2 H), 7.52 (d, *J*=8.1 Hz, 2 H), 6.94-7.05 (m, 1 H), 6.81 (d, *J*=7.6 Hz, 1 H), 6.67 (d, *J*=8.3 Hz, 1 H), 4.59 (s, 2 H), 4.43 (br. s., 1 H), 3.11-3.18 (m, 1 H), 1.67-1.76 (m, 1 H), 1.46-1.57 (m, 5 H), 1.41 (s, 9 H), 1.17-1.29 (m, 1 H), 0.74-1.09 (m, 1 H).

### Intermediate 1.09

### [5-(5-Bromo-pyridine-2-sulfonylamino)-6,7,8,9-tetrahydro-5H-benzocyclohepten-1-yloxy]-acetic acid tert-butyl ester

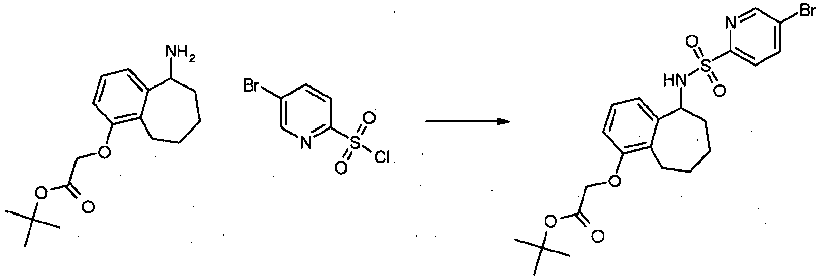

Using the conditions described for the preparation of Intermediate 1.08, (5-amino-6,7,8,9-tetrahydro-5H-benzocyclohepten-1-yloxy)-acetic acid tert-butyl ester (which may be prepared as described for Intermediate 1.04; 168.8 mg, 0.58 mmol) was reacted with diisopropylethylamine (0.2 mL, 1.2 mmol) and 5-bromo-pyridine-2-sulfonyl chloride (which may be prepared as described for Intermediate 1.06; 150 mg, 0.58 mmol) to give [5-(5-bromo-pyridine-2-sulfonylamino)-6,7,8,9-tetrahydro-5H-benzocyclohepten-1-yloxy]-acetic acid tert-butyl ester (100 mg, 34%). ¹H NMR (400 MHz, DMSO-*d*₆) δ: 8.84 (s, 1 H), 8.54 (d, *J*=7.8 Hz, 1 H), 8.24 (d, *J*=8.3 Hz, 1 H), 7.77 (d, *J*=8.3 Hz, 1 H), 6.94-7.03 (m, 1 H), 6.86 (d, *J*=7.3 Hz, 1 H), 6.66 (d, *J*=7.8 Hz, 1 H), 4.64 (t, *J*=7.8 Hz, 1 H), 4.59 (s, 2 H), 3.16-3.25 (m, 1 H), 1.50-1.82 (m, 5 H), 1.41 (s, 9 H), 1.13-1.35 (m, 2 H).

### Intermediate 1.10

### [5-(3-Bromo-benzenesulfonylamino)-6,7,8,9-tetrahydro-5H-benzocyclohepten-1-yloxy]-acetic acid tert-butyl ester

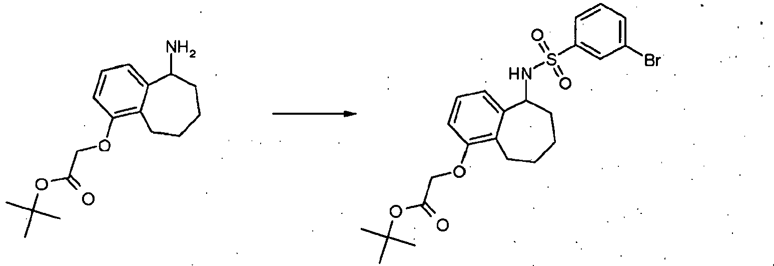

A mixture of (5-amino-6,7,8,9-tetrahydro-5H-benzocyclohepten-1-yloxy)-acetic acid tert-butyl ester (which may be prepared as described for Intermediate 1.04; 200 mg, 0.7 mmol), diisopropylethylamine (0.3 mL, 1.7 mmol), and 3-bromobenzenesulfonyl chloride (available from Aldrich Chemical Company, Inc., 1001 West Saint Paul Avenue, Milwaukee, WI 53233, USA; 150 mg, 0.59 mmol) in THF (6 mL) was stirred at room temperature under nitrogen for 3 h. The mixture was concentrated under reduced pressure and the residue was purified by silica gel column chromatography to give [5-(3-bromo-benzenesulfonylamino)-6,7,8,9-tetrahydro-5H-benzocyclohepten-1-yloxy]-acetic acid tert-butyl ester (200 mg, 57%). ¹H NMR (400 MHz, DMSO-*d*₆) δ: 8.31 (br. s., 1 H), 7.87 (s, 1 H), 7.79 (t, *J*=8.7 Hz, 2 H), 7.50 (t, *J*=7.8 Hz, 1 H), 6.95-7.07 (m, 1 H), 6.79 (d, *J*=7.6 Hz, 1 H), 6.67 (d, *J*=8.1 Hz, 1 H), 4.58 (s, 2 H), 4.47 (br. s., 1 H), 3.07-3.12 (m, 1 H), 2.54-2.64 (m, 1 H), 1.71-1.79 (m, 1 H), 1.47-1.57 (m, 4 H), 1.41 (s, 9 H), 1.21-1.31 (m, 1 H).

### Intermediate 2.01

### [5-(3,5-Bis-trifluoromethyl-benzenesulfonylamino)-6,7,8,9-tetrahydro-5H-benzocyclohepten-1-yloxy]-acetic acid tert-butyl ester

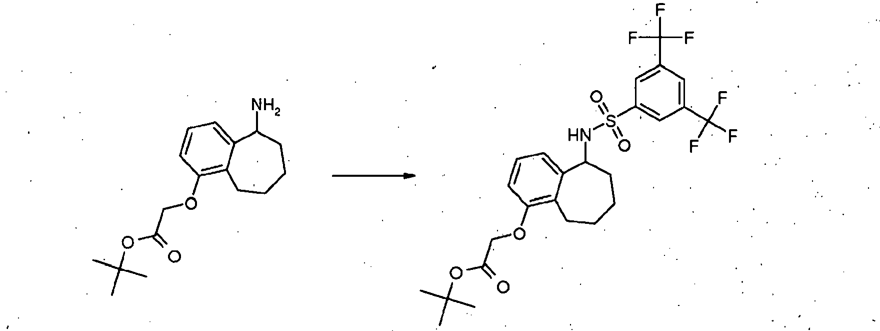

Diisopropylethylamine (0.15 mL, 0.86 mmol) was added at 0 °C to a stirred solution of (5-amino-6,7,8,9-tetrahydro-5H-benzocyclohepten-1-yloxy)-acetic acid tert-butyl ester (which may be prepared as described for Intermediate 1.04; 0.10 g, 0.34 mmol) in anhydrous CH₂Cl₂ (3 mL). The reaction mixture was stirred at this temperature for 15 min and then a solution of bis-(3,5-trifluoromethyl)benzenesulfonyl chloride (available from Aldrich Chemical Company, Inc., 1001 West Saint Paul Avenue, Milwaukee, WI 53233, USA; 0.129 g, 0.41 mmol) was added. The reaction mixture was stirred at room temperature overnight and then concentrated. EtOAc was added and the mixture was washed with water. The organic layer was dried over anhydrous Na₂SO₄, filtered, evaporated, and purified by silica gel column chromatography (eluting with 10% EtOAc/hexane) to give [5-(3,5-bis-trifluoromethyl-benzenesulfonylamino)-6,7,8,9-tetrahydro-5H-benzocyclohepten-1-yloxy]-acetic acid tert-butyl ester (85 mg, 49%) as a solid. ¹H NMR (400 MHz, DMSC)-*d*₆) δ: 8.52 (d, *J*=7.8 Hz, 1 H), 8.31 (s, 1 H), 8.19 (s, 2 H), 6.81-6.91 (m, 1 H), 6.66 (d, *J*=7.3 Hz, 1 H), 6.55 (d, *J*=8.3 Hz, 1 H), 4.60 (t, *J*=6.8 Hz, 1 H), 4.35-4.52 (m, 2 H), 2.81 (br. s., 2 H), 1.70-1.92 (m, 2 H), 1.52-1.66 (m, 2 H), 1.41 (s, 9 H), 1.17-1.28 (m, 2 H).

### Intermediate 2.02

### [5-(3,5-Dichloro-benzenesulfonylamino)-6,7,8,9-tetrahydro-5H-benzocyclohepten-1-yloxy]-acetic acid tert-butyl ester

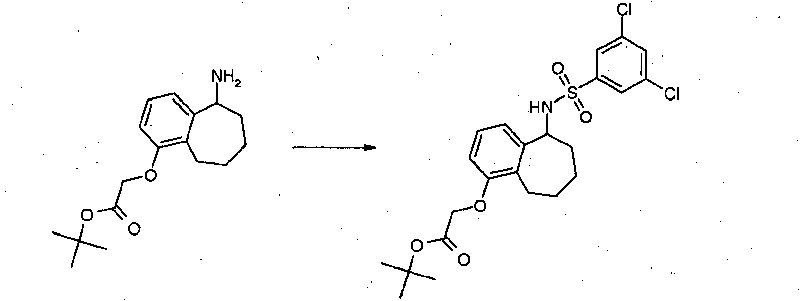

Using the conditions described for the preparation of Intermediate 2.04, (5-amino-6,7 ,8,9-tetrahydro-5H-benzocyclohepten-1-yloxy)-acetic acid tert-butyl ester (which may be prepared as described for Intermediate 1.04; 0.25 g, 0.86 mmol) was reacted with 3,5-dichlorobenzenesulfonyl chloride (available from Aldrich Chemical Company, Inc., 1001 West Saint Paul Avenue, Milwaukee, WI 53233, USA; 252 mg, 1.03 mmol) in the presence of diisopropylethylamine (0.35 mL, 2.0 mmol) in CH₂Cl₂ (5 mL) to give [[5-(3,5-dichloro-benzenesulfonylamino)-6,7,8,9-tetrahydro-5H-benzocyclohepten-1-yloxy]-acetic acid tert-butyl ester (180 mg, 42%) as a solid. ¹H NMR (400 MHz, DMSO-*d*₆) δ: 8.40 (s, 1 H), 7.86 (s, 1 H), 7.67 (d, *J*=1.5 Hz, 2 H), 6.92-7.04 (m, 1 H), 6.76 (d, *J*=7.3 Hz, 1 H), 6.67 (d, *J*=8.3 Hz, 1 H), 4.50-4.57 (m, 3 H), 2.94-3.06 (m, 1 H), 2.64-2.78 (m, 1 H), 1.75-1.85 (m, 1 H), 1.51-1.67 (m, 3 H), 1.42 (s, 9 H), 1.28-1.36 (m, 1 H).

### Intermediate 2.03

### [5-(Biphenyl-3-sulfonylamino)-6,7,8,9-tetrahydro-5H-benzocyclohepten-1-yloxy]-acetic acid tert-butyl ester

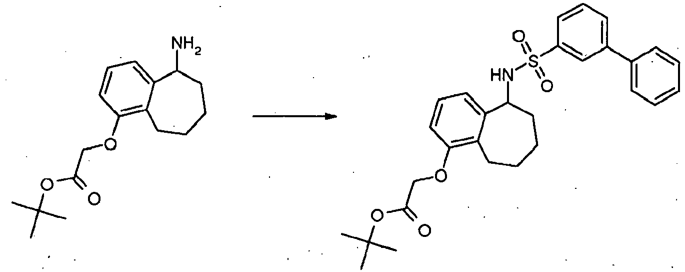

Using the conditions described for the preparation of Intermediate 2.04, (5-amino-6,7,8,9-tetrahydro-5H-benzocyclohepten-1-yloxy)-acetic acid tert-butyl ester (which may be prepared as described for Intermediate 1.04; 0.25 g, 0.86 mmol) was reacted with 3-phenylbenzenesulfonyl chloride (available from Chem-Impex International, Inc., 935 Dillon Drive, Wood Dale, IL 60191, USA; 0.26 g, 1.03 mmol) in the presence of diisopropylethylamine (0.35 mL, 2.14 mmol) in CH₂Cl₂ (5 mL) to give [5-(biphenyl-3-sulfonylamino)-6,7,8,9-tetrahydro-5H-benzocyclohepten-1-yloxy]-acetic acid tert-butyl ester (160 mg, 37%) as a solid. ¹H NMR (400 MHz, DMSO-*d*₆) δ: 8.23 (d, *J*=7.8 Hz, 1 H), 8.00 (s, 1 H), 7.89 (d, *J*=7.8 Hz, 1 H), 7.78 (d, *J*=7.8 Hz, 1 H), 7.60-7.67 (m, 3 H), 7.51 (t, *J*=7.6 Hz, 2 H), 7.40-7.46 (m, 1 H), 6.93-7.01 (m, 1 H), 6.84 (d, *J*=7.3 Hz, 1 H), 6.65 (d, *J*=7.8 Hz, 1 H), 4.55 (s, 2 H), 4.45-4.53 (m, 1 H), 3.18 (dd, *J*=13.2, 7.3 Hz, 1 H), 2.54-2.61 (m, 1 H), 1.68-1.76 (m, 1 H), 1.46-1.59 (m, 4 H), 1.41 (s, 9 H), 1.21-1.27 (m, 1 H).

### Intermediate 2.04

### [5-(Biphenyl-4-sulfonylamino)-6,7,8,9-tetrahydro-5H-benzocyclohepten-1-yloxy]-acetic acid tert-butyl ester

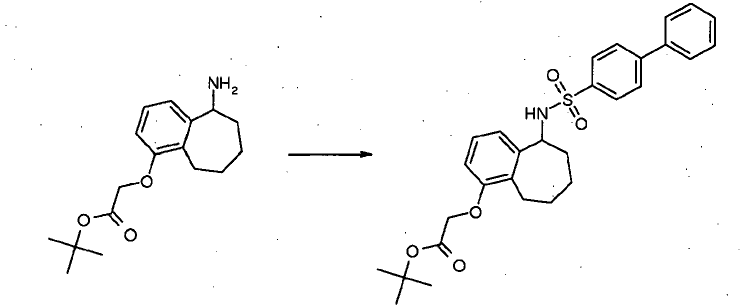

Biphenyl-4-sulfonyl chloride (available from Aldrich Chemical Company, Inc., 1001 West Saint Paul Avenue, Milwaukee, WI 53233, USA; 0.26 g, 1.03 mmol), and diisopropylethylamine (0.35 mL, 2.0 mmol) were added to a ~0°C solution of (5-amino-6,7,8,9-tetrahydro-5H-benzocyclohepten-1-yloxy)-acetic acid tert-butyl ester (which may be prepared as described for Intermediate 1.04; 0.25 g, 0.86 mmol) in CH₂Cl₂ (5 mL) under nitrogen. The mixture was stirred at room temperature for 5 h and then water (5 mL) was added. The mixture was extracted with CH₂Cl₂ (2 x 20 mL) and the combined extracts were washed with water (10 mL) and brine (10 mL), dried over anhydrous Na₂SO₄, filtered and concentrated. The residue was purified by silica gel column chromatography (eluting with 5% EtOAc/hexane) to give [5-(biphenyl-4-sulfonylamino)-6,7,8,9-tetrahydro-5H-benzocyclohepten-1-yloxy]-acetic acid tert-butyl ester (170 mg, 39%) as a solid. ¹H NMR (400 MHz, DMSO-*d*₆) δ: 8.22 (d, *J*=7.8 Hz, 1 H), 7.84 (s, 4 H), 7.73 (d, *J*=7.3 Hz, 2 H), 7.48-7.54 (m, 2 H), 7.41-7.46 (m, 1 H), 6.95-7.04 (m, 1 H), 6.86 (d, *J*=7.3 Hz, 1 H), 6.67 (d, *J*=8.3 Hz, 1 H), 4.57 (s, 2 H), 4.48 (t, *J*=6.4 Hz, 1 H), 3.14-3.23 (m, 1 H), 1.69-1.75 (m, 1 H), 1.46-1.60 (m, 4 H), 1.40 (s, 9 H), 1.20-1.31 (m, 2 H).

### Intermediate 2.05

### [5-(3-Methanesulfonyl-benzenesulfonylamino)-6,7,8,9-tetrahydro-5H-benzocyclohepten-1-yloxy]-acetic acid tert-butyl ester

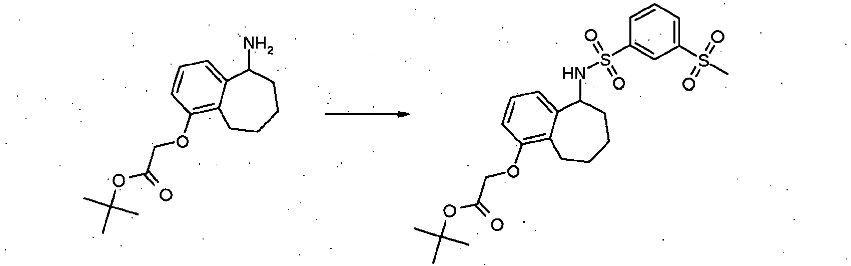

Using the conditions described for the preparation of Intermediate 2.04, (5-amino-6,7,8,9-tetrahydro-5H-benzocyclohepten-1-yloxy)-acetic acid tert-butyl ester (which may be prepared as described for Intermediate 1.04; 0.25 g, 0.86 mmol) was reacted with 3-(methylsulfonyl)benzenesulfonyl chloride (available from Oakwood Products, Inc., 1741 Old Dunbar Road, West Columbia, SC 29172, USA; 262 mg, 1.03 mmol) in the presence of diisopropylethylamine (0.35 mL, 2.0 mmol) in CH₂Cl₂ (5 mL) to give [5-(3-methanesulfonyl-benzenesulfonylamino)-6,7,8,9-tetrahydro-5H-benzocyclohepten-1-yloxy]-acetic acid tert-butyl ester (185 mg, 42%) as a solid. ¹H NMR (400 MHz, DMSO-*d*₆) δ: 8.44 (d, *J*=7.3 Hz, 1 H), 8.26 (s, 1 H), 8.12 (d, *J*=7.8 Hz, 1 H), 8.05 (d, *J*=8.1 Hz, 1 H), 7.77-7.81 (m, 1 H), 6.94 (t, *J*=7.9 Hz, 1 H), 6.74 (d, *J*=7.8 Hz, 1 H), 6.63 (d, *J*=8.1 Hz, 1 H), 4.56 (s, 2 H), 4.49-4.54 (m, 1 H), 3.24 (s, 3 H), 3.05-3.13 (m, 1 H), 2.59-2.69 (m, 1 H), 1.69-1.81 (m, 1 H), 1.49-1.61 (m, 4 H), 1.41 (s, 9 H), 1.22-1.31 (m, 1 H).

### Intermediate 2.06

### [5-(3-Fluoro-5-trifluoromethyl-benzenesulfonylamino)-6,7,8,9-tetrahydro-5H-benzocyclohepten-1-yloxy]-acetic acid tert-butyl ester

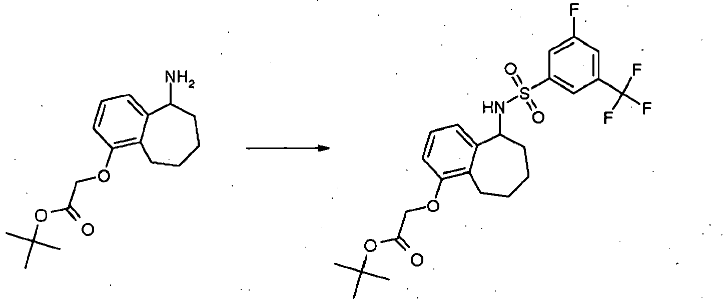

Using the conditions of General Procedure 2, (5-amino-6,7,8,9-tetrahydro-5H-benzocyclohepten-1-yloxy)-acetic acid tert-butyl ester (which may be prepared as described for Intermediate 1.04; 1.0 g, 3.4 mmol) was reacted with 3-fluoro-5-(trifluoromethyl)benzenesulfonyl chloride (available from Alfa Aesar, 26 Parkridge Road, Ward Hill, MA 01835, USA; 0.9 g, 3.4 mmol) in the presence of diisopropylethylamine (0.89 g, 6.9 mmol) in THF (20 mL) to give [5-(3-fluoro-5-trifluoromethyl-benzenesulfonylamino)-6,7,8,9-tetrahydro-5H-benzocyclohepten-1-yloxy]-acetic acid tert-butyl ester (0.7 g, 39%). ¹H NMR (400 MHz, DMSO-*d*₆) δ: 8.48 (br s, 1 H), 7.96 (d, *J*=8.3 Hz, 1 H), 7.84 (s, 1 H), 7.80 (d, *J*=7.6 Hz, 1 H), 6.93 (t, *J*=8.1 Hz, 1 H), 6.72 (d, *J*=7.6 Hz, 1 H), 6.63 (d, *J*=8.1 Hz, 1 H), 4.49-4.58 (m, 3 H), 2.93-3.02 (m, 1 H), 2.66-2.76 (m, 1 H), 1.51-1.85 (m, 5 H), 1.41 (s, 9 H), 1.16-1.27 (m, 1 H).

### Intermediate 2.07

### [5-(3-Bromo-5-trifluoromethyl-benzenesulfonylamino)-6,7,8,9-tetrahydro-5H-benzocyclohepten-1-yloxy]-acetic acid tert-butyl ester

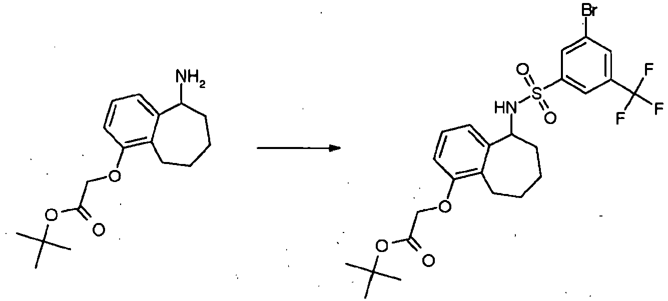

Using the conditions described for the preparation of Intermediate 1.08, (5-amino-6,7,8,9-tetrahydro-5H-benzocyclohepten-1-yloxy)-acetic acid tert-butyl ester (which may be prepared as described for Intermediate 1.04; 400 mg, 1.4 mmol) was reacted with 3-bromo-5-(trifluoromethyl)benzenesulfonyl chloride (available from Alfa Aesar, 26 Parkridge Road, Ward Hill, MA 01835, USA; 444 mg, 1.4 mmol) in the presence of diisopropylethylamine (0.46 g, 2.6 mmol) to give [5-(3-bromo-5-trifluoromethyl-benzenesulfonylamino)-6,7,8,9-tetrahydro-5H-benzocyclohepten-1-yloxy]-acetic acid tert-butyl ester (300 mg, 38%). ¹H NMR (400 MHz, DMSO-*d*₆) δ: 8.45 (br. s., 1 H), 8.19 (br. s.; 1 H), 8.04 (br. s., 1 H), 7.95 (s, 1 H), 6.89-6.97 (m, 1 H), 6.71 (d, *J*=7.6 Hz, 1 H); 6.62 (d, *J*=8.1 Hz, 1 H), 4.48-4.62 (m, 3 H), 1.50-1.62 (m, 1 H), 1.42 (s, 9 H), 1.13-1.31 (s, 1 H).

### Intermediate 2.08

### [5-(3,5-Bis-methanesulfonyl-benzenesulfonylamino)-6,7,8,9-tetrahydro-5H-benzocyclohepten-1-yloxy]-acetic acid tert-butyl ester

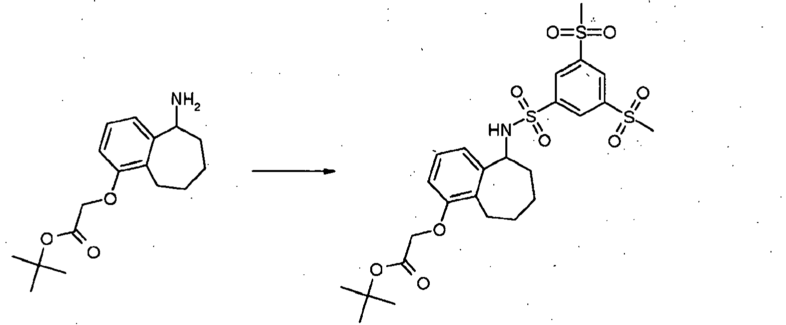

Using the conditions of General Procedure 2, (5-amino-6,7,8,9-tetrahydro-5H-benzocyclohepten-1-yloxy)-acetic acid tert-butyl ester (which may be prepared as described for Intermediate 1.04; 0.2 g, 0.69 mmol) was reacted with 3,5-bis(methylsulfonyl)benzenesulfonyl chloride (available from Oakwood Products, Inc., 1741 Old Dunbar Road, West Columbia, SC 29172, USA; 0.229 g, 0.69 mmol) in the presence of diisopropylethylamine (2.4 mL, 1.4 mmol), in THF (5 mL), to give [5-(3,5-bis-methanesulfonyl-benzenesulfonylamino)-6,7,8,9-tetrahydro-5H-benzocyclohepten-1-yloxy]-acetic acid tert-butyl ester (0.22 g, 54%). ¹H NMR (400 MHz, DMSO-*d*₆) δ: 8.62 (d, *J*=7.3 Hz, 1 H), 8.52 (s, 1 H), 8.45 (s, 2 H), 6.84-6.94 (m, 1 H), 6.67 (d, *J*=7.8 Hz, 1 H), 6.55 (d, *J*=8.3 Hz, 1 H), 4.56-4.66 (m, 1 H), 4.51 (s, 2 H), 2.69-2.96 (m, 2 H), 1.52-1.89 (m, 4 H), 1.42 (s, 9 H), 1.22-1.28 (m, 1 H).

### Intermediate 2.09

### [5-(Biphenyl-4-sulfonylamino)-6,7,8,9-tetrahydro-5H-benzocyclohepten-1-yloxy]-acetic acid tert-butyl ester

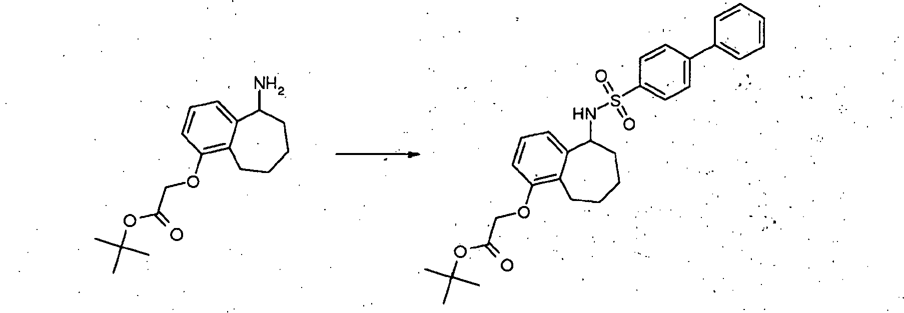

Using the conditions of General Procedure 2, (5-amino-6,7,8,9-tetrahydro-5H-benzocyclohepten-1-yloxy)-acetic acid tert-butyl ester (which may be prepared as described for Intermediate 1.04; 0.2 g, 0.69 mmol) was reacted with 4-biphenylsulfonyl chloride (available from Aldrich Chemical Company, Inc., 1001 West Saint Paul Avenue, Milwaukee, WI 53233, USA; 0.174 g, 0.69 mmol) in the presence of diisopropylethylamine (0.178 g, 1.37 mmol) in THF (5 mL) to give [5-(biphenyl-4-sulfonylamino)-6,7,8,9-tetrahydro-5H-benzocyclohepten-1-yloxy]-acetic acid tert-butyl ester (0.21 g, 60%). ¹H NMR (400 MHz, DMSO-*d*₆) δ: 8.24 (d, *J*=7.8 Hz, 1 H), 7.85 (s, 4 H), 7.73 (d, *J*=7.3 Hz, 2 H), 7.39-7.56 (m, 2 H), 6.94-7.04 (m, 1 H), 6.86 (d, *J*=7.3 Hz, 1 H), 6.66 (d, *J*=8.3 Hz, 1 H), 4.57 (s, 2 H), 4.47 (br. s., 1 H), 1.46-1.80 (m, 5 H), 1.40 (s, 9 H), 1.20-1.30 (m, 1 H).

### [5-(3-Acetyl-5-trifluoromethyl-benzenesulfonylamino)-6,7,8,9-tetrahydro-5H-benzocyclohepten-1-yloxy]-acetic acid tert-butyl ester

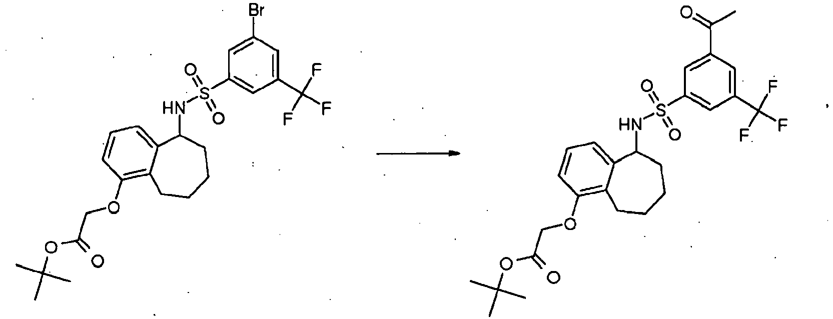

A mixture of [5-(3-bromo-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.07; 200 mg, 0.35 mmol), tris(dibenzylideneacetone)dipalladium(0) (available from Aldrich Chemical Company, Inc., 1001 West Saint Paul Avenue, Milwaukee, WI 53233, USA; 35 mg, 0.04 mmol), triphenylarsine (available from Aldrich Chemical Company, Inc., 1001 West Saint Paul Avenue, Milwaukee, WI 53233, USA; 35 mg, 0.11 mmol), and tributyl(1-ethoxyvinyl)tin (available from Aldrich Chemical Company, Inc., 1001 West Saint Paul Avenue, Milwaukee, WI 53233, USA; 0.2 mL, 0.6 mmol) in DMF (2 mL) was heated at 80 °C for 2 h. The reaction mixture was cooled to room temperature and 4 N HCl (0.1 mL) was added. The mixture was stirred for 10 min at room temperature and then poured into water (4 mL). The mixture was extracted with EtOAc (3 x 10 mL). The organic layers were combined, washed with brine (10 mL), dried over anhydrous Na₂SO₄, filtered, and evaporated. The residue was purified by silica gel chromatography to give [5-(3-acetyl-5-trifluoromethyl-benzenesulfonylamino)-6,7,8,9-tetrahydro-5H-benzocyclohepten-1-yloxy]-acetic acid tert-butyl ester (165 mg, 88%). ¹H NMR (400 MHz, DMSO-*d*₆) δ: 8.49 (d, *J*=7.8 Hz, 1 H), 8.36 (s, 1 H), 8.32 (s, 1 H), 8.16 (s, 1 H), 6.84-6.91 (m, 1 H), 6.69 (d, *J*=7.8 Hz, 1 H), 6.55 (d, *J*=8.3 Hz, 1 H), 4.56 (d, *J*=6.8 Hz, 1 H), 4.41-4.53 (m, 2 H), 2.69-2.95 (m, 2 H), 2.65 (s, 3 H), 1.52-1.89 (m, 5 H), 1.42 (s, 9 H).

### Intermediate 2.11

### [5-(3-Methanesulfonyl-5-trifluoromethyl-benzenesulfonylamino)-6,7,8,9-tetrahydro-5H-benzocyclohepten-1-yloxy]-acetic acid tert-butyl ester

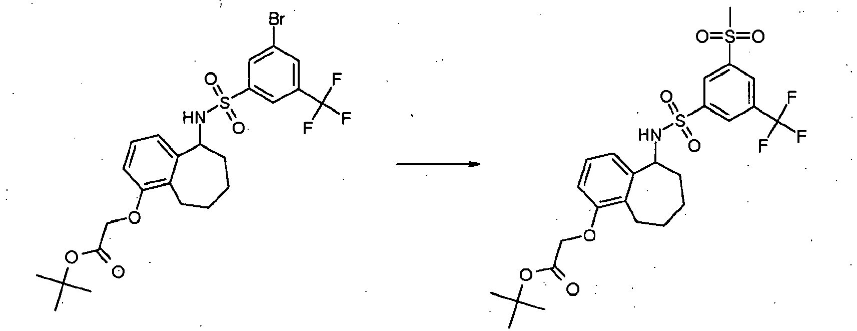

Sodium methanesulfinate (available from Aldrich Chemical Company, Inc., 1001 West Saint Paul Avenue, Milwaukee, WI 53233, USA; 80 mg, 0.78 mmol) and copper(I) iodide (149 mg, 0.78 mmol) were added to a degassed solution of [5-(3-bromo-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.07; 100 mg, 0.17 mmol) in NMP (5 mL). The reaction mixture was heated at 150 °C for 3 h. The reaction mixture was cooled to room temperature. Ethyl acetate (20 mL) was added and the mixture was washed with brine (10 mL). The organic layer was dried over anhydrous Na₂SO₄, filtered, and evaporated. The residue was purified by silica gel chromatography, using 15% EtOAc/hexanes as eluent, to give [5-(3-methanesulfonyl-5-trifluoromethyl-benzenesulfonylamino)-6,7,8,9-tetrahydro-5H-benzocyclohepten-1-yloxy]-acetic acid tert-butyl ester (80 mg, 80%).

### Intermediate 2.12

### [5-(3'-Isopropyl-biphenyl-4-sulfonylamino)-6,7,8,9-tetrahydro-5H-benzocyclohepten-1-yloxy]-acetic acid tert-butyl ester

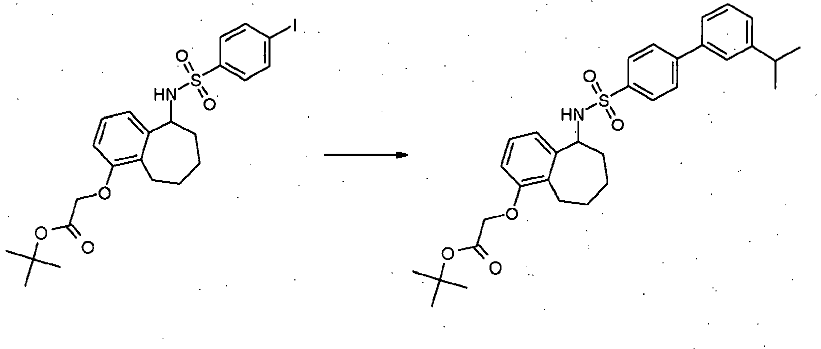

Using the conditions of General Procedure 4, [5-(4-iodo-benzenesulfonylamino)-6,7,8,9-tetrahydro-5H-benzocyclohepten-1-yloxy]-acetic acid tert-butyl ester (which may be prepared as described for Intermediate 1.08; 300 mg, 0.54 mmol) was reacted with 3-isopropylphenylboronic acid (available from Alfa Aesar, 26 Parkridge Road, Ward Hill, MA 01835, USA; 106 mg, 0.65 mmol) in the presence of Pd(PPh₃)₄ (available from Aldrich Chemical Company, Inc., 1001 West Saint Paul Avenue, Milwaukee, WI 53233, USA; 60 mg, 0.05 mmol), and 1 M aqueous K₂CO₃ (0.8 mL, 0.8 mmol) in dioxane (7 mL) to give [5-(3'-isopropyl-biphenyl-4-sulfonylamino)-6,7,8,9-tetrahydro-5H-benzocyclohepten-1-yloxy]-acetic acid tert-butyl ester (280 mg, 97%). ¹H NMR (400 MHz, DMSO-*d*₆) δ: 8.22 (d, *J*=7.6 Hz, 1 H), 7.84 (s, 4 H), 7.58 (s, 1 H), 7.53 (d, *J*=7.6 Hz, 1 H), 7.42 (t, *J*=7.7 Hz, 1 H), 7.32 (d, *J*=7.6 Hz, 1 H), 6.94-7.04 (m, 1 H), 6.87 (d, *J*=7.6 Hz, 1 H), 6.67 (d, *J*=8.3 Hz, 1 H), 4.57 (s, 2 H), 4.47 (br. s., 1 H), 3.16-3.23 (m, 1 H), 2.99 (dt, *J*=13.7, 6.8 Hz, 1 H), 1.45-1.78 (m, 5 H), 1.40 (s, 9 H), 1.22-1.31 (m, 10 H).

### Intermediate 2.13

### [5-(3'-tert-Butyl-5'-methyl-biphenyl-4-sulfonylamino)-6,7,8,9-tetrahydro-5H-benzocyclohepten-1-yloxy]-acetic acid tert-butyl ester

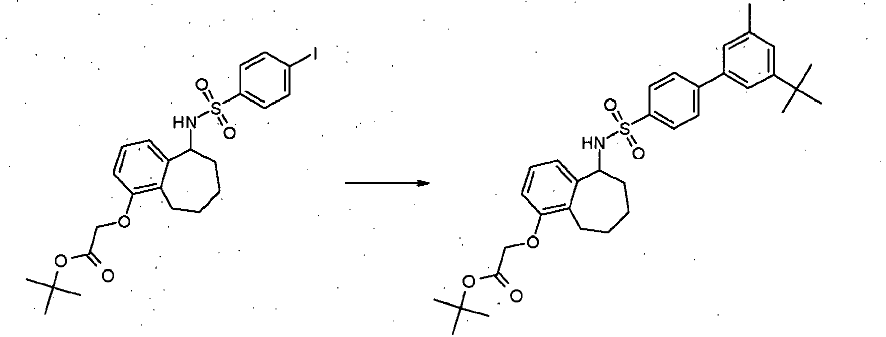

Using the conditions of General Procedure 4, [5-(4-iodo-benzenesulfonylamino)-6,7,8,9-tetrahydro-5H-benzocyclohepten-1-yloxy]-acetic acid tert-butyl ester (which may be prepared as described for Intermediate 1.08; 300 mg, 0.54 mmol) was reacted with (3-t-butyl-5-methylphenyl)boronic acid (available from Combi-Blocks Inc., 7949 Silverton Avenue, Suite 915, San Diego, CA 92126, USA; 124 mg, 0.65 mmol) in the presence of Pd(PPh₃)₄ (available from Aldrich Chemical Company, Inc., 1001 West Saint Paul Avenue, Milwaukee, WI 53233, USA; 60 mg, 0.05 mmol), and 1 M aqueous K₂CO₃ (0.8 mL, 0.8 mmol) in dioxane (7 mL) to give [5-(3'-tert-butyl-5'-methyl-biphenyl-4-sulfonylamino)-6,7,8,9-tetrahydro-5H-benzocyclohepten-1-yloxy]-acetic acid tert-butyl ester (230 mg, 74%). ¹H NMR (400 MHz, DMSO-*d*₆) δ: 8.22 (d, *J*=7.8 Hz, 1 H), 7.76-7.88 (m, 4 H), 7.47 (s, 1 H), 7.34 (s, 1 H), 7.28 (s, 1 H), 6.95-7.05 (m, 1 H), 6.87 (d, *J*=7.6 Hz, 1 H), 6.66 (d, *J*=8.1 Hz, 1 H), 4.57 (s, 2 H), 4.44-4.48 (m, 1 H), 3.12-3.25 (m, 1 H), 2.38 (s, 3 H), 1.45-1.79 (m, 6 H), 1.40 (s, 9 H), 1.33 (s, 9 H), 1.21-1.28 (m, 3 H).

### Intermediate 2.14

### [5-(4'-Hydroxy-biphenyl-4-sulfonylamino)-6,7,8,9-tetrahydro-5H-benzocyclohepten-1-yloxy]-acetic acid tert-butyl ester

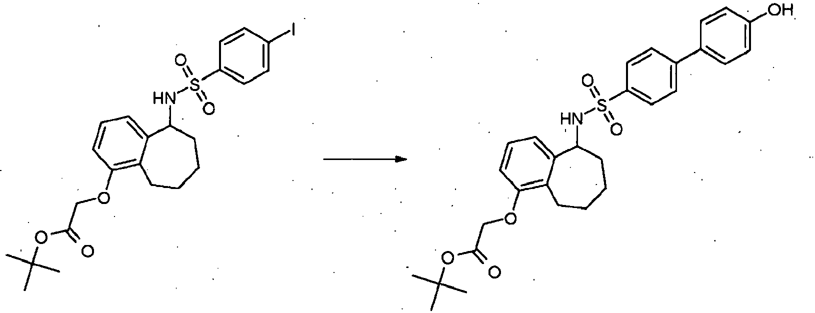

Using the conditions of General Procedure 4, [5-(4-iodo-benzenesulfonylamino)-6,7,8,9-tetrahydro-5H-benzocyclohepten-1-yloxy]-acetic acid tert-butyl ester (which may be prepared as described for Intermediate 1.08; 200 mg, 0.36 mmol) was reacted with 4-hydroxyphenylboronic acid (available from Aldrich Chemical Company, Inc., 1001 West Saint Paul Avenue, Milwaukee, WI 53233, USA; 58 mg, 0.42 mmol) in the presence of Pd(PPh₃)₄ (available from Aldrich Chemical Company, Inc., 1001 West Saint Paul Avenue, Milwaukee, WI 53233, USA; 40 mg, 0.035 mmol), and 1 M aqueous K₂CO₃ (0.6 mL, 0.6 mmol) in dioxane (5 mL) to give [5-(4'-hydroxy-biphenyl-4-sulfonylamino)-6,7,8,9-tetrahydro-5H-benzocyclohepten-1-yloxy]-acetic acid tert-butyl ester (120 mg, 64%).

### Intermediate 2.15

### {5-[4-(5-Methyl-pyridin-3-yl)-benzenesulfonylamino]-6,7,8,9-tetrahydro-5H-benzocyclohepten-1-yloxy}-acetic acid tert-butyl ester

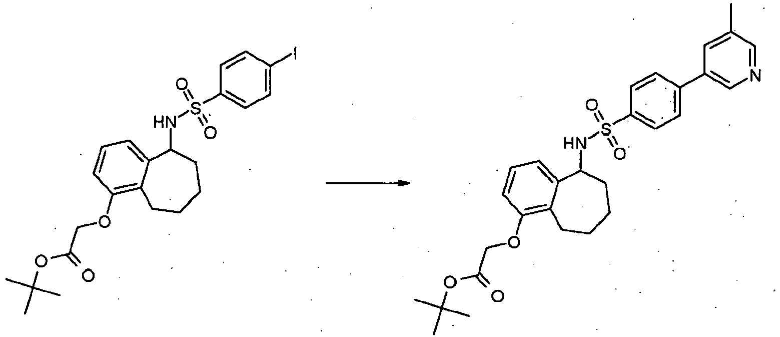

Using the conditions of General Procedure 4, [5-(4-iodo-benzenesulfonylamino)-6,7,8,9-tetrahydro-5H-benzocyclohepten-1-yloxy]-acetic acid tert-butyl ester (which may be prepared as described for Intermediate 1.08; 300 mg, 0.54 mmol) was reacted with 5-methylpyridine-3-boronic acid (available from Combi-Blocks Inc., 7949 Silverton Avenue, Suite 915, San Diego, CA 92126, USA; 89 mg, 0.65 mmol) in the presence of Pd(PPh₃)₄ (available from Aldrich Chemical Company, Inc., 1001 West Saint Paul Avenue, Milwaukee, WI 53233, USA; 60 mg, 0.05 mmol), and 1 M aqueous K₂CO₃ (0.8 mL, 0.8 mmol) in dioxane (20 mL) to give {5-[4-(5-methyl-pyridin-3-yl)-benzenesulfonylamino]-6,7,8,9-tetrahydro-5H-benzocyclohepten-1-yloxy}-acetic acid tert-butyl ester (195 mg, 69%).

### Intermediate 2.16

### [5-(3'-Methylsulfanyl-biphenyl-4-sulfonylamino)-6,7,8,9-tetrahydro-5H-benzocyclohepten-1-yloxy]-acetic acid tert-butyl ester

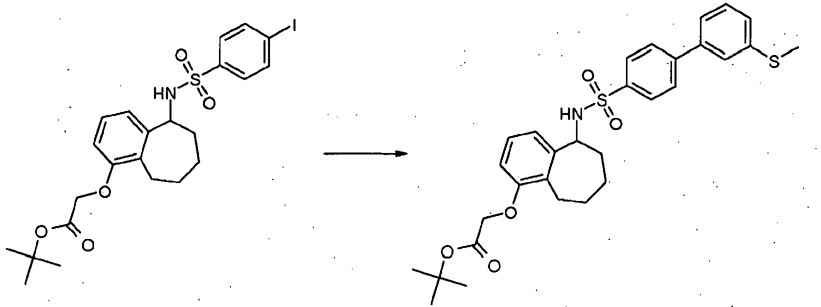

Using the conditions of General Procedure 4, [5-(4-iodo-benzenesulfonylamino)-6,7,8,9-tetrahydro-5H-benzocyclohepten-1-yloxy]-acetic acid tert-butyl ester (which may be prepared as described for Intermediate 1.08; 300 mg, 0.54 mmol) was reacted with 3-(methylthio)phenylboronic acid (available from Aldrich Chemical Company, Inc., 1001 West Saint Paul Avenue, Milwaukee, WI 53233, USA; 109 mg, 0.65 mmol) in the presence of Pd(PPh₃)₄ (available from Aldrich Chemical Company, Inc., 1001 West Saint Paul Avenue, Milwaukee, WI 53233, USA; 60 mg, 0.05 mmol), and 1 M aqueous K₂CO₃ (0.8 mL, 0.8 mmol) in dioxane (7 mL) to give [5-(3'-methylsulfanyl-biphenyl-4-sulfonylamino)-6,7,8,9-tetrahydro-5H-benzocyclohepten-1-yloxy]-acetic acid tert-butyl ester (195 mg, 65%). ¹H NMR (400 MHz, DMSO-*d*₆) 8: 8.24 (d, *J*=7.8 Hz, 1 H), 7.85 (s, 4 H), 7.55 (s, 1 H), 7.41-7.51 (m, 2 H), 7.33 (d, *J*=7.6 Hz, 1 H), 6.96-7.02 (m, 1 H), 6.86 (d, *J*=7.6 Hz, 1 H), 6.67 (d, *J*=8.1 Hz, 1 H), 4.57 (s, 2 H), 4.45-4.49 (m, 1 H), 3.16-3.22 (m, 1 H), 2.56 (s, 3 H), 1.45-1.78 (m, 6 H), 1.40 (s, 9 H), 1.21-1.29 (m, 4 H).

### Intermediate 2.17

### [5-(3'-Methanesulfonyl-biphenyl-4-sulfonylamino)-6,7,8,9-tetrahydro-5H-benzocyclohepten-1-yloxy]-acetic acid tert-butyl ester

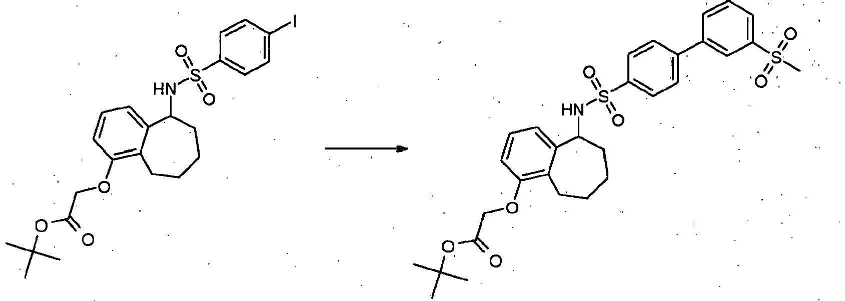

Using the conditions of General Procedure 4, [5-(4-iodo-benzenesulfonylamino)-6,7,8,9-tetrahydro-5H-benzocyclohepten-1-yloxy]-acetic acid tert-butyl ester (which may be prepared as described for Intermediate 1.08; 300 mg, 0.54 mmol) was reacted with 3-(methylsulfonyl)phenylboronic acid (available from Combi-Blocks Inc., 7949 Silverton Avenue, Suite 915, San Diego, CA 92126, USA; 129 mg, 0.65 mmol) in the presence of Pd(PPh₃)₄ (available from Aldrich Chemical Company, Inc., 1001 West Saint Paul Avenue, Milwaukee, WI 53233, USA; 60 mg, 0.05 mmol), and 1 M aqueous K₂CO₃ (0.8 mL, 0.8 mmol) in dioxane (7 mL) to give [5-(3'-methanesulfonyl-biphenyl-4-sulfonylamino)-6,7,8,9-tetrahydro-5H-benzocyclohepten-1-yloxy]-acetic acid tert-butyl ester (200 mg, 63%). ¹H NMR (400 MHz, DMSO-*d*₆) δ: 8.28 (d, *J*=7.8 Hz, 1 H), 8.22 (s, 1 H), 8.10 (d, *J*=7.8 Hz, 1 H), 7.88-8.01 (m, 5 H), 7.77-7.82 (m, 1 H), 6.97-7.03 (m, 1 H), 6.86 (d, *J*=7.8 Hz, 1 H), 6.67 (d, *J*=7.8 Hz, 1 H), 4.57 (s, 2 H), 4.46-4.52 (m, 1 H), 1.46-1.77 (m, 5 H), 1.40 (s, 9 H), 1.21-1.29 (m, 2 H).

### Intermediate 2.18

### {5-[5-(3-Isopropyl-phenyl)-pyridine-2-sulfonylamino]-6,7,8,9-tetrahydro-5H-benzocyclohepten-1-yloxy}-acetic acid tert-butyl ester

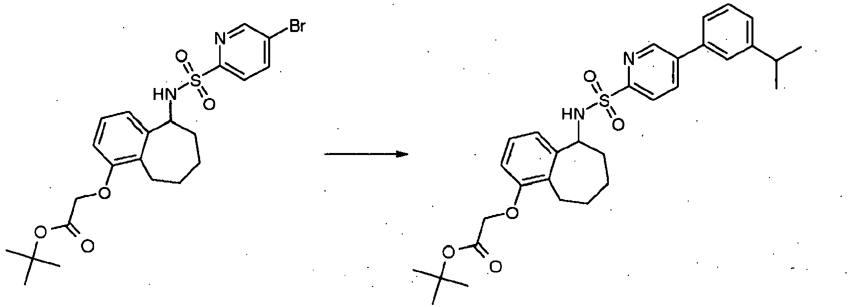

Using the conditions of General Procedure 5, [5-(5-bromo-pyridine-2-sulfonylamino)-6,7,8,9-tetrahydro-5H-benzocyclohepten-1-yloxy]-acetic acid tert-butyl ester (which may be prepared as described for Intermediate 1.09; 500 mg, 0.98 mmol) was reacted with 3-isopropylphenylboronic acid (available from Alfa Aesar, 26 Parkridge Road, Ward Hill, MA 01835, USA; 0.192 g, 1.17 mmol), in the presence of Pd(PPh₃)₄ (available from Aldrich Chemical Company, Inc., 1001 West Saint Paul Avenue, Milwaukee, WI 53233, USA; 80 mg, 0.07 mmol), and 1 M aqueous K₂CO₃ (1.5 mL, 1.5 mmol) in dioxane to give {5-[5-(3-isopropyl-phenyl)-pyridine-2-sulfonylamino]-6,7,8,9-tetrahydro-5H-benzocyclohepten-1-yloxy}-acetic acid tert-butyl ester (450 mg, 84%).

### Intermediate 2.19

### {5-[5-(3-Trifluoromethyl-phenyl)-pyridine-2-sulfonylamino]-6,7,8,9-tetrahydro-5H-benzocyclohepten-1-yloxy}-acetic acid tert-butyl ester

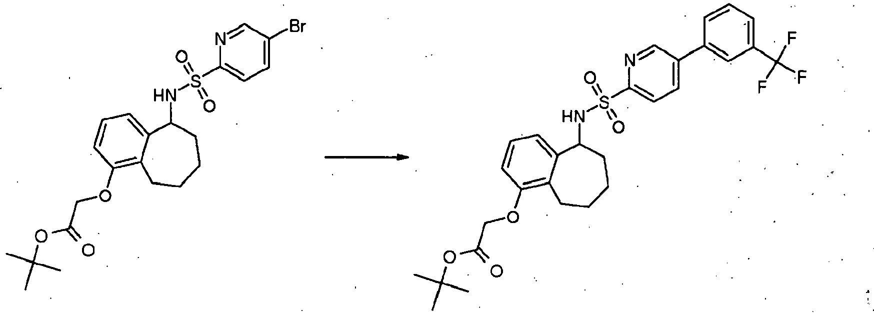

Using the conditions of General Procedure 5, [5-(5-bromo-pyridine-2-sulfonylamino)-6,7,8,9-tetrahydro-5H-benzocyclohepten-1-yloxy]-acetic acid tert-butyl ester (which may be prepared as described for Intermediate 1.09; 150 mg, 0.29 mmol) was reacted with 3-(trifluoromethyl)phenylboronic acid (available from Aldrich Chemical Company, Inc., 1001 West Saint Paul Avenue, Milwaukee, WI 53233, USA; 65 mg, 0.34 mmol), in the presence of Pd(PPh₃)₄ (available from Aldrich Chemical Company, Inc., 1001 West Saint Paul Avenue, Milwaukee, WI 53233, USA; 30 mg, 0.03 mmol), and 1 M aqueous K₂CO₃ (0.6 mL, 0.6 mmol) in dioxane (5 mL) to give {5-[5-(3-trifluoromethyl-phenyl)-pyridine-2-sulfonylamino]-6,7,8,9-tetrahydro-5H-benzocyclohepten-1-yloxy}-acetic acid tert-butyl ester (185 mg, crude yield: 109%). This material was used directly in the next step without further purification.

### Intermediate 2.20

### {5-[5-(3-tert-Butyl-5-methyl-phenyl)-pyridine-2-sulfonylamino]-6,7,8,9-tetrahydro-5H-benzocyclohepten-1-yloxy}-acetic acid tert-butyl ester

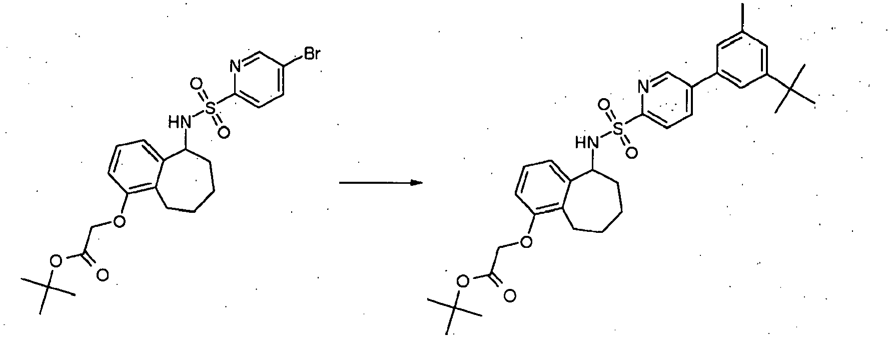

Using the conditions of General Procedure 5, [5-(5-bromo-pyridine-2-sulfonylamino)-6,7,8,9-tetrahydro-5H-benzocyclohepten-1-yloxy]-acetic acid tert-butyl ester (which may be prepared as described for Intermediate 1.09; 500 mg, 0.98 mmol) was reacted with (3-t-butyl-5-methylphenyl)boronic acid (available from Combi-Blocks Inc., 7949 Silverton Avenue, Suite 915, San Diego, CA 92126, USA; 220 mg, 1.15 mmol), in the presence of Pd(PPh₃)₄ (available from Aldrich Chemical Company, Inc., 1001 West Saint Paul Avenue, Milwaukee, WI 53233, USA; 80 mg, 0.07 mmol), and 1 M aqueous K₂CO₃ (1.5 mL, 1.5 mmol) to give {5-[5-(3-tert-butyl-5-methyl-phenyl)-pyridine-2-sulfonylamino]-6,7,8,9-tetrahydro-5H-benzocyclohepten-1-yloxy}-acetic acid tert-butyl ester (470 mg, 83%).

### Intermediate 2.21

### (5-{5-[3-(2-Hydroxy-ethyl)-phenyl]-pyridine-2-sulfonylamino}-6,7,8,9-tetrahydro-5H-benzocyclohepten-1-yloxy)-acetic acid tert-butyl ester

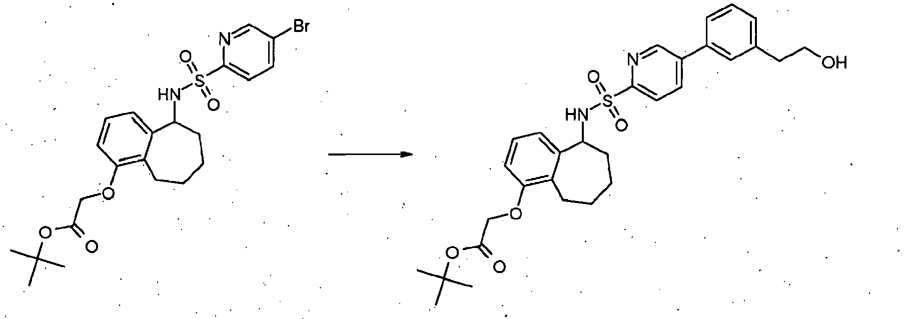

Using the conditions of General Procedure 5, [5-(5-bromo-pyridine-2-sulfonylamino)-6,7,8,9-tetrahydro-5H-benzocyclohepten-1-yloxy]-acetic acid tert-butyl ester (which may be prepared as described for Intermediate 1.09; 500 mg, 0.98 mmol) was reacted with 3-(2-hydroxyethyl)benzeneboronic acid (available from Combi-Blocks Inc., 7949 Silverton Avenue, Suite 915, San Diego, CA 92126, USA; 195 mg, 1.17 mmol), in the presence of Pd(PPh₃)₄ (available from Aldrich Chemical Company, Inc., 1001 West Saint Paul Avenue, Milwaukee, WI 53233, USA; 80 mg, 0.07 mmol), and 1 M aqueous K₂CO₃ (1.5 mL, 1.5 mmol) to give (5-{5-[3-(2-hydroxy-ethyl)-phenyl]-pyridine-2-sulfonylamino}-6,7,8,9-tetrahydro-5H-benzocyclohepten-1-yloxy)-acetic acid tert-butyl ester (480 mg, 89%).

### Intermediate 2.22

### [5-(4'-Methyl-biphenyl-3-sulfonylamino)-6,7,8,9-tetrahydro-5H-benzocyclohepten-1-yloxy]-acetic acid tert-butyl ester

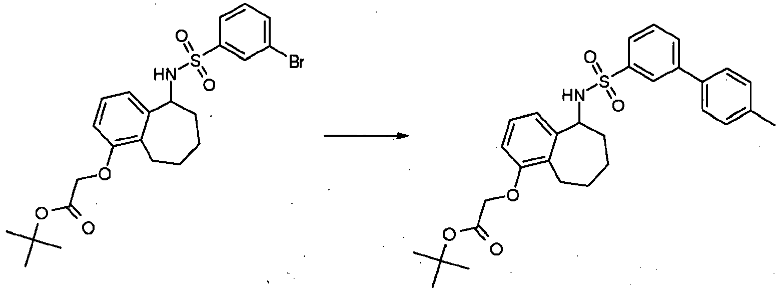

Using the conditions of General Procedure 6, [5-(3-bromo-benzenesulfonylamino)-6,7,8,9-tetrahydro-5H-benzocyclohepten-1-yloxy]-acetic acid tert-butyl ester (which may be prepared as described for Intermediate 1.10; 450 mg, 0.88 mmol) was reacted with 4-methylphenylboronic acid (available from Aldrich Chemical Company, Inc., 1001 West Saint Paul Avenue, Milwaukee, WI 53233, USA; 0.213 g, 1.57 mmol), in the presence of Pd(PPh₃)₄ (available from Aldrich Chemical Company, Inc., 1001 West Saint Paul Avenue, Milwaukee, WI 53233, USA; 40 mg, 0.03 mmol), and 1 M aqueous K₂CO₃ (1.35 mL, 1.35 mmol) in dioxane (7 mL) to give [5-(4'-methylbiphenyl-3-sulfonylamino)-6,7,8,9-tetrahydro-5H-benzocyclohepten-1-yloxy]-acetic acid tert-butyl ester (350 mg, 76%).

### Intermediate 2.23

### [5-(3'-Isopropyl-biphenyl-3-sulfonylamino)-6,7,8,9-tetrahydro-5H-benzocyclohepten-1-yloxy]-acetic acid tert-butyl ester

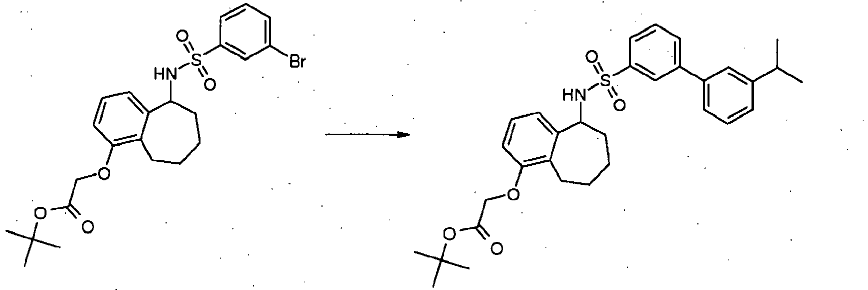

Using the conditions of General Procedure 6, [5-(3-bromo-benzenesulfonylamino)-6,7,8,9-tetrahydro-5H-benzocyclohepten-1-yloxy]-acetic acid tert-butyl ester (which may be prepared as described for Intermediate 1.10; 200 mg, 0.4 mmol) was reacted with 3-isopropylphenylboronic acid (available from Aldrich Chemical Company, Inc., 1001 West Saint Paul Avenue, Milwaukee, WI 53233, USA; 80 mg, 0.5 mmol), in the presence of Pd(PPh₃)₄ (available from Aldrich Chemical Company, Inc., 1001 West Saint Paul Avenue, Milwaukee, WI 53233, USA; 20 mg, 0.02 mmol), and 1 M aqueous K₂CO₃ (1.5 mL, 1.5 mmol) in dioxane (5 mL) to give [5-(3'-isopropyl-biphenyl-3-sulfonylamino)-6,7,8,9-tetrahydro-5H-benzocyclohepten-1-yloxy]-acetic acid tert-butyl ester (125 mg, 58%).

### Intermediate 2.24

### [5-(3-Isopropyl-5-trifluoromethyl-benzenesulfonylamino)-6,7,8,9-tetrahydro-5H-benzocycloh-epten-1-yloxy]-acetic acid tert-butyl ester

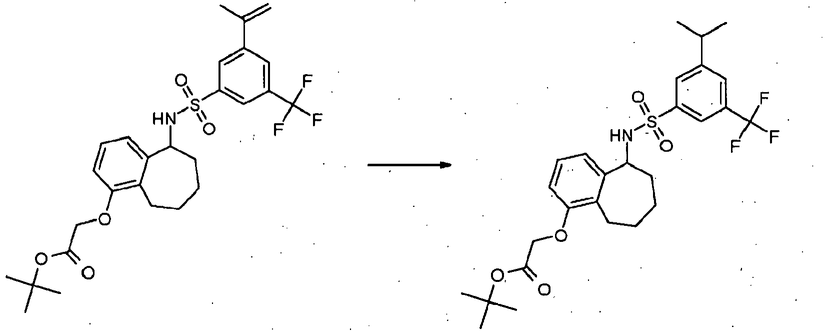

A mixture of [5-(3-isopropenyl-5-trifluoromethyl-benzenesulfonylamino)-6,7,8,9-tetrahydro-5H-benzocyclohepten-1-yloxy]-acetic acid tert-butyl ester (which may be prepared as described for Intermediate 1.07; 200 mg, 0.38 mmol) and 10% palladium-on-carbon (40 mg) in methanol was stirred for 16 h at room temperature under an atmosphere of hydrogen. The mixture was filtered through celite and the celite was washed with methanol. The filtrates were concentrated to give [5-(3-isopropyl-5-trifluoromethyl-benzenesulfonylamino)-6,7,8,9-tetrahydro-5H-benzocyclohepten-1-yloxy]-acetic acid tert-butyl ester (150 mg, 75%) as a white solid. ¹H NMR (400 MHz, DMSO-*d*₆) δ: 8.35 (br. s., 1 H), 7.89 (s, 1 H), 7.77 (s, 2 H), 6.90 (t, *J*=7.8 Hz, 1 H), 6.72 (d, *J*=7.3 Hz, 1 H), 6.61 (d, *J*=8.3 Hz, 1 H), 4.47-4.56 (m, 3 H), 3.01-3.12 (m, 2 H), 2.59-2.68 (m, 1 H), 1.46-1.83 (m, 5 H), 1.41 (s, 9 H), 1.27-1.35 (m, 1 H), 1.16-1.24 (m, 6 H).

### Intermediate 3.01

### {5-[(3-Fluoro-5-trifluoromethyl-benzenesulfonyl)-methyl-amino]-6,7,8,9-tetrahydro-5H-benzocyclohepten-1-yloxy}-acetic acid tert-butyl ester

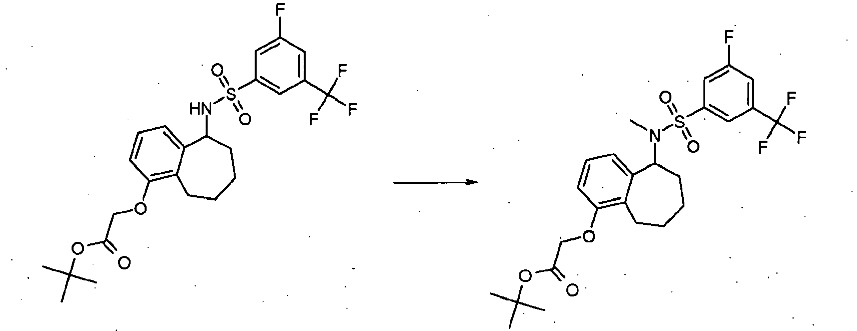

A mixture of [5-(3-fluoro-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.06; 0.30 g, 0.6 mmol), methyl iodide (0.091 g, 0.64 mmol) and K₂CO₃ (88 mg, 0.64 mmol) in DMF (2 mL) was stirred for 20 h at room temperature. Ice-water (6 mL) was added and the mixture was extracted with EtOAc (2 x 15 mL). The organic layers were dried over anhydrous Na₂SO₄, filtered, evaporated, and purified by silica gel chromatography, using 5-8% EtOAc/hexanes as eluent, to give {5-[(3-fluoro-5-trifluoromethyl-benzenesulfonyl)-methyl-amino]-6,7,8,9-tetrahydro-5H-benzocyclohepten-1-yloxy}-acetic acid tert-butyl ester (0.26 g, 84%). ¹H NMR (400 MHz), DMSO-*d*₆) δ: 8.13-88.17 (m, 2 H), 8.00 (s, 1 H), 7.08 (t, *J*=7.9 Hz, 1 H), 6.75 (dd, *J*=7.7, 2.8 Hz, 2 H), 5.21 (d, *J*=8.6 Hz, 1 H), 4.64 (s, 2 H), 4.59 (s, 1 H), 3.45 (dd, *J*=14.1, 6.7 Hz, 1 H), 2.88 (s, 3 H), 2.39-2.45 (m, 1 H), 1.51-1.90 (m, 4 H), 1.42 (s, 9 H), 1.23 (s, 4 H), 1.05 (br. s., 1 H).

### Intermediate 3.02

### {5-[(3-Bromo-5-trifluoromethyl-benzenesulfonyl)-methyl-amino]-6,7,8,9-tetrahydro-5H-benzocyclohepten-1-yloxy}-acetic acid tert-butyl ester

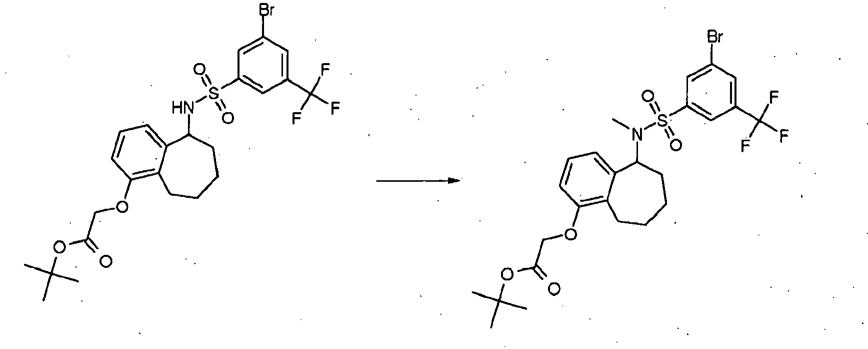

Using the conditions described for the preparation of Intermediate 3.20, [5-(3-bromo-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.07; 0.15 g, 0.26 mmol) was reacted with methyl iodide (0.034 mL, 0.55 mmol) in the presence of K₂CO₃ (79 mg, 0.57 mmol) to give {5-[(3-bromo-5-trifluoromethyl-benzenesulfonyl)-methyl-amino]-6,7,8,9-tetrahydro-5H-benzocyclohepten-1-yloxy}-acetic acid tert-butyl ester (150 mg, 98%). ¹H NMR (400 MHz, CDCl₃) δ: 8.11 (s, 1 H), 8.00 (s, 2 H), 7.95 (s, 1 H), 7.92 (s, 1 H), 7.00 (t, *J*=7.8 Hz, 1 H), 6.65 (d, *J*=7.8 Hz, 1 H), 6.62 (d, *J*=8.3 Hz, 1 H), 5.29 (d, *J*=9.8 Hz, 1 H), 4.48 (s, 2 H), 3.56 (dd, *J*=14.4, 7.1 Hz, 1 H), 2.94 (s, 8 H), 2.87 (s, 8 H), 2.42 (t, *J*=13.0 Hz, 1 H), 1.89-1.96 (m, 2 H), 1.52-1.69 (m, 8 H), 1.47 (s, 9 H), 1.21-1.31 (m, 4 H).

### Intermediate 3.03

### {5-[(3,5-Bis-methanesulfonyl-benzenesulfonyl)-methyl-amino]-6,7,8,9-tetrahydro-5H-benzocyclohepten-1-yloxy}-acetic acid tert-butyl ester

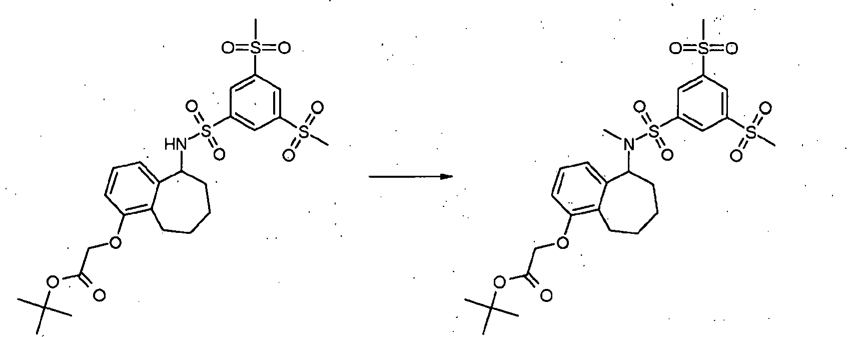

Using the conditions of General Procedure 3, [5-(3,5-bis-methanesulfonyl-benzenesulfonylamino)-6,7,8,9-tetrahydro-5H-benzocyclohepten-1-yloxy]-acetic acid tert-butyl ester (which may be prepared as described for Intermediate 2.08; 0.15 g, 0.26 mmol) was reacted with methyl iodide (72.5 mg, 0.51 mmol) in the presence of K₂CO₃ (72.5 mg, 0.51 mmol) in DMF (2 mL) to give {5-[(3,5-bis-methanesulfonyl-benzenesulfonyl)-methyl-amino]-6,7,8,9-tetrahydro-5H-benzocyclohepten-1-yloxy}-acetic acid tert-butyl ester (110 mg, 72%). ¹H NMR (400 MHz, DMSO-*d*₆) δ: 8.66 (s, 1 H), 8.63 (s, 2 H), 7.05-7.11 (m, 1 H), 6.72-6.76 (m, 2 H), 5.30 (d, *J*=8.8 Hz, 1 H), 4.64 (s, 2 H), 3.46 (s, 6 H), 2.91 (s, 3 H), 1.42 (s, 9 H), 1.20-1.30 (m, 8 H).

### Intermediate 3.04

### {5-[(Biphenyl-4-sulfonyl)-methyl-amino]-6,7,8,9-tetrahydro-5H-benzocyclohepten-1-yloxy}-acetic acid tert-butyl ester

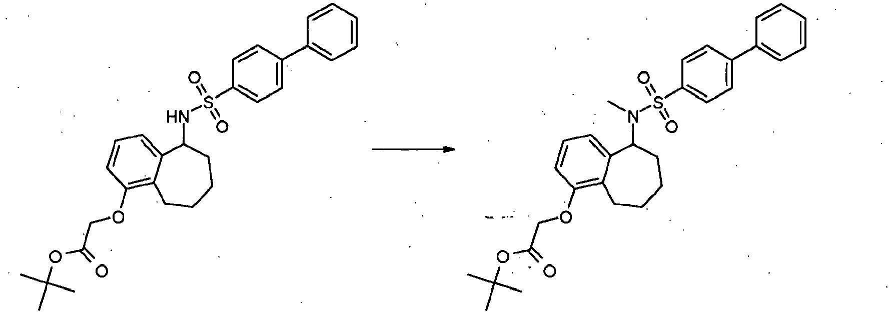

Using the conditions of General Procedure 3, [5-(biphenyl-4-sulfonylamino)-6,7,8,9-tetrahydro-5H-benzocyclohepten-1-yloxy]-acetic acid tert-butyl ester,(which may be prepared as described for Intermediate 2.09; 0.15 g, 0.30 mmol) was reacted with methyl iodide (84 mg, 0.59 mmol) in the presence of K₂CO₃ (81.5 mg, 0.59 mmol) in DMF (2 mL) to give {5-[(biphenyl-4-sulfonyl)-methyl-amino]-6,7,8,9-tetrahydro-5H-benzocyclohepten-1-yloxy}-acetic acid tert-butyl ester (100 mg, 65%). This material was used directly in the next step without characterization.

### Intermediate 3.05

### {5-[(3-Acetyl-5-trifluoromethyl-benzenesulfonyl)-methyl-amino]-6,7,8,9-tetrahydro-5H-benzocyclohepten-1-yloxy}-acetic acid tert-butyl ester

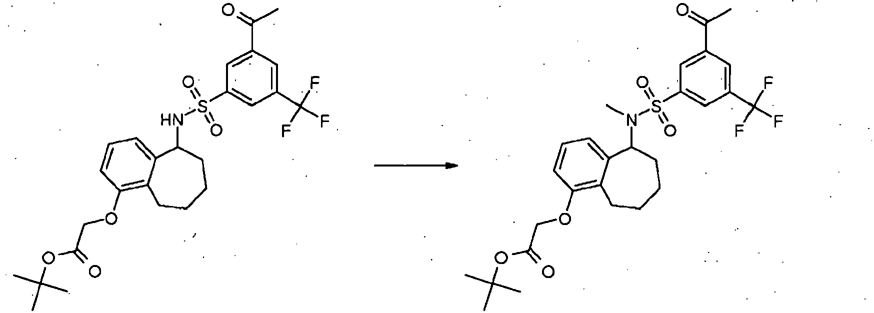

Using the conditions of General Procedure 3, [5-(3-acetyl-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.10; 0.15 g, 0.28 mmol) was reacted with methyl iodide (79 mg, 0.56 mmol) in the presence of K₂CO₃ (76.6 mg, 0.55 mmol) in DMF (2 mL) to give {5-[(3-acetyl-5-trifluoromethyl-benzenesulfonyl)-methyl-amino]-6,7,8,9-tetrahydro-5H-benzocyclohepten-1-yloxy}-acetic acid tert-butyl ester (150 mg, 97%). This material was used directly in the next step without characterization.

### Intermediate 3.06

### {5-[(3-Methanesulfonyl-5-trifluoromethyl-benzenesulfonyl)-methyl-amino]-6,7,8,9-tetrahydro-5H-benzocyclohepten-1-yloxy}-acetic acid tert-butyl ester

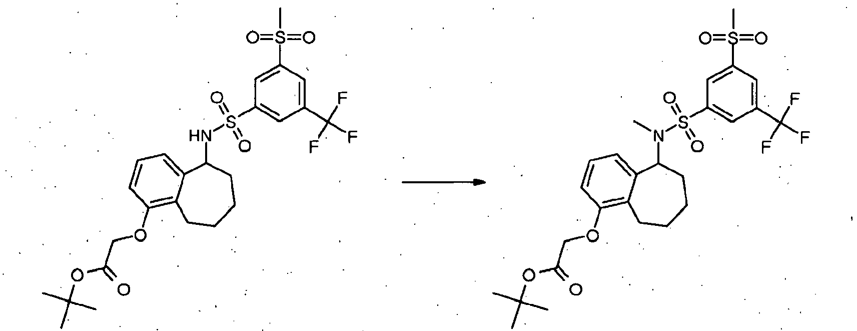

Using the conditions of General Procedure 3, [5-(3-methanesulfonyl-5-trifluoromethyl-benzenesulfonylamino)-6,7,8,9-tetrahydro-5H-benzocyclohepteh-1-yloxy]-acetic acid tert-butyl ester (which may be prepared as described for Intermediate 2.11; 80 mg, 0.14 mmol) was reacted with methyl iodide (40 mg, 0.28 mmol) in the presence of K₂CO₃ (39 mg, 0.28 mmol) in DMF (2 mL) to give {5-[(3-methanesulfonyl-5-trifluoromethyl-benzenesulfonyl)-methyl-amino]-6,7,8,9-tetrahydro-5H-benzocyclohepten-1-yloxy}-acetic acid tert-butyl ester (80 mg, 98%). This material was used directly in the next step without characterization.

### Intermediate 3.07

### {5-[(3'-Isopropyl-biphenyl-4-sulfonyl)-methyl-amino]-6,7,8,9-tetrahydro-5H-benzocyclohepten-1-yloxy}-acetic acid tert-butyl ester

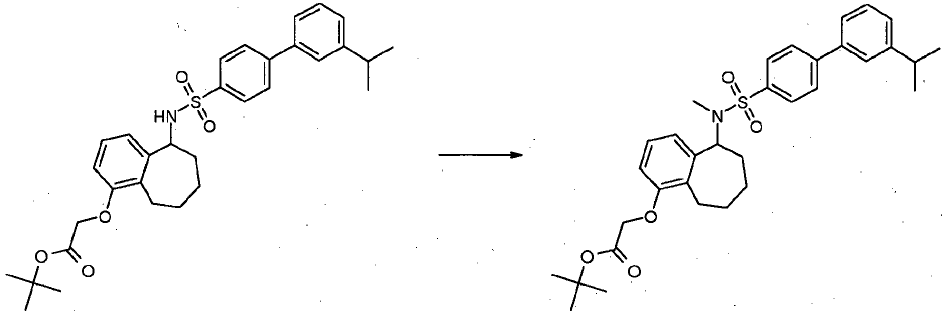

Using the conditions of General Procedure 3, [5-(3'-isopropyl-biphenyl-4-sulfonylamino)-6,7,8,9-tetrahydro-5H-benzocyclohepten-1-yloxy]-acetic acid tert-butyl ester (which may be prepared as described for Intermediate 2.12; 100 mg, 0.18 mmol) was reacted with methyl iodide (29 mg, 0.2 mmol) in the presence of K₂CO₃ (28 mg, 0.2 mmol) in DMF to give {5-[(3'-isopropyl-biphenyl-4-sulfonyl)-methyl-amino]-6,7,8,9-tetrahydro-5H-benzocyclohepten-1-yloxy}-acetic acid tert-butyl ester (70 mg, 68%). ¹H NMR (400 MHz, DMSO-*d*₆) δ: 7.98-8.05 (m, 2 H), 7.89 (d, *J*=7.8 Hz, 1 H), 7.71-7.77 (m, 1 H), 7.50-7.59 (m, 2 H), 7.41-7.47 (m, 1 H), 7.33 (d, *J*=7.8 Hz, 1 H), 7.10 (t, *J*=7.8 Hz, 1 H), 6.83 (d, *J*=7.8 Hz, 1 H), 6.74 (d, *J*=8.3 Hz, 1 H), 5.21 (br. s., 1 H), 4.63 (s, 2 H), 3.44-3.52 (m, 1 H), 2.96-3.04 (m, 1 H), 2.87 (s, 3 H), 1.70-1.80 (m, 1 H), 1.42 (s, 9 H), 1.15-1.28 (m, 8 H).

### Intermediate 3.08

### {5-[(3'-tert-Butyl-5'-methyl-biphenyl-4-sulfonyl)-methyl-amino]-6,7,8,9-tetrahydro-5H-benzocyclohepten-1-yloxy}-acetic acid tert-butyl ester

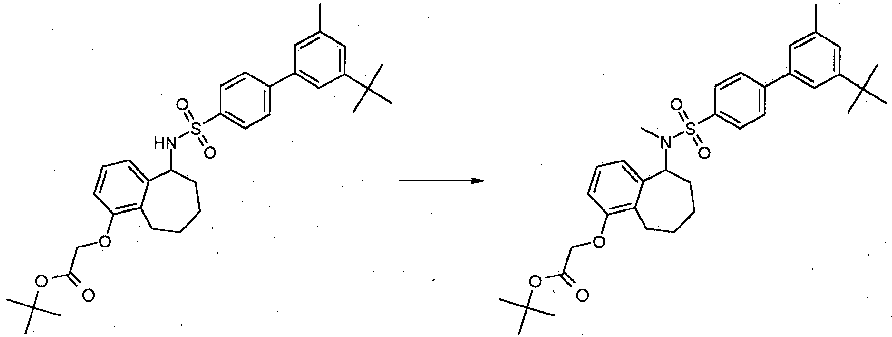

Using the conditions of General Procedure 3, [5-(3'-tert-butyl-5'-methyl-biphenyl-4-sulfonylamino)-6,7,8,9-tetrahydro-5H-benzocyclohepten-1-yloxy]-acetic acid tert-butyl ester (which may be prepared as described for Intermediate 2.13; 100 mg, 0.19 mmol) was reacted with methyl iodide (27 mg, 0.19 mmol) in the presence of K₂CO₃ (26 mg, 0.19 mmol) in DMF (2 mL) to give {5-[(3'-tert-butyl-5'-methyl-biphenyl-4-sulfonyl)-methyl-amino]-6,7,8,9-tetrahydro-5H-benzocyclohepten-1-yloxy}-acetic acid tert-butyl ester (45 mg, 44%).

### Intermediate 3.09

### {5-[(4'-Hydroxy-biphenyl-4-sulfonyl)-methyl-amino]-6,7,8,9-tetrahydro-5H-benzocyclohepten-1-yloxy}-acetic acid tert-butyl ester

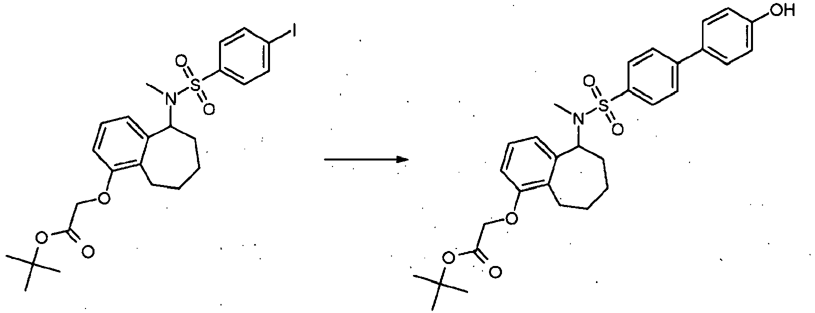

Using the conditions of General Procedure 4, {5-[(4-Iodo-benzenesulfonyl)-methyl-amino]-6,7,8,9-tetrahydro-5H-benzocyclohepten-1-yloxy}-acetic acid tert-butyl ester (which may be prepared as described for Intermediate 3.20; 200 mg, 0.35 mmol) was reacted with 4-hydroxyphenylboronic acid (available from Aldrich Chemical Company, Inc., 1001 West Saint Paul Avenue, Milwaukee, WI 53233, USA; 58 mg, 0.42 mmol) in the presence of Pd(PPh₃)₄ (available from Aldrich Chemical Company, Inc., 1001 West Saint Paul Avenue, Milwaukee, WI 53233, USA; 40 mg, 0.035 mmol), and 1 M aqueous K₂CO₃ (0.6 mL, 0.6 mmol) to give {5-[(4'-hydroxy-biphenyl-4-sulfonyl)-methyl-amino]-6,7,8,9-tetrahydro-5H-benzocyclohepten-1-yloxy}-acetic acid tert-butyl ester (100 mg, 52%).

### Intermediate 3.10

### (5-{Methyl-[4-(5-methyl-pyridin-3-yl)-benzenesulfonyl]-amino}-6,7,8,9-tetrahydro-5H-benzocyclohepten-1-yloxy)-acetic acid tert-butyl ester

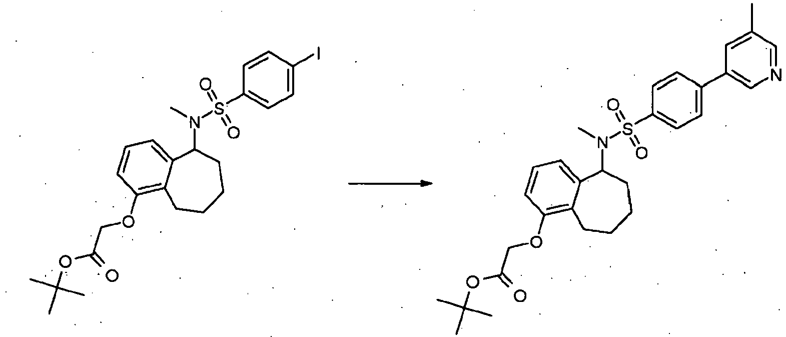

Using the conditions of General Procedure 4, {5-[(4-iodo-benzenesulfonyl)-methyl-amino]-6,7,8,9-tetrahydro-5H-benzocyclohepten-1-yloxy}-acetic acid tert-butyl ester (which may be prepared as described for Intermediate 3.20; 100 mg, 0.17 mmol) was reacted with 5-methylpyridine-3-boronic acid (available from Combi-Blocks Inc., 7949 Silverton Avenue, Suite 915, San Diego, CA 92126, USA; 29 mg, 0.21 mmol) in the presence of Pd(PPh₃)₄ (available from Aldrich Chemical Company, Inc., 1001 West Saint Paul Avenue, Milwaukee, WI 53233, USA; 10 mg, 0.01 mmol), and 1 M aqueous K₂CO₃ (1.5 mL, 1.5 mmol) in dioxane (5 mL) to give (5-{methyl-[4-(5-methyl-pyridin-3-yl)-benzenesulfonyl]-amino}-6,7,8,9-tetrahydro-5H-benzocyclohepten-1-yloxy)-acetic acid tert-butyl ester (50 mg, 53%). ¹H NMR (400 MHz, DMSO-*d*₆) δ: 8.79 (s, 1 H), 8.50 (s, 1 H), 7.96-8.07 (m, 5 H), 7.11 (t, *J*=8.1 Hz, 1 H), 6.84 (d, *J*=8.3 Hz, 1 H), 6.75 (d, *J*=7.8 Hz, 1 H), 5.14-5.19 (m, 1 H), 4.64 (s, 2 H), 3.44-3.52 (m, 1 H), 2.87 (s, 3 H), 2.64-2.70 (m, 1 H), 2.33-2.42 (m, 5 H), 1.42 (s, 9 H), 1.22-1.39 (m, 3 H).

### Intermediate 3.11

### {5-[Methyl-(3'-methylsulfanyl-biphenyl-4-sulfonyl)-amino]-6,7,8,9-tetrahydro-5H-benzocyclohepten-1-yloxy}-acetic acid tert-butyl ester

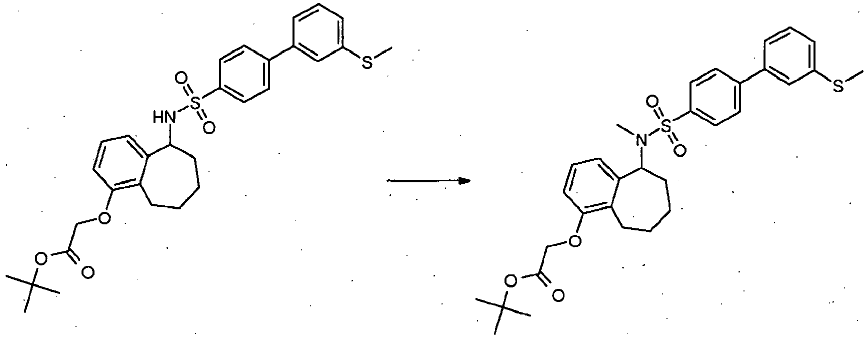

Using the conditions of General Procedure 3, [5-(3'-methylsulfanyl-biphenyl-4-sulfonylamino)-6,7,8,9-tetrahydro-5H-benzocyclohepten-1-yloxy]-acetic acid tert-butyl ester (which may be prepared as described for Intermediate 2.16; 100 mg, 0.18 mmol) was reacted with methyl iodide (29 mg, 0.20 mmol) in the presence of K₂CO₃ (28 mg, 0.20 mmol) in DMF (2 mL) to give {5-[methyl-(3'-methylsulfanyl-biphenyl-4-sulfonyl)-amino]-6,7,8,9-tetrahydro-5H-benzocyclohepten-1-yloxy}-acetic acid tert-butyl ester (45 mg, 42%).

### Intermediate 3.12

### {5-[(3'-Methanesulfonyl-biphenyl-4-sulfonyl)-methyl-amino]-6,7,8,9-tetrahydro-5H-benzocyclohepten-1-yloxy}-acetic acid tert-butyl ester

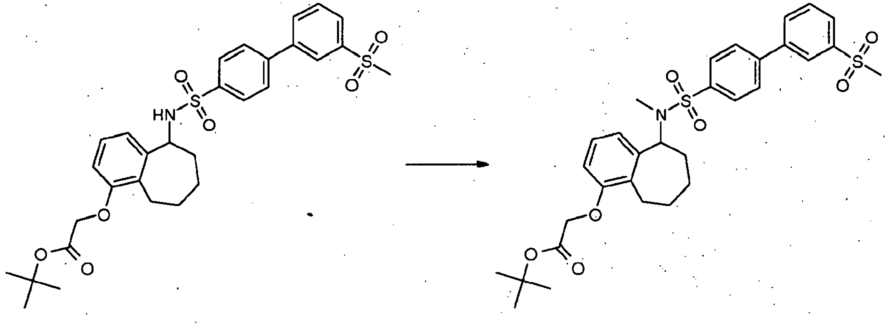

Using the conditions of General Procedure 3, [5-(3'-methanesulfonyl-biphenyl-4-sulfonylamino)-6,7,8,9-tetrahydro-5H-benzocyclohepten-1-yloxy]-acetic acid tert-butyl ester (which may be prepared as described for Intermediate 2.17; 100 mg, 0.17 mmol) was reacted with methyl iodide (46 mg, 0.33 mmol) in the presence of K₂CO₃ (45 mg, 0.33 mmol) in DMF (2 mL) to give {5-[(3'-methanesulfonyl-biphenyl-4-sulfonyl)-methyl-amino]-6,7,8,9-tetrahydro-5H-benzocyclohepten-1-yloxy}-acetic acid tert-butyl ester (98 mg, 96%).

### Intermediate 3.13

### (5-{[5-(3-Isopropyl-phenyl)-pyridine-2-sulfonyl]-methyl-amino}-6,7,8,9-tetrahydro-5H-benzocyclohepten-1-yloxy)-acetic acid tert-butyl ester

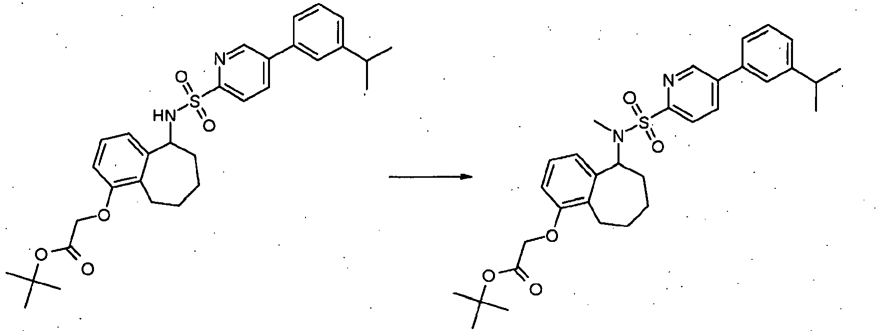

Using the conditions described for the preparation of Intermediate 3.14, {5-[5-(3-isopropyl-phenyl)-pyridine-2-sulfonylamino]-6,7,8,9-tetrahydro-5H-benzocyclohepten-1-yloxy}-acetic acid tert-butyl ester (which may be prepared as described for Intermediate 2.18; 120 mg, 0.22 mmol) was reacted with methyl iodide (34 mg, 0.24 mmol) in the presence of K₂CO₃ (33 mg, 0.24 mmol) in DMF to give (5-{[5-(3-isopropyl-phenyl)-pyridine-2-sulfonyl]-methyl-amino}-6,7,8,9-tetrahydro-5H-benzocyclohepten-1-yloxy)-acetic acid tert-butyl ester (90 mg, 73%). ¹H NMR (400 MHz, DMSO-*d*₆) δ: 9.13 (d, *J*=1.5 Hz, 1 H), 8.39 (dd, *J*=8.2, 2.1 Hz, 1 H), 8.03 (d, *J*=8.3 Hz, 1 H), 7.71 (s, 1 H), 7.65 (d, *J*=7.6 Hz, 1 H), 7.48 (t, *J*=7.7 Hz, 1 H), 7.36-7.41 (m, 1 H), 7.12 (t, *J*=8.1 Hz, 1 H), 6.87 (d, *J*=7.8 Hz, 1 H), 6.74 (d, *J*=8.3 Hz, 1 H), 5.27 (d, *J*=10.3 Hz, 1 H), 4.64 (s, 2 H), 3.92 (quin, *J*=7.3 Hz, 1 H), 2.99-3.05 (m, 1 H), 2.97 (s, 3 H), 2.24-2.34 (m, 1 H), 1.56-1.89 (m, 4 H), 1.42 (s, 9 H), 1.28 (d, *J*=6.8 Hz, 6 H), 1.04-1.12 (m, 1 H).

### Intermediate 3.14

### (5-{Methyl-[5-(3-trifluoromethyl-phenyl)-pyridine-2-sulfonyl]-amino}-6,7,8,9-tetrahydro-5H-benzocyclohepten-1-yloxy)-acetic acid tert-Butyl Ester

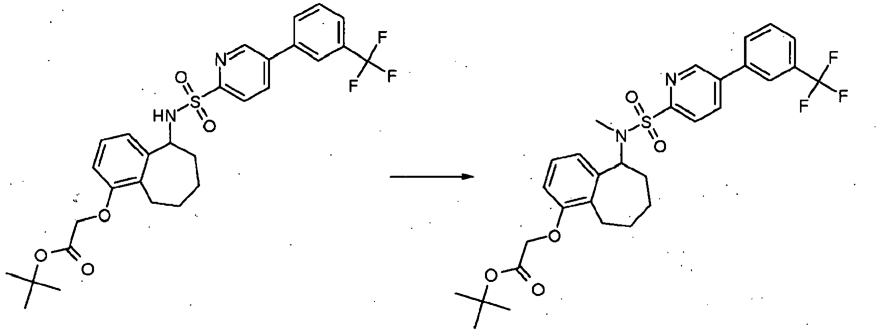

A mixture of {5-[5-(3-trifluoromethyl-phenyl)-pyridine-2-sulfonylamino]-6,7,8,9-tetrahydro-5H-benzocyclohepten-1-yloxy}-acetic acid tert-butyl ester (which may be prepared as described for Intermediate 2.19; 120 mg, 0.21 mmol), methyl iodide (33 mg, 0.23 mmol), and K₂CO₃ (32 mg, 0.23 mmol) in DMF (2 mL) was stirred at room temperature overnight. The reaction mixture was then partitioned between EtOAc and H₂O. The organic layer was concentrated and purified by column chromatography to give (5-{methyl-[5-(3-trifluoromethyl-phenyl)-pyridine-2-sulfonyl]-amino}-6,7,8,9-tetrahydro-5H-benzocyclohepten-1-yloxy)-acetic acid tert-butyl ester (75 mg, 61%). ¹H NMR (400 MHz, DMSO-d₆) 8: 9.22 (d, *J*=1.5 Hz, 1 H), 8.50 (dd, *J*=8.1, 2.0 Hz, 1 H), 8.22 (s, 1 H), 8.18 (d, *J*=7.6 Hz, 1 H), 8.07 (d, *J*=8.1 Hz, 1 H), 7.85-7.90 (m, 1 H), 7.78-7.83 (m, 1 H), 7.12 (t, *J*=7.9 Hz, 1 H), 6.87 (d, *J*=7.8 Hz, 1 H), 6.74 (d, *J*=8.3 Hz, 1 H), 5.28 (d, *J*=10.3 Hz, 1 H), 4.64 (s, 2 H), 3.49 (dd, *J*=13.8, 6.5 Hz, 1 H), 2.97 (s, 3 H), 2.67 (br. s., 1 H), 2.25-2.34 (m, 2 H), 1.57-1.91 (m, 4 H), 1.42 (s, 9 H), 1.21-1.37 (m, 2 H), 1.04-1.11 (m, 1H).

### Intermediate 3.15

### (5-{[5-(3-tert-Butyl-5-methyl-phenyl)-pyridine-2-sulfonyl]-methyl-amino}-6,7,8,9-tetrahydro-5H-benzocyclohepten-1-yloxy)-acetic acid tert-butyl ester

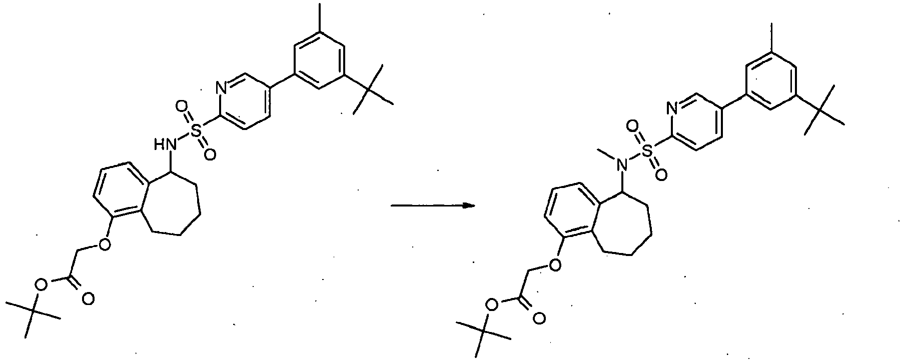

Using the conditions described for the preparation of Intermediate 3.14, {5-[5-(3-tert-butyl-5-methyl-phenyl)-pyridine-2-sulfonylamino]-6,7,8,9-tetrahydro-5H-benzocyclohepten-1-yloxy}-acetic acid tert-butyl ester (which may be prepared as described for Intermediate 2.20; 200 mg, 0.35 mmol) was reacted with methyl iodide (54 mg, 0.38 mmol) in the presence of K₂CO₃ (52.5 mg, 0.38 mmol) in DMF (3 mL) to give (5-{[5-(3-tert-butyl-5-methyl-phenyl)-pyridine-2-sulfonyl]-methyl-amino}-6,7,8,9-tetrahydro-5H-benzocyclohepten-1-yloxy)-acetic acid tert-butyl ester (150 mg, 73%). ¹H NMR (400 MHz, DMSO-*d*₆) δ: 9.11 (s, 1 H), 8.33-8.42 (m, 1 H), 8.02 (d, *J*=8.3 Hz, 1 H), 7.59 (s, 1 H), 7.46 (s, 1 H), 7.35 (s, 1 H), 7.12 (t, *J*=7.9 Hz, 1 H), 6.87 (d, *J*=7.8 Hz, 1 H), 6.74. (d, *J*=8.1 Hz, 1 H), 5.27 (d, *J*=10.3 Hz, 1 H), 4.64 (s, 2 H), 3.49 (dd, *J*=13.8, 6.7 Hz, 1 H), 2.96 (s, 3 H), 2.41 (s, 3 H), 2.21-2.35 (m, 1 H), 1.55-1.91 (m, 4 H), 1.42 (s, 9 H), 1.35 (s, 9 H), 1.03-1.12 (m, 2 H).

### Intermediate 3.16

### [5-({5-[3-(2-Hydroxy-ethyl)-phenyl]-pyridine-2-sulfonyl}-methyl-amino)-6,7,8,9-tetrahydro-5H-benzocyclohepten-1-yloxy]-acetic acid tert-butyl ester

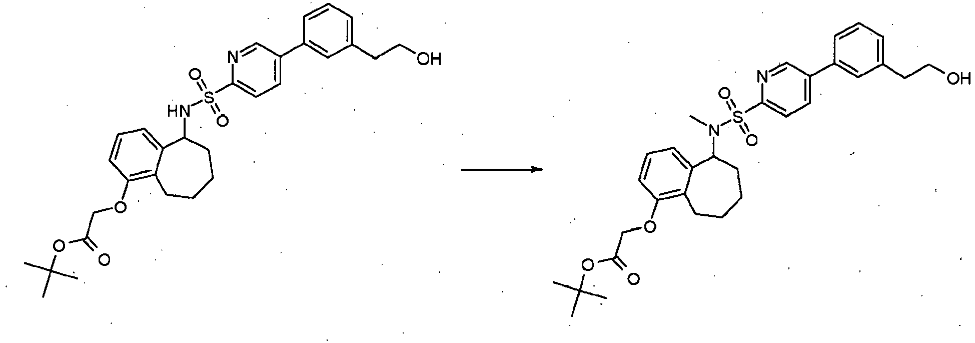

Using the conditions described for the preparation of Intermediate 3.14, {5-[5-(3-tert-butyl-5-methyl-phenyl)-pyridine-2-sulfonylamino]-6,7,8,9-tetrahydro-5H-benzocyclohepten-1-yloxy}-acetic acid tert-butyl ester (which may be prepared as described for Intermediate 2.21; 200 mg, 0.36 mmol) was reacted with methyl iodide (57 mg, 0.4 mmol) in the presence of K₂CO₃ (55 mg, 0.4 mmol) in DMF (3 mL) to give [5-({5-[3-(2-hydroxy-ethyl)-phenyl]-pyridine-2-sulfonyl}-methyl-amino)-6,7,8,9-tetrahydro-5H-benzocyclohepten-1-yloxy]-acetic acid tert-butyl ester (100 mg, 49%)..¹H NMR (400 MHz, DMSO-*d*₆) δ: 9.12 (d, *J*=1.7 Hz, 1 H), 8.37 (dd, *J*=8.2, 2.1 Hz, 1 H), 8.04 (d, *J*=8.1 Hz, 1 H), 7.62-7.74 (m, 2 H), 7.46 (t, *J*=7.7 Hz, 1 H), 7.36 (d, *J*=7.6 Hz, 1 H), 7.12 (t, *J*=8.1 Hz, 1 H), 6.87 (d, *J*=7.8 Hz, 1 H), 6.74 (d, *J*=8.3 Hz, 1 H), 5.26 (d, *J*=10.0 Hz, 1 H), 4.69 (t, *J*=5.1 Hz, 1 H), 4.64 (s, 2 H), 3.63-3.73 (m, 2 H), 3.49 (dd, *J*=13.9, 6.8 Hz, 1 H), 2.96 (s, 3 H), 2.83 (t, *J*=6.8 Hz, 2 H), 2.22-2.36 (m, 1 H), 1.55-1.92 (m, 4 H), 1.42 (s, 9 H), 1.21-1.28 (m, 2 H), 1.00-1.13 (m, 1 H).

### Intermediate 3.17

### {5-[Methyl-(4'-methyl-biphenyl-3-sulfonyl)-amino]-6,7,8,9-tetrahydro-5H-benzocyclohepten-1-yloxy}-acetic acid tert-butyl ester

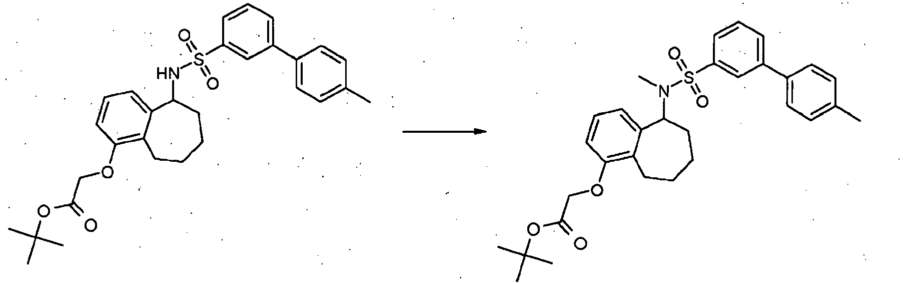

Using the conditions of General Procedure 3, {5-[methyl-(4'-methyl-biphenyl-3-sulfonyl)-amino]-6,7,8,9-tetrahydro-5H-benzocyclohepten-1-yloxy}-acetic acid tert-butyl ester (which may be prepared as described for Intermediate 2.22; 150 mg, 0.29 mmol) was reacted with methyl iodide (82 mg, 0.58 mmol) in the presence of K₂CO₃ (80 mg, 0.58 mmol) in DMF (2 mL) to give {5-[methyl-(4'-methyl-biphenyl-3-sulfonyl)-amino]-6,7,8,9-tetrahydro-5H-benzocyclohepten-1-yloxy}-acetic acid tert-butyl ester (100 mg, 65%).

### Intermediate 3.18

### {5-[(3'-Isopropyl-biphenyl-3-sulfonyl)-methyl-amino]-6,7,8,9-tetrahydro-5H-benzocyclohepten-1-yloxy}-acetic acid tert-butyl ester

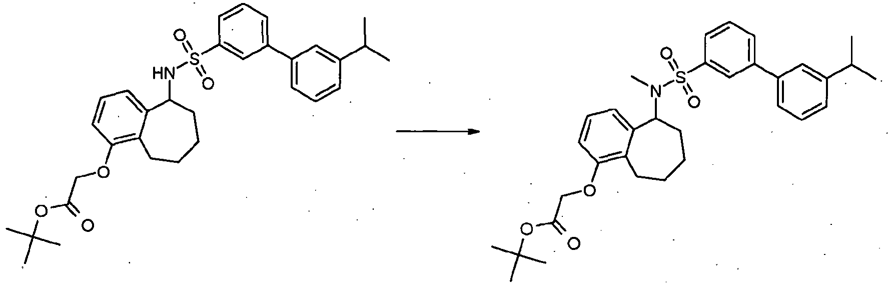

Using the conditions of General Procedure 3, {5-[(3'-isopropyl-biphenyl-3-sulfonyl)-methyl-amino]-6,7,8,9-tetrahydro-5H-benzocyclohepten-1-yloxy}-acetic acid tert-butyl ester (which may be prepared as described for Intermediate 2.23; 100 mg, 0.18 mmol) was reacted with methyl iodide (29 mg, 0.2 mmol) in the presence of K₂CO₃ (28 mg, 0.2 mmol) in DMF (2 mL) to give {5-[(3'-isopropyl-biphenyl-3-sulfonyl)-methyl-amino]-6,7,8,9-tetrahydro-5H-benzocyclohepten-1-yloxy}-acetic acid tert-butyl ester (70 mg, 72%). ¹H NMR (400 MHz, DMSO-*d*₆) δ: 7.97-8.07 (m, 2 H), 7.89 (d, *J*=7.8 Hz, 1 H), 7.69-7.80 (m, 1 H), 7.49-7.60 (m, 2 H), 7.40-7.48 (m, 1 H), 7.33 (d, *J*=7.8 Hz, 1 H), 7.10 (t, *J*=7.8 Hz, 1 H), 6.83 (d, *J*=7.8 Hz, 1 H), 6.74 (d, *J*=8.3 Hz, 1 H), 5.21 (br. s., 1 H), 4.63 (s, 2 H), 3.44-3.50 (m, 1 H), 2.95-3.09 (m, 1 H), 2.87 (s, 3 H), 2.67 (br. s., 1 H), 1.47-1.85 (m, 1 H), 1.42 (s, 9 H), 1.27 (d, *J*=6.8 Hz, 6 H), 1.01-1.10 (m, 1 H).

### Intermediate 3.19

### {5-[(3-Isopropyl-5-trifluoromethyl-benzenesulfonyl)-methyl-amino]-6,7,8,9-tetrahydro-5H-benzocyclohepten-1-yloxy}-acetic acid tert-butyl ester

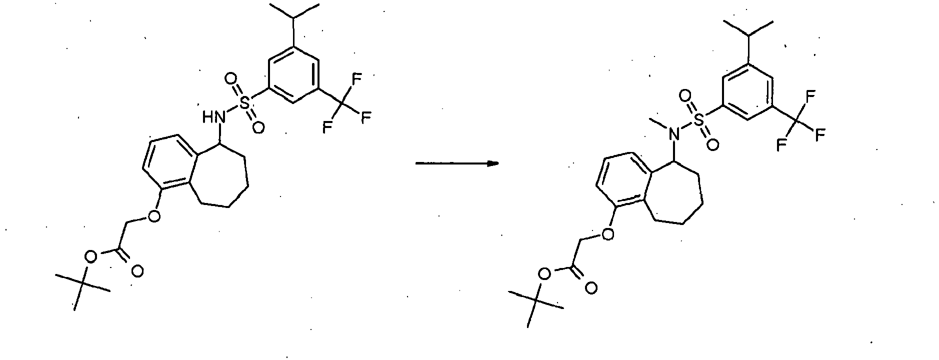

A mixture of [5-(3-isopropyl-5-trifluoromethyl-benzenesulfonylamino)-6,7,8,9-tetrahydro-SH-benzocyclohepten-1-yloxy]-acetic acid tert-butyl ester (which may be prepared as described for Intermediate 2.24; 100 mg, 0.18 mmol), K₂CO₃ (53 mg, 0.38 mmol), and methyl iodide (81 mg, 0.57 mmol) in CH₂Cl₂ (5 mL) was stirred at room temperature for 16 h. The reaction mixture was concentrated and diluted with EtOAc (10 mL). The mixture was washed with water (3 x 10 mL). The organic layer was concentrated under reduced pressure to give crude {5-[(3-isopropyl-5-trifluoromethyl-benzenesulfonyl)-methyl-amino]-6,7,8,9-tetrahydro-5H-benzocyclohepten-1-yloxy}-acetic acid tert-butyl ester (120 mg, 117% crude yield) as a yellow solid, which was used directly in the next step without further purification.

### Intermediate 3.20

### {5-[(4-Iodo-benzenesulfonyl)-methyl-amino]-6,7,8,9-tetrahydro-5H-benzocyclohepten-1-yloxy}-acetic acid tert-butyl ester

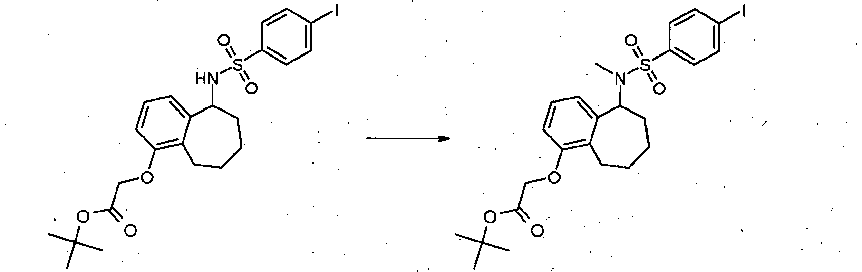

A mixture of [5-(4-iodo-benzenesulfonylamino)-6,7,8,9-tetrahydro-5H-benzocyclohepten-1-yloxy]-acetic acid tert-butyl ester (which may be prepared as described for Intermediate 1.08; 250 mg, 0.45 mmol), methyl iodide (0.06 mL, 0.96 mmol), and K₂CO₃ (138 mg, 1 mmol) in DMF (2 mL) was stirred overnight at room temperature. The reaction mixture was partitioned between EtOAc and H₂O. The organic layers were combined, washed with water and brine, dried over MgSO₄, filtered, and concentrated to give {5-[(4-iodo-benzenesulfonyl)-methyl-amino]-6,7,8,9-tetrahydro-5H-benzocyclohepten-1-yloxy}-acetic acid tert-butyl ester (210 mg, 82%). ¹H NMR (400 MHz, DMSO-*d*₆) δ: 8.03 (d, *J*=8.3 Hz, 2 H), 7.65 (d, *J*=8.3 Hz, 2 H), 7.06-7.16 (m, 1 H), 6.81 (d, *J*=7.8 Hz, 1 H), 6.74 (d, *J*=8.1 Hz, 1 H), 5.08 (d, *J*=9.0 Hz, 1 H), 4.64 (s, 2 H), 3.46 (dd, *J*=14.2, 6.8 Hz, 1 H), 2.82 (s, 3 H), 2.33 (t, *J*=12.8 Hz, 1 H), 1.73-1.85 (m, 2 H), 1.49-1.60 (m, 1 H), 1.42 (s, 9 H), 1.22-1.35 (m, 2 H), 1.04 (q, *J*=11.5 Hz, 1 H).

### PART III: PREPARATION OF COMPOUNDS OF INTEREST

### Example 1

### [5-(3,5-Bis-trifluoromethyl-benzenesulfonylamino)-6,7,8,9-tetrahydro-5H-benzocyclohepten-1-yloxy]-acetic acid

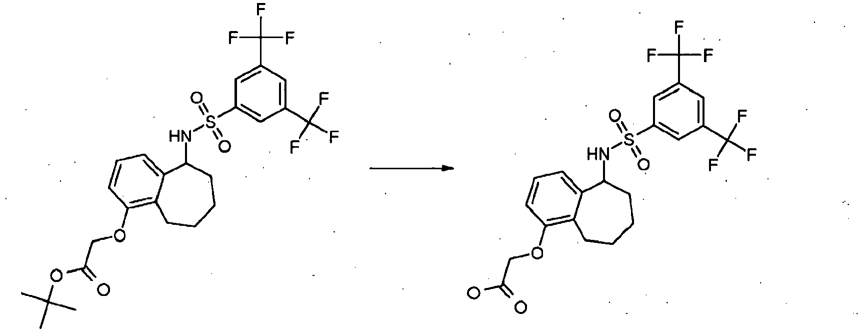

A solution of lithium hydroxide monohydrate (19 mg, 0.45 mmol) in water (2 mL) was added to a solution of [5-(3,5-bis-trifluoromethyl-benzenesulfonylamino)-6,7,8,9-tetrahydro-5H-benzocyclohepten-1-yloxy]-acetic acid tert-butyl ester (which may be prepared as described for Intermediate 2.01; 85 mg, 0.15 mmol) in THF (8 mL) at room temperature. The reaction mixture was stirred for 48 h at room temperature. TLC showed that the reaction was not complete so a second portion of lithium hydroxide monohydrate (19 mg, 0.45 mmol) in water (1 mL) was added and the reaction mixture was stirred at room temperature overnight. The reaction mixture was concentrated under reduced pressure. H₂O (2 mL) was added and the mixture was extracted with ethyl acetate (3 mL). The aqueous layer was acidified using dilute aq. HCl. The resulting mixture was extracted with EtOAc (3 x 10 mL). The organic extracts were combined, dried over anhydrous Na₂SO₄, filtered, and evaporated to give [5-(3,5-bis-trifluoromethyl-benzenesulfonylamino)-6,7,8,9-tetrahydro-5H-benzocyclohepten-1-yloxy]-acetic acid (45 mg, 59%) as a white solid. ¹H NMR (400 MHz, DMSO-*d*₆) δ: 12.95 (br. s., 1 H), 8.52 (d, *J*=7.8 Hz, 1 H), 8.32 (s, 1 H), 8.19 (s, 2 H), 6.87 (t, *J*=7.8 Hz, 1 H), 6.66 (d, *J*=7.6 Hz, 1 H), 6.57 (d, *J*=8.3 Hz, 1 H), 4.56-4.65 (m, 1 H), 4.40-4.54 (m, 2 H), 2.81 (br. s., 2 H), 1.71-1.93 (m, 2 H), 1.59 (br. s., 2 H), 1.11-1.51 (m, 2 H).

### Example 2

### [5-(3,5-Dichloro-benzenesulfonylamino)-6,7,8,9-tetrahydro-5H-benzocyclohepten-1-yloxy]-acetic acid

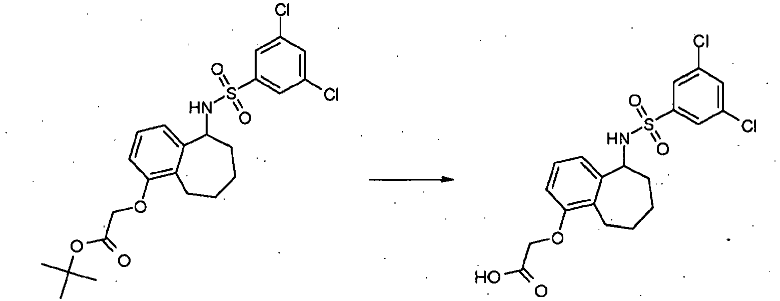

Using the conditions described for the preparation of Example 4, a solution of [5-(3,5-dichloro-benzenesulfonylamino)-6,7,8,9-tetrahydro-5H-benzocyclohepten-1-yloxy]-acetic acid tert-butyl ester (which may be prepared as described for Intermediate 2.02; 180 mg, 0.36 mmol) in THF (4 mL) was reacted with a solution of lithium hydroxide monohydrate (75 mg, 1.8 mmol) in water (2 mL) and MeOH (1 mL) to give [5-(3,5-dichloro-benzenesulfonylamino)-6,7,8,9-tetrahydro-5H-benzocyclohepten-1-yloxy]-acetic acid (130 mg, 81%) as a white solid. ¹H NMR (300 MHz, DMSO-*d₆*) δ 12.95 (br s, 1 H), 8.37 (d, J = 7.8 Hz, 1 H), 7.85 (t, J = 1.8 Hz, 1 H), 6.97 (t, J = 7.8 Hz, 1 H), 6.73 (d, J = 7.6 Hz, 1 H), 6.67 (d, J = 7.5 Hz, 1 H), 4.55 (s, 2 H), 4.49-4.54 (m, 1 H), 2.93-3.01 (m, 1 H), 2.64-2.74 (m, 1 H), 1.33-1.86 (m, 6 H). HRMS [M-H⁻] observed: 442.0287, calculated for C₁₉H₁₈Cl₂NO₅S: 442.0288.

### Example 3

### [5-(Biphenyl-3-sulfonylamino)-6,7,8,9-tetrahydro-5H-benzocyclohepten-1-yloxy]-acetic acid

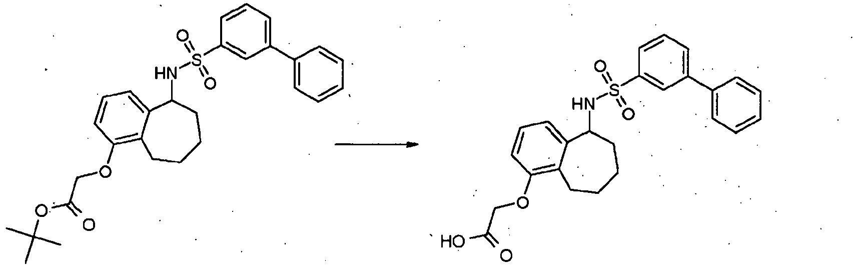

Using the conditions described for the preparation of Example 4, a solution of [5-(biphenyl-3-sulfonylamino)-6,7,8,9-tetrahydro-5H-benzocyclohepten-1-yloxy]-acetic acid tert-butyl ester (which may be prepared as described for Intermediate 2.03; 160 mg, 0.32 mmol) in THF (4 mL) was reacted with a solution of lithium hydroxide monohydrate (66 mg, 1.6 mmol) in water (2 mL) and MeOH (1 mL) to give [5-(biphenyl-3-sulfonylamino)-6,7,8,9-tetrahydro-5H-benzocyclohepten-1-yloxy]-acetic acid (120 mg, 84%) as an off-white solid. ¹H NMR (300 MHz, DMSO-*d*₆) δ 12.93 (br s, 1 H), 8.20 (d, J = 8.1 Hz, 1 H), 7.98-7.99 (m, 1 H), 7.86-7.89 (m, 1 H), 7.75-7.79 (m, 1 H), 7.59-7.64 (m, 3 H), 7.47-7.52 (m, 2 H), 7.39-7.44 (m, 1 H), 6.95 (t, J = 8.0 Hz, 1 H), 6.81 (d, J = 7.8 Hz, 1 H), 6.65 (d, J = 8.1 Hz, 1 H), 4.55 (s, 2 H), 4.44-4.51 (m, 1 H), 3.11-3.20 (m, 1 H), 2.55-2.60 (m, 1 H), 1.65-1.76 (m, 1 H), 1.44-1.59 (m, 4 H), 1.16-1.28 (m, 1 H)

### Example 4

### [5-(Biphenyl-4-sulfonylamino)-6,7,8,9-tetrahydro-5H-benzocyclohepten-1-yloxy]-acetic acid

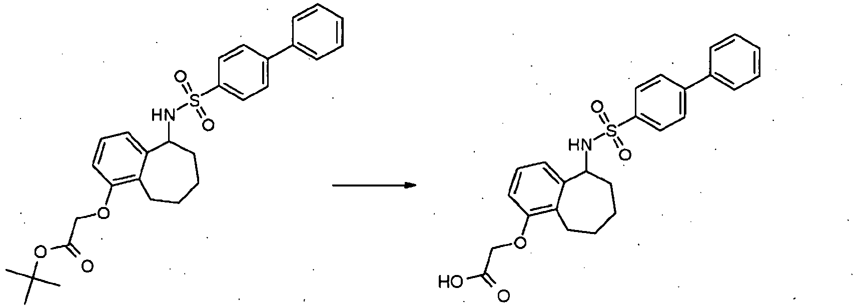

A solution of lithium hydroxide monohydrate (70 mg, 1.67 mmol) in water (2 mL) and MeOH (1 mL) was added to a solution of [5-(biphenyl-4-sulfonylamino)-6,7,8,9-tetrahydro-5H-benzocyclohepten-1-yloxy]-acetic acid tert-butyl ester (which may be prepared as described for Intermediate 2.04; 170 mg, 0.33 mmol) in THF (4 mL) at room temperature. The reaction mixture was stirred for 6 h at room temperature. The reaction mixture was concentrated under reduced pressure. H₂O (5 mL) was added and the mixture was acidified to pH ∼3 using 50% aq. HCl. The mixture was extracted with EtOAc (3 x 10 mL). The organic extracts were combined, washed with water (5 mL) and brine (5 mL), dried over anhydrous Na₂SO₄, filtered, and evaporated. The product was recrystallized from hexane to give [5-(biphenyl-4-sulfonylamino)-6,7,8,9-tetrahydro-5H-benzocyclohepten-1-yloxy]-acetic acid (100 mg, 66%) as an off-white solid. ¹H NMR (300 MHz, DMSO-*d*₆) δ 12.92 (br s, 1 H), 8.20 (d, J = 7.8 Hz, 1 H), 7.82-7.86 (m, 3 H), 7.70-7.74 (m, 2 H), 7.52-7.38 (m, 3 H), 6.95 (t, J = 7.9 Hz, 1 H), 6.84 (d, J = 7.5 Hz, 1 H), 6.68 (d, J = 7.5 Hz, 1 H), 4.57 (s, 2 H), 4.45 (br t, 1 H), 3.14-3:22 (m, 1 H), 1.44-1.74 (m, 6 H). HRMS [M+H⁺] observed: 452.1532, calculated for C₂₅H₂₆NO₅S: 452.1526.

### Example 5

### [5-(3-Methanesulfonyl-benzenesulfonylamino)-6,7,8,9-tetrahydro-5H-benzocyclohepten-1-yloxy]-acetic acid

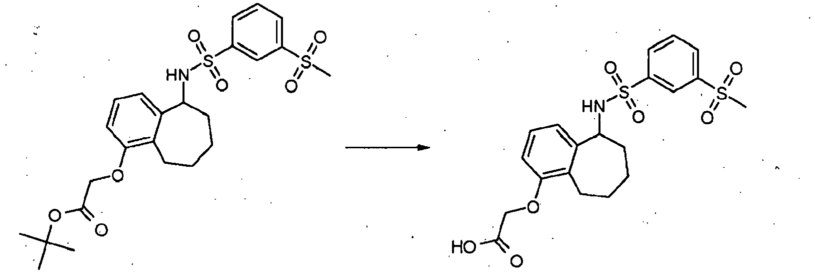

Using the conditions described for the preparation of Example 4, [5-(3-methanesulfonyl-benzenesulfonylamino)-6,7,8,9-tetrahydro-5H-benzocyclohepten-1-yloxy]-acetic acid tert-butyl ester (which may be prepared as described for Intermediate 2.05; 185 mg, 0.36 mmol) in THF (4 mL) was reacted with a solution of lithium hydroxide monohydrate (61 mg, 1.5 mmol) in water (2 mL) and MeOH (1 mL) to give [5-(3-methanesulfonyl-benzenesulfonylamino)-6,7,8,9-tetrahydro-5H-benzocyclohepten-1-yloxy]-acetic acid (85 mg, 52%) as an off-white solid. ¹H NMR (300 MHz, DMSO-*d₆*) δ 12.95 (br s, 1 H), 8.42 (d, J = 7.8 Hz, 1 H), 8.24 (t, J = 1.8 Hz, 1 H), 8.08-8.11 (m, 1 H), 8.05-8.01 (m, 1 H), 7.77 (t, J = 7.8 Hz, 1 H), 6.92 (t, J = 7.9 Hz, 1 H), 6.71 (d, J = 7.8 Hz, 1 H), 6.63 (d, J = 7.8 Hz, 1 H), 4.55 (s, 2 H), 4.45-4.52 (m, 1 H), 3.22,(s, 3 H), 3.02-3.11 (m, 1 H), 2.54-2.64 (m, 1 H), 1.78-1.24 (m, 6 H). HRMS [M+H⁺] observed: 454.0994, calculated for C₂₀H₂₄NO₇S₂: 454.0989.

### Example 6

### [5-(3-Fluoro-5-trifluoromethyl-benzenesulfonylamino)-6,7,8,9-tetrahydro-5H-benzocyclohepten-1-yloxy]-acetic acid

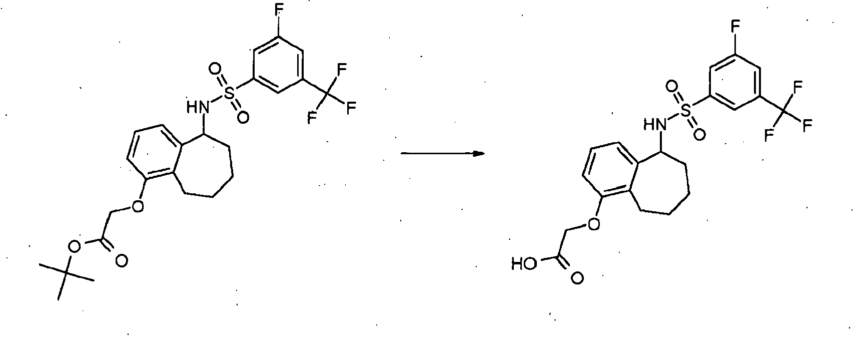

A mixture of [5-(3-fluoro-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.06; 70 mg, 0.14 mmol) and 2 N NaOH solution (1.35 mL, 2.7 mmol) in THF (1 mL) was stirred at room temperature for 20 h. The reaction mixture was concentrated under reduced pressure and the residue was washed with ether (2 x 2 mL), diluted with EtOAc (10 mL) and acidified with 2 N HCl solution. The layers were separated and the aqueous layer was extracted with EtOAc (2 x 5 mL). The organic layers were combined, dried over anhydrous Na₂SO₄, filtered, and evaporated to give [5-(3-fluoro-5-trifluoromethyl-benzenesulfonylamino)-6,7,8,9-tetrahydro-5H-benzocyclohepten-1-yloxy]-acetic acid (40 mg, 64%). ¹H NMR (400 MHz, DMSO-*d*₆) δ: 8.48 (d, *J*=7.6 Hz, 1 H), 7.96 (d, *J*=8.1 Hz, 1 H), 7.73-7.88 (m, 2 H), 6.94 (t, *J*=7.9 Hz, 1 H), 6.72 (d, *J*=7.3 Hz, 1 H), 6.66 (d, *J*=8.3 Hz, 1 H), 4.53-4.60 (m, 3 H), 2.89-3.05 (m, 1 H), 2.61-2.81 (m, 1 H), 1.75-1.86 (m, 1 H), 1.05-1.73 (m, 6 H). HRMS [M+Na] observed: 484.0812, calculated for C₂₀H₁₉F₄NNaO₅S: 484.0812.

### Example 7

### {5-[(3-Fluoro-5-trifluoromethyl-benzenesulfonyl)-methyl-amino]-6,7,8,9-tetrahydro-5H-benzocyclohepten-1-yloxy}-acetic acid

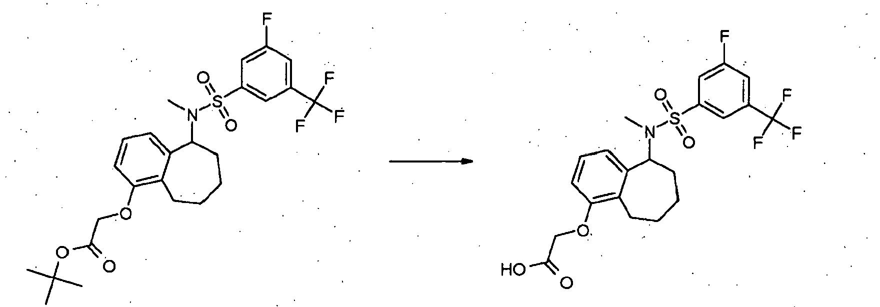

Using the conditions of General Procedure 7, {5-[(3-fluoro-5-trifluoromethyl-benzenesulfonyl)-methyl-amino]-6,7,8,9-tetrahydro-5H-benzocyclohepten-1-yloxy}-acetic acid tert-butyl ester (which may be prepared as described for Intermediate 3.01; 60 mg, 0.11 mmol) was hydrolyzed using 2 N NaOH solution (1.13 mL, 2.3 mmol) to give {5-[(3-fluoro-5-trifluoromethyl-benzenesulfonyl)-methyl-amino]-6,7,8,9-tetrahydro-5H-benzocyclohepten-1-yloxy}-acetic acid (20 mg, 37%). ¹H NMR (400 MHz, DMSO-*d*₆) δ: 8.15 (apparent t, *J*=7.7 Hz, 2 H), 7.99 (s, 1 H), 7.04 (t, *J*=8.1 Hz, 1 H), 6.70 (d, *J*=8.1 Hz, 2 H), 5.19 (d, *J*=9.3 Hz, 1 H), 4.36 (br. s., 2 H), 3.43-3.51 (m, 1 H), 2.88 (s, 3 H), 2.67 (br. s., 1 H), 2.29-2.45 (m, 2 H), 1.72-1.86 (m, 2 H), 1.56-1.63 (m, 1 H), 1.19-1.46 (m, 3 H), 0.99-1.10 (m, 1 H). HRMS [M+Na] observed: 498.0968, calculated for C₂₁H₂₁F₄NNaO₅S: 498.0969.

### Example 8

### [5-(3-Bromo-5-trifluoromethyl-benzenesulfonylamino)-6,7,8,9-tetrahydro-5H-benzocyclohepten-1-yloxy] -acetic acid

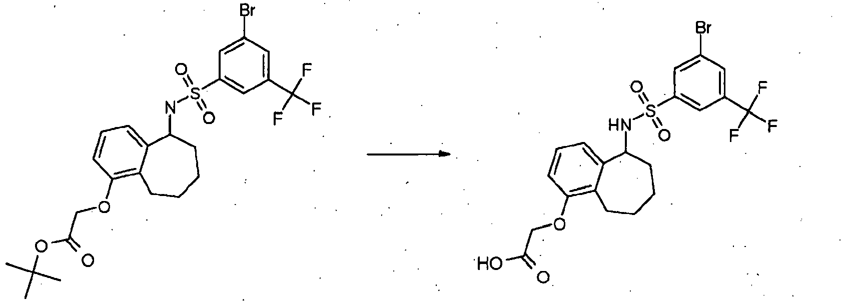

Using the conditions described for the preparation of Example 23, [5-(3-bromo-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.07; 100 mg, 0.17 mmol) was hydrolyzed using 2 N NaOH solution (1.73 mL, 3.5 mmol) to give [5-(3-bromo-5-trifluoromethyl-benzenesulfonylamino)-6,7,8,9-tetrahydro-5H-benzocyclohepten-1-yloxy]-acetic acid (60 mg, 66% yield). ¹H NMR (300 MHz, DMSO-*d₆*) δ 12.93 (br s, 1 H), 8.42 (d, J = 7.8 Hz, 1 H), 8.18 (s, 1 H), 8.02 (s, 1 H), 7.94 (d, J = 0.6 Hz, 1 H), 6.92 (t, J = 8.0 Hz, 1 H), 6.68 (d, J = 7.6 Hz, 1 H), 6.63 (d, J = 7.5 Hz, 1 H), 4.53-4.60 (m, 1 H), 4.51 (d, J = 7.5 Hz, 2 H), 2.70-2.93 (m, 2 H), 1.33-1.88 (m, 6 H).

### Example 9

### {5-[(3-Bromo-5-trifluoromethyl-benzenesulfonyl)-methyl-amino]-6,7,8,9-tetrahydro-5H-benzocyclohepten-1-yloxy}-acetic acid

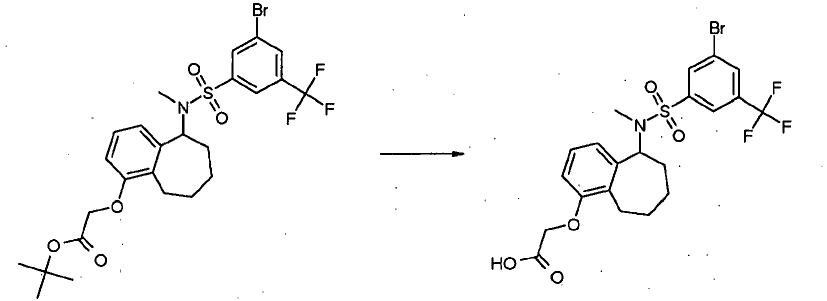

Using the conditions described for the preparation of Example 23, {5-[(3-bromo-5-trifluoromethyl-benzenesulfonyl)-methyl-amino]-6,7,8,9-tetrahydro-5H-benzocyclohepten-1-yloxy}-acetic acid tert-butyl ester (which may be prepared as described for Intermediate 3.02; 150 mg, 0.25 mmol) was hydrolyzed using 2 N NaOH solution (1.73 mL, 3.5 mmol) to give {5-[(3-bromo-5-trifluoromethyl-benzenesulfonyl)-methyl-amino]-6,7,8,9-tetrahydro-5H-benzocyclohepten-1-yloxy}-acetic acid (60 mg, 44% yield). ¹H NMR (400 MHz, DMSO-*d*₆) δ: 12.95 (br. s., 1 H), 8.39 (s, 1 H), 8.14 (s, 1 H), 7.08 (t, J = 8.2 Hz, 1 H), 6.72-6.77 (m, 1 H), 5.24 (d, *J*=8.8 Hz, 1 H), 4.63-4.67 (m, 2 H), 3.46 (dd, *J*=13.9, 6.8 Hz, 1 H), 2.87 (s, 3 H), 2.37-2.46 (m, 1 H), 1.54-1.95 (m, 3 H), 1.19-1.48 (m, 4 H), 1.00-1.08 (m, 1 H). HRMS [M-H⁻] observed: 520.0043, calculated for C₂₀H₁₈BrF₃NO₅S: 520.0047.

### Example 10

### [5-(3,5-Bis-methanesulfonyl-benzenesulfonylamino)-6,7,8,9-tetrahydro-5H-benzocyclohepten-1-yloxy]-acetic acid

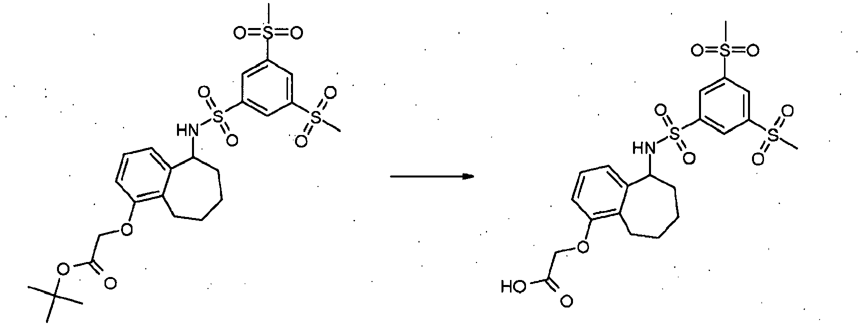

Using the conditions of General Procedure 7, [5-(3,5-bis-methanesulfonyl-benzenesulfonylamino)-6,7,8,9-tetrahydro-5H-benzocyclohepten-1-yloxy]-acetic acid tert-butyl ester (which may be prepared as described for Intermediate 2.08; 100 mg, 0.17 mmol) was hydrolyzed using 2 N NaOH solution (1.7 mL), 3.4 mmol) to give [5-(3,5-bis-methanesulfonyl-benzenesulfonylamino)-6,7,8,9-tetrahydro-5H-benzocyclohepten-1-yloxy]-acetic acid (55 mg, 61% yield). ¹H NMR (400 MHz, DMSO-*d*₆) δ: 8.59 (d, *J*=7.8 Hz, 1 H), 8.51 (s, 1 H), 8.45 (s, 2 H), 6.85-6.92 (m, 1 H), 6.66 (d, *J*=7.3 Hz, 1 H), 6.58 (d, *J*=8.3 Hz, 1 H), 4.60 (t, *J*=7.3 Hz, 1 H), 4.52 (s, 2 H), 1.52-1.93 (m, 4 H), 1.35-1.45 (m, 1 H). HRMS [M+Na] observed: 554.0583, calculated for C₂₂H₂₇NNaO₉S₃: 554.0583.

### Example 11

### {5-[(3,5-Bis-methanesulfonyl-benzenesulfonyl)-methyl-amino]-6,7,8,9-tetrahydro-5H-benzocyclohepten-1-yloxy}-acetic acid

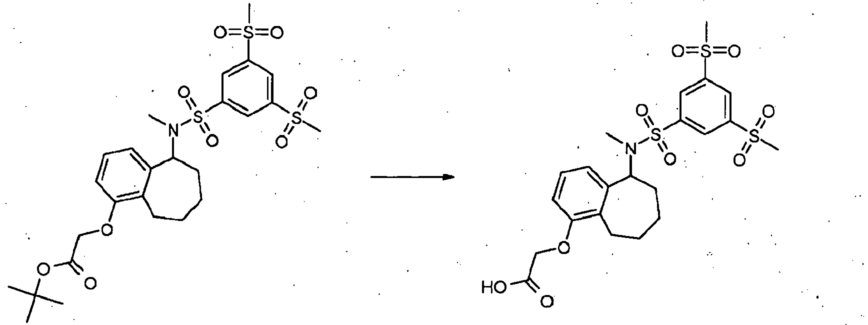

Using the conditions of General Procedure 7, {5-[(3,5-bis-methanesulfonyl-benzenesulfonyl)-methyl-amino]-6,7,8,9-tetrahydro-5H-benzocyclohepten-1-yloxy}-acetic acid tert-butyl ester (which may be prepared as described for Intermediate 3.03; 100 mg, 0.17 mmol) was hydrolyzed using 2 N NaOH solution (1.65 mL, 3.3 mmol) to give {5-[(3,5-bis-methanesulfonyl-benzenesulfonyl)-methyl-amino]-6,7,8,9-tetrahydro-5H-benzocyclohepten-1-yloxy}-acetic acid (45 mg, 50% yield). ¹H NMR (400 MHz, DMSO-*d*₆) δ: 8.66 (s, 1 H), 8.63 (s, 2 H), 7.08 (t, *J*=7.8 Hz, 1 H), 6.73-6.78 (m, 2 H), 5.30 (d, *J*=8.8 Hz, 1 H), 4.66 (s, 2 H), 3.46 (s, 6H), 2.91 (s, 3 H), 2.31-2.44 (m, 1 H), 1.63-1.91 (m, 2 H), 1.32-1.54 (m, 3 H), 0.93-1.11 (m, 1 H). HRMS [M+Na] observed: 568.0740, calculated for C₂₁H₂₅NNaO₉S₃: 568.0740.

### Example 12

### {5-[(Biphenyl-4-sulfonyl)-methyl-amino]-6,7,8,9-tetrahydro-5H-benzocyclohepten-1-yloxy}-acetic acid

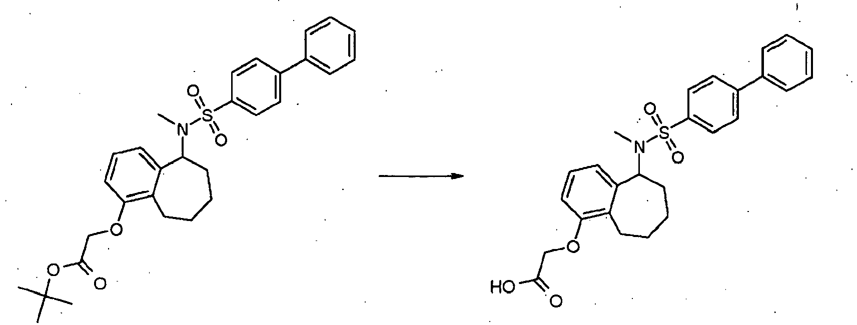

Using the conditions of General Procedure 7, {5-[(biphenyl-4-sulfonyl)-methyl-amino]-6,7,8,9-tetrahydro-5H-benzocyclohepten-1-yloxy}-acetic acid tert-butyl ester (which may be prepared as described for Intermediate 3.04; 90 mg, 0.17 mmol) was hydrolyzed using 2 N NaOH solution (1.7 mL, 3.4 mmol) to give {5-[(biphenyl-4-sulfonyl)-methyl-amino]-6,7,8,9-tetrahydro-5H-benzocyclohepten-1-yloxy}-acetic acid (50 mg, 62% yield). ¹H NMR (DMSO-d₆) δ: 8.02 (s, 4 H), 7.84 (d, J = 7.3 Hz, 2 H), 7.56-7.63 (m, 2 H), 7.49-7.56 (m, 1 H), 7.12-7.23 (m, 1 H), 6.91 (d, J = 7.8 Hz, 1 H), 6.84 (d, J = 8.3 Hz, 1 H), 5.17-5.25 (m, 1 H), 4.72 (s, 2 H), 3.55 (dd, J = 13.9, 6.9 Hz, 1 H), 2.92 (s, 3 H), 2.41 (t, J = 12.7 Hz, 1 H), 1.76-1.95 (m, 2 H), 1.63 (d, J = 11.3 Hz, 1 H), 1.35-1.49 (m, 2 H), 1.11 (q, J = 11.8 Hz, 1 H). HRMS [M+Na] observed: 488.1499, calculated for C₂₆H₂₇NNaO₅S: 488.1502.

### Example 13

### [5-(3-Methoxy-5-trifluoromethyl-benzenesulfonylamino)-6,7,8,9-tetrahydro-5H-benzocyclohepten-1-yloxy]-acetic acid

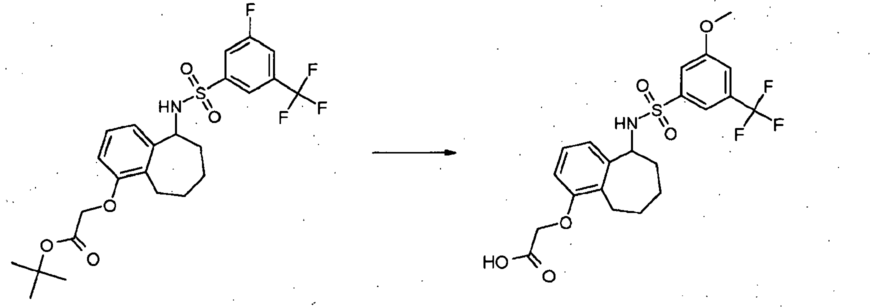

A mixture of [5-(3-fluoro-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.06; 100 mg, 0.19 mmol) and NaOMe (52 mg, 0.96 mmol) in MeOH/DMF (1:1; 4 mL) was heated at 150 °C in a microwave reactor for 45 min. MeOH was removed by evaporation under reduced pressure and water was added. The mixture was stilted for 15 min and extracted with ether. The aqueous layer was acidified with 1 N HCl and extracted with ethyl acetate. The EtOAc extracts were combined, washed with water and brine, dried over anhydrous Na₂SO₄, filtered, and evaporated to give [5-(3-methoxy-5-trifluoromethyl-benzenesulfonylamino)-6,7,8,9-tetrahydro-5H-benzocyclohepten-1-yloxy]-acetic acid (60 mg, 66%). ¹H NMR (DMSO-d₆) δ: 8.37 (d, J = 7.0 Hz, 1 H), 7.57 (s, 1 H), 7.46 (d, J = 11.3 Hz, 2 H), 6.89-7.00 (m, 1 H), 6.61-6.78 (m, 2 H), 4.45-4.61 (m, 3 H), 3.85 (s, 2 H), 3.05 (br. s., 1 H), 2.67 (br. s., 1 H), 1.12-1.90 (m, 6 H). HRMS [M+Na] observed: 496.1012, calculated for C₂₁H₂₂F₃NNaO₆S: 496.1012.

### Example 14

### {5-[(3-Methoxy-5-trifluoromethyl-benzenesulfonyl)-methyl-amino]-6,7,8,9-tetrahydro-5H-benzocyclohepten-1-yloxy}-acetic acid

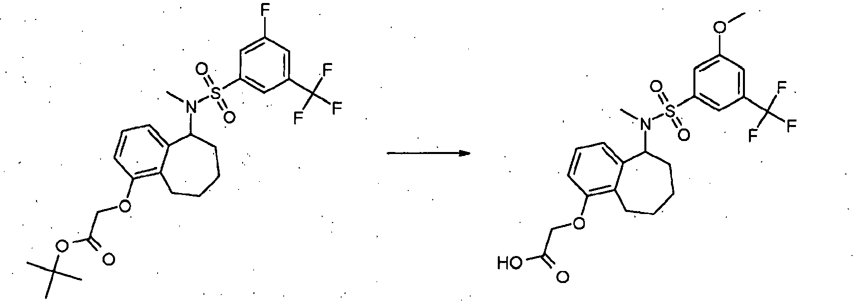

A mixture of {5-[(3-fluoro-5-trifluoromethyl-benzenesulfonyl)-methyl-amino]-6,7,8,9-tetrahydro-5H-benzocyclohepten-1-yloxy}-acetic acid tert-butyl ester (which may be prepared as described for Intermediate 3.01; 60 mg, 0.11 mmol) and NaOMe (30.5 mg, 0.56 mmol) in MeOH/DMF (1:1; 2 mL) was heated at 150 °C in a microwave reactor for 45 min. MeOH was removed by evaporation under reduced pressure and water was added. The mixture was stirred for 15 min and extracted with ether. The aqueous layer was acidified with 1 N HCl and extracted with ethyl acetate. The EtOAc extracts were combined, washed with water and brine, dried over anhydrous Na₂SO₄, filtered, and evaporated to give {5-[(3-methoxy-5-trifluoromethyl-benzenesulfonyl)-methyl-amino]-6,7,8,9-tetrahydro-5H-benzocyclohepten-1-yloxy}-acetic acid (20 mg, 36%). ¹H NMR (DMSO-d₆) δ: 7.68 (s, 1 H), 7.62 (s, 2 H), 7.06-7.13 (m, 1 H), 6.77 (d, J = 8.3 Hz, 2 H), 5.20 (d, J = 9.0 Hz, 1 H), 4.65 (s, 2 H), 3.94 (s, 3 H), 3.47 (dd, J = 14.2, 6.7 Hz, 1 H), 2.86 (s, 3 H), 2.32-2.45 (m, 1 H), 0.94-1.90 (m, 6 H). HRMS [M+Na] observed 510.1170, calculated for C₂₂H₂₄F₃NNaO₆S: 510.1168.

### Example 15

### [5-(3-Acetyl-5-trifluoromethyl-benzenesulfonylamino)-6,7,8,9-tetrahydro-5H-benzocyclohepten-1-yloxy]-acetic acid

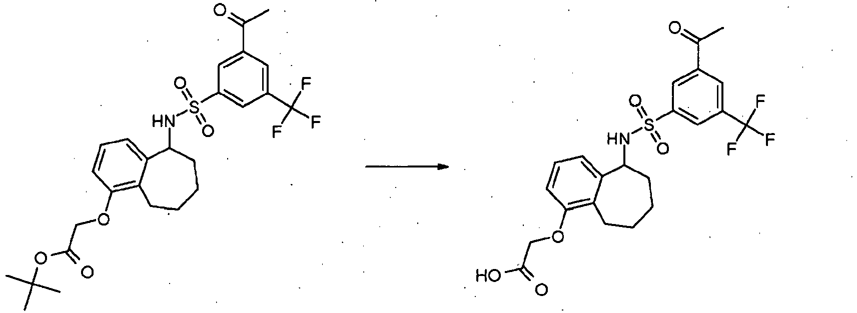

Using the conditions of General Procedure 7, [5-(3-acetyl-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.10; 70 mg, 0.13 mmol) was hydrolyzed using 2 N NaOH solution (1.3 mL, 2.6 mmol) to give [5-(3-acetyl-5-trifluoromethyl-benzenesulfonylamino)-6,7,8,9-tetrahydro-5H-benzocyclohepten-1-yloxy]-acetic acid (35 mg, 56% yield). ¹H NMR (DMSO-*d₆*) δ: 8.48 (d, J = 6.8 Hz, 1 H), 8.36 (s, 1 H), 8.32 (s, 1 H), 8.16 (s, 1 H), 6.83-6.93 (m, 1 H), 6.68 (d, J = 7.5 Hz, 1 H), 6.58 (d, J = 8.3 Hz, 1 H), 4.54 (d, J = 10.0 Hz, 1 H); 4.42-4,50 (m, 2 H), 2.88 (br. s., 1 H), 2.73 (d, J = 13.1 Hz, 1 H), 2.61-2.68 (m, 3 H), 1.66-1.90 (m, 2 H), 1.58 (d, J = 10.3 Hz, 2 H), 1.40 (br. s., 2 H). HRMS [M+Na] observed: 508.1015, calculated for C₂₂H₂₂F₃NNaO₆S: 508.1012.

### Example 16

### {5-[(3-Acetyl-5-trifluoromefhyl-benzenesulfonyl)-methyl-amino]-6,7,8,9-tetrahydro-5H-benzocyclohepten-1-yloxy}-acetic acid

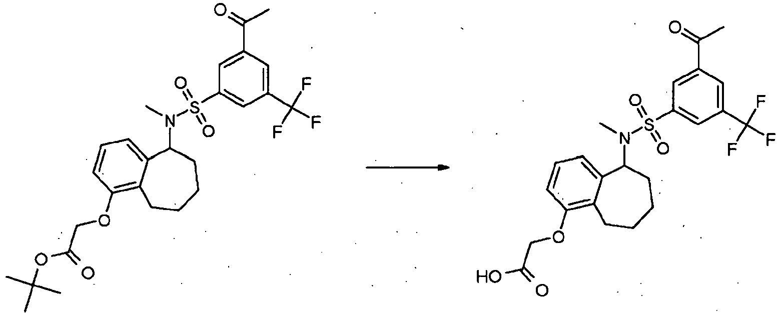

Using the conditions of General Procedure 7, {5-[(3-acetyl-5-trifluoromethyl-benzenesulfonyl)-methyl-amino]-6,7,8,9-tetrahydro-5H-benzocyclohepten-1-yloxy}-acetic acid tert-butyl ester (which may be prepared as described for Intermediate 3.05; 150 mg, 0.27 mmol) was hydrolyzed using 2 N NaOH solution (2.9 mL, 5.8 mmol) to give {5-[(3-acetyl-5-trifluoromethyl-benzenesulfonyl)-methyl-amino]-6,7,8,9-tetrahydro-5H-benzocyclohepten-1-yloxy}-acetic acid (70 mg, 52% yield). ¹H NMR (400 MHz, DMSO-*d*₆) δ: 8.50 (s, 2 H), 8.39 (s, 1 H), 7.07 (t, *J*=8.0 Hz, 1 H), 6.75 (t, *J*=8.5 Hz, 2 H), 5.23-5.33 (m, 1 H), 4.65 (s, 2 H), 3.47 (dd, *J*=14.2, 6.9 Hz, 1 H), 2.37-2.47 (m, 1 H), 1.54-1.88 (m, 3 H), 1.21-1.44 (m, 4 H), 0.98-1.10 (m, 1 H). HRMS [M+H⁺] observed: 500.1355, calculated for C₂₃H₂₅F₃NO₆S: 500.1349.

### Example 17

### [5-(3-Methanesulfonyl-5-trifluoromethyl-benzenesulfonylamino)-6,7,8,9-tetrahydro-5H-benzocyclohepten-1-yloxy]-acetic acid

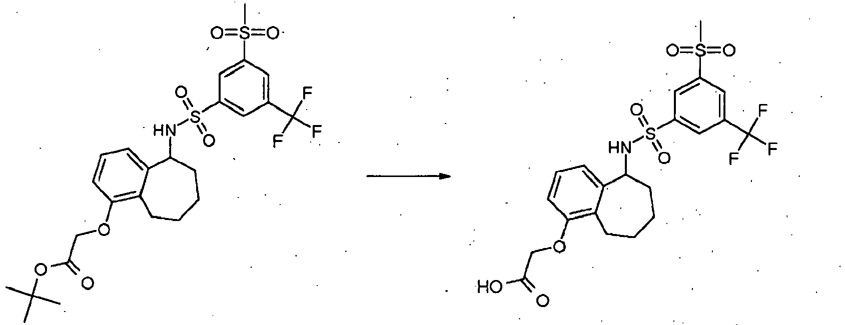

Using the conditions of General Procedure 7, [5-(3-methanesulfonyl-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.11; 20 mg, 0.035 mmol) was hydrolyzed using 2 N NaOH solution (0.2 mL, 0.4 mmol) to give [5-(3-methanesulfonyl-5-trifluoromethyl-benzenesulfonylamino)-6,7,8,9-tetrahydro-5H-benzocyclohepten-1-yloxy]-acetic acid (5 mg, 28% yield). ¹H NMR (400 MHz, DMSO-*d*₆) δ: 8.62 (d, *J*=7.8 Hz, 1 H), 8.50 (s, 1 H), 8.46 (s, 1 H), 8.25 (s, 1 H), 6.89-6.97 (m, 1 H), 6.72 (d, *J*=7.8 Hz, 1 H), 6.63 (d, *J*=8.3 Hz, 1 H), 4.66 (t, *J*=6.9 Hz, 1 H), 4.53 (d, *J*=2.8 Hz, 2 H), 2.84-2.93 (m, 2 H), 2.61 (s, 3 H), 1.26-1.96 (m, 9 H). HRMS [M-H⁻] observed:. 520.0713, calculated for C₂₁H₂₁NO₇S₂: 520.0717.

### Example 18

### {5-[(3-Methanesulfonyl-5-trifluoromethyl-benzenesulfonyl)-methyl-amino]-6,7,8,9-tetrahydro-5H-benzocyclohepten-1-yloxy}-acetic acid

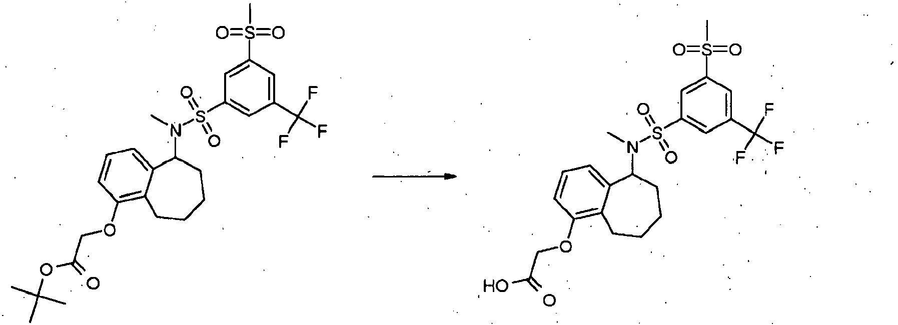

Using conditions similar to those described for Example 43, {5-[(3-methanesulfonyl-5-trifluoromethyl-benzenesulfonyl)-methyl-amino]-6,7,8,9-tetrahydro-5H-benzocyclohepten-1-yloxy}-acetic acid tert-butyl ester (which may be prepared as described for Intermediate 3.06; 80 mg, 0.13 mmol) was hydrolyzed using a solution of lithium hydroxide monohydrate (18 mg, 0.4 mmol) in H₂O/MeOH (2:1; 3 mL) to give {S-[(3-methanesulfonyl-5-trifluoromethyl-benzenesulfonyl)-methyl-amino]-6,7,8,9-tetrahydro-5H-benzocyclohepten-1-yloxy}-acetic acid (15 mg, 21% yield). ¹H NMR (400 MHz, DMSO-*d*₆) δ: 8.58 (s, 1 H), 8.56 (s, 1 H), 8.51 (s, 1 H); 7.05 (t, *J*=8.0 Hz, 1 H), 6.68-6.76 (m, 2 H), 5.28-5.31 (m, 1 H), 4.53 (s, 2 H), 3.46 (s, 3 H), 2.90 (s, 3 H), 2.37-2.47 (m, 1 H), 1.59-1.93 (m, 2 H), 0.95-1.53 (m, 4 H).

### Example 19

### [5-(3'-Isopropyl-biphenyl-4-sulfonylamino)-6,7,8,9-tetrahydro-5H-benzocyclohepten-1-yloxy]-acetic acid

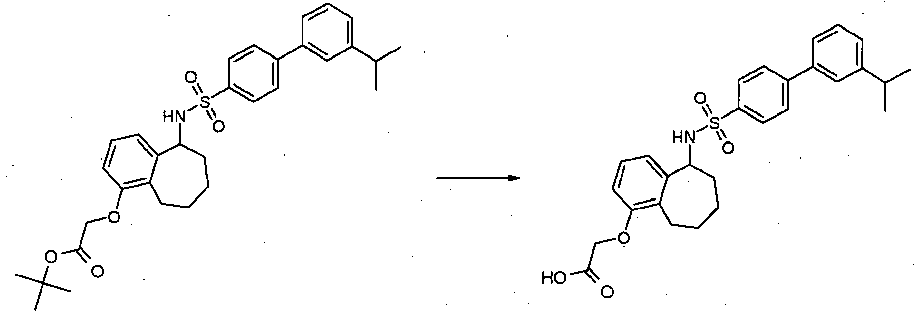

Using the conditions of General Procedure 7, [5-(3'-isopropyl-biphenyl-4-sulfonylamino)-6,7,8,9-tetrahydro-5H-benzocyclohepten-1-yloxy] -acetic acid tert-butyl ester (which may be prepared as described for Intermediate 2.12; 80 mg, 0.15 mmol) was hydrolyzed using 2 N NaOH solution (1.4 mL, 2.8 mmol) to give [5-(3'-isopropyl-biphenyl-4-sulfonylamino)-6,7,8,9-tetrahydro-5H-benzocyclohepten-1-yloxy]-acetic acid (28 mg, 39% yield). ¹H NMR (DMSO-d₆) δ: 8.22 (d, J = 7.8 Hz, 1 H), 7.80-7.94 (m, 4 H), 7.59 (s, 1 H), 7.53 (d, J = 7.8 Hz, 1 H), 7.42 (t, J = 7.5 Hz, 1 H), 7.32 (d, J = 7.5 Hz, 1 H), 6.95-7.05 (m, 1 H), 6.86 (d, J = 7.5 Hz, 1 H), 6.69 (d, J = 8.3 Hz, 1 H), 4.57 (s, 2 H), 4.46 (br. s., 1 H), 3.14-3.25 (m, 1 H), 2.99 (dt, J = 13.7, 7.0 Hz, 1 H), 1.72 (br. s., 1 H), 1.52 (br. s., 4 H), 1.1.8-1.30 (m, 8H). HRMS [M+Na] observed: 516.1815, calculated for C₂₈H₃₁NNaO₅S: 516.1815.

### Example 20

### {5-[(3'-Isopropyl-biphenyl-4-sulfonyl)-methyl-amino]-6,7,8,9-tetrahydro-5H-benzocyclohepten-1-yloxy}-acetic acid

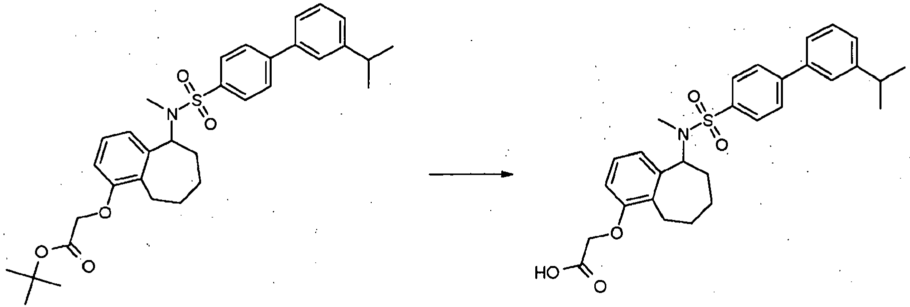

Using the conditions of General Procedure 7, {5-[(3'-isopropyl-biphenyl-4-sulfonyl)-methyl-amino]-6,7,8,9-tetrahydro-5H-benzocyclohepten-1-yloxy}-acetic acid tert-butyl ester (which may be prepared as described for Intermediate 3.07; 50 mg, 0.09 mmol) was hydrolyzed using 2 N NaOH solution (0.9 mL, 1.8 mmol) to give {5-[(3'-isopropyl-biphenyl-4-sulfonyl)-methyl-amino]-6,7,8,9-tetrahydro-5H-benzocyclohepten-1-yloxy}-acetic acid (28 mg, 62% yield). ¹H NMR (400 MHz, DMSO-*d*₆) δ: 7.95 (s, 4 H), 7.62 (s, 1 H), 7.57 (d, *J*=7.8 Hz, 1 H), 7.44 (t, *J*=7.7 Hz, 1 H), 7.34 (d, *J*=7.8 Hz, 1 H), 7.04-7.19 (m, 1 H), 6.85 (d, *J*=7.8 Hz, 1 H), 6.77 (d, *J*=8.3 Hz, 1 H), 5.08-5.20 (m, 1 H), 4.62 (s, 2 H), 3.48 (dd, *J*=14.1, 7.0 Hz, 1 H), 3.00 (dt, *J*=13.8,6.9 Hz, 1 H), 2.86 (s, 3 H), 2.28-2.43 (m, 1 H), 1.69-1.90 (m, 2 H), 1.48-1.66 (m, 1 H), 1.33-1.40 (m, 2 H), 1.27 (d, *J*=6.8 Hz, 6 H), 1.05 (q, *J*=11.5 Hz, 1 H). HRMS [M+H] observed: 508.2156, calculated for C₂₉H₃₄NO₅S: 508.2152.

### Example 21

### [5-(3'-tert-Butyl-5'-methyl-biphenyl-4-sulfonylamino)-6,7,8,9-tetrahydro-5H-benzocyclohepten-1-yloxy]-acetic acid

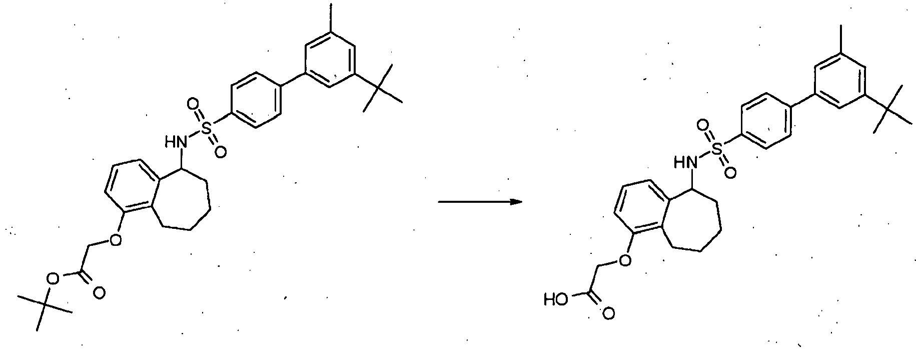

Using the conditions of General Procedure 7, [5-(3'-tert-butyl-5'-methyl-biphenyl-4-sulfonylamino)-6,7,8,9-tetrahydro-5H-benzocyclohepten-1-yloxy]-acetic acid tert-butyl ester (which may be prepared as described for Intermediate 2.13; 80 mg, 0.14 mmol) was hydrolyzed using 2 N NaOH solution (1.4 mL, 2.8 mmol) to give [5-(3'-tert-butyl-5'-methyl-biphenyl-4-sulfonylamino)-6,7,8,9-tetrahydro-5H-benzocyclohepten-1-yloxy]-acetic acid (13 mg, 18% yield). ¹H NMR (400 MHz, DMSO-*d*₆) δ: 8.19 (d, *J*=6.78 Hz, 1 H). 7.80-7.88 (m, 4 H), 7.48 (s, 1 H), 7.35 (s, 1 H), 7.28 (s, 1 H), 6.91-7.04 (m, 1 H), 6.81 (d, *J*=7.53 Hz, 1 H), 6.64 (d, *J*=8.03 Hz, 1 H), 4.45 (br. s., 1 H), 4.34 (s, 2 H), 2.38 (s, 3 H), 1.37-1.57 (m, 6 H), 1.33 (s., 9 H), 1.17-1.27 (m, 3 H).

### Example 22

### {5-[(3'-tert-Butyl-5'-methyl-biphenyl-4-sulfonyl)-methyl-amino]-6,7,8,9-tetrahydro-5H-benzocyclohepten-1-yloxy}-acetic acid

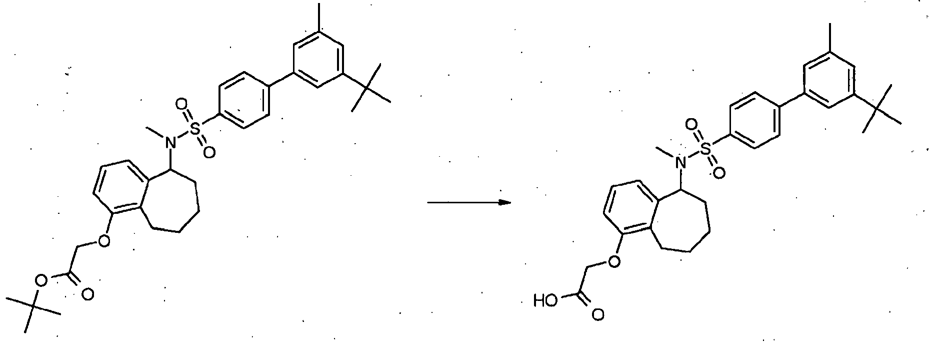

Using the conditions of General Procedure 7, {5-[(3'-tert-butyl-5'-methyl-biphenyl-4-sulfonyl)-methyl-amino]-6,7,8,9-tetrahydro-5H-benzocyclohepten-1-yloxy}-acetic acid tert-butyl ester (which may be prepared as described for Intermediate 3.08; 40 mg, 0.07 mmol) was hydrolyzed using 2 N NaOH solution (0.7 mL, 1.4 mmol) to give {5-[(3'-tert-butyl-5'-methyl-biphenyl-4-sulfonyl)-methyl-amino]-6,7,8,9-tetrahydro-5H-benzocyclohepten-1-yloxy}-acetic acid (20 mg, 55% yield). ¹H NMR (400 MHz, DMSO-*d*₆) δ: 8.00 (s, 4 H), 7.58 (s, 1 H), 7.45 (s, 1 H), 7.36 (s, 1 H), 7.18 (t, *J*=8.0 Hz, 1 H), 6.91 (d, *J*=7.8 Hz, 1 H), 6.83 (d, *J*=8.3 Hz, 1 H), 5.19-5.24 (m, 1 H), 4.69 (s, 2 H), 3.55 (dd, *J*=13.8, 6.8 Hz, 1 H), 2.92 (s, 3 H), 2.35-2.50 (m, 4 H), 1.52-1.97 (m, 3 H), 1.38-1.45 (m, 11 H), 1.00-1.21 (m, 1 H).

### Example 23

### [5-(4'-Hydroxy-biphenyl-4-sulfonylamino)-6,7,8,9-tetrahydro-5H-benzocyclohepten-1-yloxy]-acetic acid

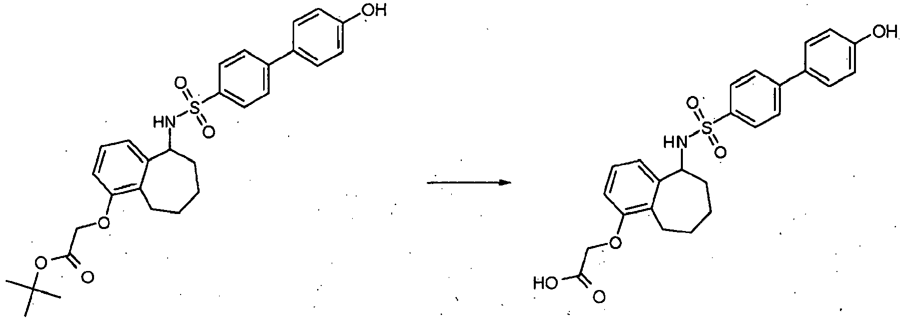

A mixture of [5-(4'-hydroxy-biphenyl-4-sulfonylamino)-6,7,8,9-tetrahydro-5H-benzocyclohepten-1-yloxy]-acetic acid tert-butyl ester (which may be prepared as described for Intermediate 2.14; 100 mg, 0.19 mmol) and 2 N NaOH solution (1.45 mL, 2.9 mmol) in THF (1 mL) was stirred at room temperature overnight. The THF was removed under reduced pressure and water was added. The mixture was washed with ether. The aqueous layer was acidified to approximately pH 2 by the addition of dilute HCl and the mixture was extracted three times with EtOAc. The organic layers were dried over Na₂SO₄, filtered, and evaporated to give [5-(4'-hydroxy-biphenyl-4-sulfonylamino)-6,7,8,9-tetrahydro-5H-benzocyclohepten-1-yloxy]-acetic acid (45 mg, 50% yield). ¹H NMR (300 MHz, DMSO-*d₆*) δ: 9.71 (s, 1 H), 8.13 (d, J = 7.8 Hz, 1 H), 7.79 (d, J = 9.1 Hz, 2 H), 7.73 (d, J = 8.8 Hz, 2 H), 7.56 (d, J = 8.8 Hz, 2 H), 6.98 (t, J = 7.8 Hz, 1 H), 6.82-6.88 (m, 3 H), 6.67 (d, J = 8.2 Hz, 1 H), 4.56 (s, 2 H), 4.42 (br t, 1 H), 3.14-3.23 (m, 1 H), 1.42-1.76 (m, 6 H). HRMS [M+H] observed: 468.1480, calculated for C₂₅H₂₆NO₆S: 468.1475.

### Example 24

### {5-[(4'-Hydroxy-biphenyl-4-sulfonyl)-methyl-amino]-6,7,8,9-tetrahydro-5H-benzocyclohepten-1-yloxy}-acetic acid

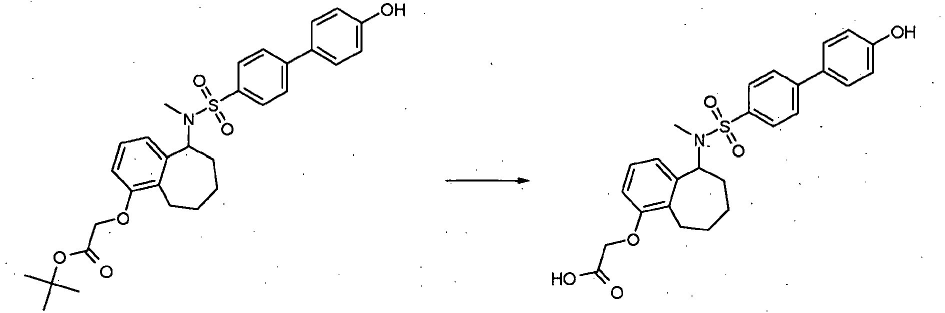

Using the conditions described for the preparation of Example 23, {5-[(4'-hydroxybiphenyl-4-sulfonyl)-methyl-amino]-6,7,8,9-tetrahydro-5H-benzocyclohepten-1-yloxy}-acetic acid tert-butyl ester (which may be prepared as described for Intermediate 3.09; 100 mg, 0.19 mmol) was hydrolyzed using 2 N NaOH solution (1.85 mL, 3.7 mmol) to give {5-[(4'-hydroxy-biphenyl-4-sulfonyl)-methyl-amino]-6,7,8,9-tetrahydro-5H-benzocyclohepten-1-yloxy}-acetic acid (45 mg, 50% yield). ¹H NMR (300 MHz, DMSO-*d₆*) δ: 9.76 (br s, 1 H), 7.88 (d, J = 8.8 Hz, 2 H), 7.84 (d, J = 8.8 Hz, 2 H), 7.60 (d, J = 8.8 Hz, 2 H), 7.10 (t, J = 8 Hz, 1 H), 6.88 (d, J = 8.5 Hz, 2 H), 6.84 (d, J = 8.2 Hz, 1 H), 6.75 (d, J = 8.5 Hz, 1 H), 5.09-5.12 (m, 1 H), 4.63 (s, 2 H), 3.43-3.50 (m, 1 H), 2.82 (s, 3 H), 2.32 (t, J = 12.7 Hz, 1 H), 12.95 (br s, 1 H), 1.66-1.86 (m, 2 H), 1.47-1.61 (m, 1 H), 1.25-1.38 (m, 2 H), 0.95-1.13 (m, 1 H). HRMS [M+H] observed: 482.1640, calculated for C₂₆H₂₈NO₆S: 482.1632.

### Example 25

### {5-[4-(5-Methyl-pyridin-3-yl)-benzenesulfonylamino]-6,7,8,9-tetrahydro-5H-benzocyclohepten-1-yloxy}-acetic acid

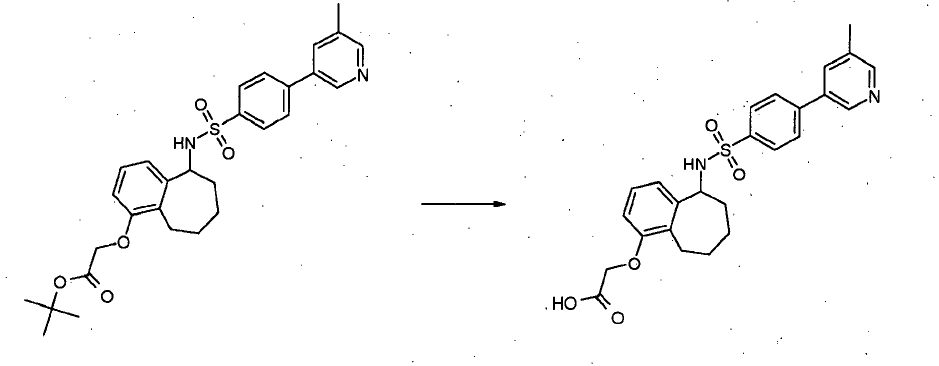

Using the conditions of General Procedure 7, {5-[4-(5-methyl-pyridin-3-yl)-benzenesulfonylamino]-6,7,8,9-tetrahydro-5H-benzocyclohepten-1-yloxy}-acetic acid tert-butyl ester (which may be prepared as described for Intermediate 2.15; 100 mg, 0.19 mmol) was hydrolyzed using 2 N NaOH solution (1.9 mL, 3.8 mmol) to give {5-[4-(5-methyl-pyridin-3-yl)-benzenesulfonylamino]-6,7,8,9-tetrahydro-5H-benzocyclohepten-1-yloxy}-acetic acid (65 mg, 73% yield). ¹H NMR (DMSO-d₆) δ: 13.00 (br. s., 1 H), 8.82 (d, J = 2.0 Hz, 1 H), 8.54 (s, 1 H), 8.32 (d, J = 7.8 Hz, 1 H), 8.05 (s, 1 H), 7.87-7.99 (m, 4 H), 7.02-7.17 (m, 1 H), 6.92 (d, J = 7.8 Hz, 1 H), 6.76 (d, J = 8.0 Hz, 1 H), 4.66 (s, 2 H), 4.54 (br. s., 1 H), 3.24 (br. s., 1 H), 2.61 (s, 1 H), 2.46 (s, 3 H), 1.80 (br. s., 1 H), 1.60 (d, J = 7.3 Hz, 4 H), 1.30 (br. s., 1 H). HRMS [M+H] observed: 467.1632, calculated for C₂₅H₂₆N₂NaO₅S: 467.1635.

### Example 26

### (5-{Methyl-[4-(5-methyl-pyridin-3-yl)-benzenesulfonyl]-amino}-6,7,8,9-tetrahydro-5H-benzocyclohepten-1-yloxy)-acetic acid

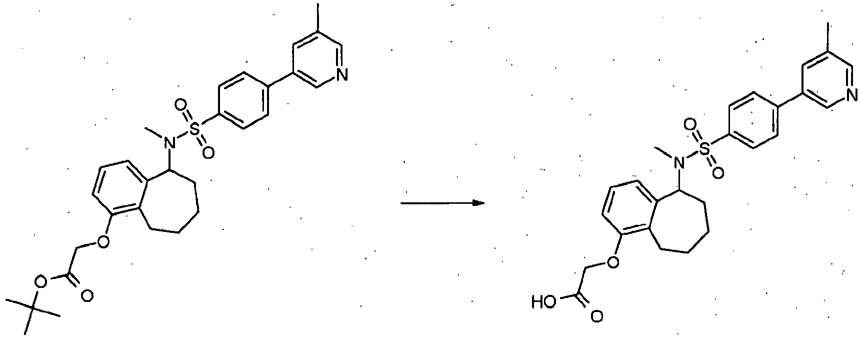

Using conditions similar to those described for Example 43, (5-{methyl-[4-(5-methyl-pyridin-3-yl)-benzenesulfonyl]-amino}-6,7,8,9-tetrahydro-5H-benzocyclohepten-1-yloxy)-acetic acid tert-butyl ester (which may be prepared as described for Intermediate 3.10; 45 mg, 0.08 mmol) was hydrolyzed using lithium hydroxide monohydrate (10 mg, 0.24 mmol) to give (5-{methyl-[4-(5-methyl-pyridin-3-yl)-benzenesulfonyl]-amino}-6,7,8,9-tetrahydro-5H-benzocyclohepten-1-yloxy)-acetic acid (20 mg, 50%). ¹H NMR (400 MHz, DMSO-*d*₆) δ: 8.79 (s, 1 H), 8.50 (s, 1 H), 7.97-8.03 (m, 5 H), 7.11 (t, *J*=8.1 Hz, 1 H), 6.84 (d, *J*=7.8 Hz, 1 H), 6.77 (d, *J*=8.3 Hz, 1 H), 5.15 (d, *J*=8.3 Hz, 1 H), 4.64 (s, 2 H), 3.44-3.53 (m, 1 H), 2.86 (s, 3 H), 2.40 (s, 3 H), 1.73-1.85 (m, 2 H), 1.55-1.61 (m, 1 H), 1.33-1.39 (m, 2 H), 0.95-1.13 (m, 1 H).

### Example 27

### [5-(3'-Methylsulfanyl-biphenyl-4-sulfonylamino)-6,7,8,9-tetrahydro-5H-benzocyclohepten-1-yloxy]-acetic acid

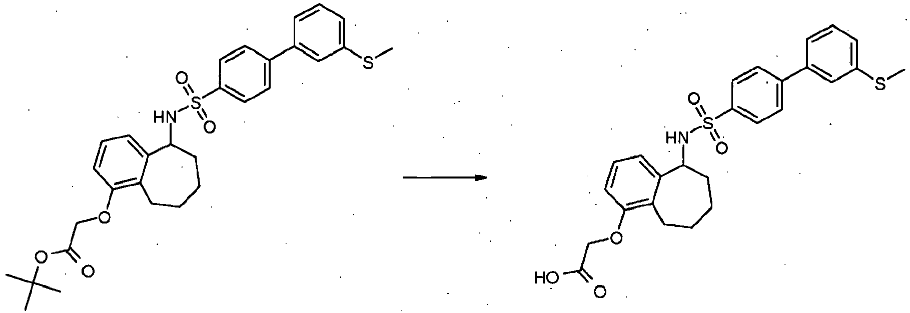

Using the conditions of General Procedure 7, [5-(3'-methylsulfanyl-biphenyl-4-sulfonylamino)-6,7,8,9-tetrahydro-5H-benzocyclohepten-1-yloxy]-acetic acid tert-butyl ester (which may be prepared as described for Intermediate 2.16; 50 mg, 0.09 mmol) was hydrolyzed using 2 N NaOH solution (0.9 mL, 1.8 mmol) to give [5-(3'-methylsulfanyl-biphenyl-4-sulfonylamino)-6,7,8,9-tetrahydro-5H-benzocyclohepten-1-yloxy]-acetic acid (18 mg, 40% yield) as an off-white solid. HPLC indicated that the purity of this sample was 81%. Impurities are visible in the NMR at δ 8.06-8.08, 7.96-8.00, and 7.76-7.84 ppm. ¹H NMR (400 MHz, DMSO-*d₆*) δ: 8.24-8.37 (m, 1 H), 7.93 (s, 4 H), 7.63 (s, 1 H), 7.54-7.58 (m, 1 H), 7.51 (t, J=7.5 Hz, 1 H), 7.39 (d, J=7.8 Hz, 1 H), 7.05 (t, J=8.0 Hz, 1 H), 6.91 (d, J=7.5 Hz, 1 H), 6.74 (d, J=8.3 Hz, 1 H), 4.49-4.63 (m, 3 H), 3.21-3.29 (m, 1 H), 2.74 (s, 1 H), 2.39 (s, 1 H), 1.78-1.83 (m, 1 H), 1.52-1.66 (m, 3 H), 1.25-1.35 (m, 2 H).

### Example 28

### {5-[Methyl-(3'-methylsulfanyl-biphenyl-4-sulfonyl)-amino]-6,7,8,9-tetrahydro-5H-benzocyclohepten-1-yloxy}-acetic acid

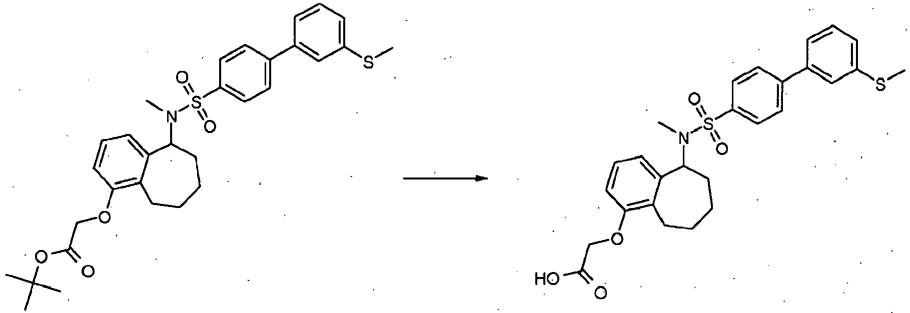

Using the conditions of General Procedure 7, {5-[methyl-(3'-methylsulfanyl-biphenyl-4-sulfonyl)-amino]-6,7,8,9-tetrahydro-5H-benzocyclohepten-1-yloxy}-acetic acid tert-butyl ester (which may be prepared as described for Intermediate 3.11; 40 mg, 0.07 mmol) was hydrolyzed using 2 N NaOH solution (0.7 mL, 1.4 mmol) to give {5-[methyl-(3'-methylsulfanyl-biphenyl-4-sulfonyl)-amino]-6,7,8,9-tetrahydro-5H-benzocyclohepten-1-yloxy}-acetic acid (25 mg, 69% yield) as a white solid. HPLC indicated that the purity of this sample was 82.5%. Impurities are visible in the NMR at δ 7.40, 7.09, 4.48, 2.80, and 1.25 ppm. ¹H NMR (400 MHz, CDCl₃) 8: 7.89 (d, *J*=8.3 Hz, 2 H), 7.69 (d, *J*=8.3 Hz, 2 H), 7.47 (s, 1 H), 7.35-7.38 (m, 2 H), 7.30 (d, *J*=7.3 Hz, 1 H), 7.89 (d, *J*=8.3 Hz, 1 H), 6.91 (d, *J*=7.3 Hz, 1 H), 6.69 (d, *J*=7.8 Hz, 1 H), 5.30 (d, *J*=9.8 Hz, 1 H), 4.63 (s, 2 H), 3.49 (dd, *J*=14.2, 7.3 Hz, 1 H), 2.93 (s, 3 H), 2.55 (s, 3 H), 2.45 (t, *J*=13.0 Hz, 1 H), 1.55-1.96 (m, 7 H).

### Example 29

### [5-(3'-Methanesulfonyl-biphenyl-4-sulfonylamino)-6,7,8,9-tetrahydro-5H-benzocyclohepten-1-yloxy]-acetic acid

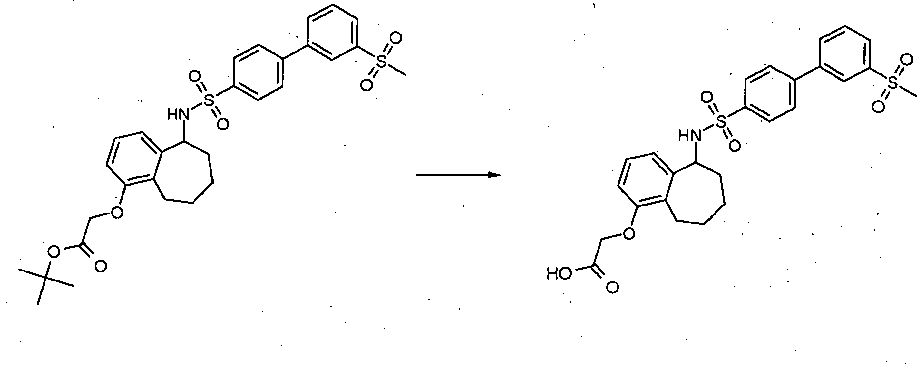

Using the conditions of General Procedure 7, [5-(3'-methanesulfonyl-biphenyl-4-sulfonylamino)-6,7,8,9-tetrahydro-5H-benzocyclohepten-1-yloxy]-acetic acid tert-butyl ester (which may be prepared as described for Intermediate 2.17; 100 mg, 0.17 mmol) was hydrolyzed using 2 N NaOH solution (1.66 mL, 3.3 mmol) to give [5-(3'-methanesulfonyl-biphenyl-4-sulfonylamino)-6,7,8,9-tetrahydro-5H-benzocyclohepten-1-yloxy]-acetic acid (75 mg, 83% yield) as an off-white solid. ¹H NMR (DMSO-d₆) δ: 8.28 (d, J = 7.8 Hz, 1 H), 8.23 (s, 1 H), 8.10 (d, J = 8.0 Hz, 1 H), 7.99 (s, 1 H), 7.95 (s, 2 H), 7.88-7.93 (m, 2 H), 7.76-7.83 (m, 1 H), 6.96-7.04 (m, 1 H), 6.86 (d, J = 7.8 Hz, 1 H), 6.70 (d, J = 8.3 Hz, 1 H), 4.59 (s, 2 H), 4.48 (br. s., 1 H), 3.33 (s, 3 H), 3.12-3.25 (m, 1 H), 2.54 (s, 1 H), 1.73 (d, J = 4.5 Hz, 1 H), 1.54 (d, J = 8.8 Hz, 4 H), 1.23 (br. s., 1 H). HRMS [M+Na] observed: 552.1117, calculated for C₂₆H₂₇NNaO₇S₂: 552.1121.

### Example 30

### {5-[(3'-Methanesulfonyl-biphenyl-4-sulfonyl)-methyl-amino]-6,7,8,9-tetrahydro-5H-benzocyclohepten-1-yloxy}-acetic acid

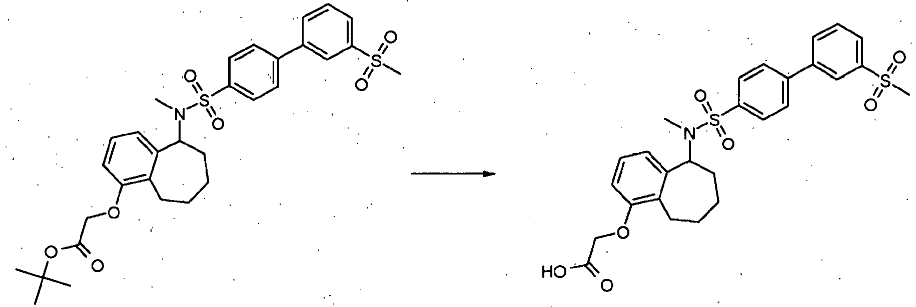

Using the conditions of General Procedure 7, {5-[(3'-methanesulfonyl-biphenyl-4-sulfonyl)-methyl-amino]-6,7,8,9-tetrahydro-5H-benzocyclohepten-1-yloxy} -acetic acid tert-butyl ester (which may be prepared as described for Intermediate 3.12; 100 mg, 0.17 mmol) was hydrolyzed using 2 N NaOH solution (1.66 mL, 3.3 mmol) to give {5-[(3'-methanesulfonyl-biphenyl-4-sulfonyl)-methyl-amino]-6,7,8,9-tetrahydro-5H-benzocyclohepten-1-yloxy}-acetic acid (30 mg, 33% yield) as a brown solid. ¹H NMR (400 MHz, DMSO-d₆) δ: 8.27 (s, 1 H), 8.15 (d, *J*=8.0 Hz, 1 H), 7.97-8.09 (m, 5 H), 7.81 (t, *J*=7.8 Hz, 1 H), 7.12 (t, *J*=8.2 Hz, 1 H), 6.85 (d, *J*=7.8 Hz, 1 H), 6.77 (d, *J*=8.3 Hz, 1 H), 5.12-5.20 (m, 1 H), 4.65 (s, 2 H), 3.48 (dd, *J*=14.05, 6.78 Hz, 1 H), 2.87 (s, 3 H), 2.54 (s, 3 H), 2.29-2.43 (m, 1 H), 1.71-1.90 (m, 2 H), 1.58 (d, *J*=11.8 Hz, 1 H), 1.18-1.46 (m, 3 H), 0.95-1.13 (m, 1 H). HRMS [M+H⁺] observed: 544.1465, calculated for C₂₇H₃₀NO₇S₂: 544.1458.

### Example 31

### {5-[5-(3-Isopropyl-phenyl)-pyridine-2-sulfonylamino]-6,7,8,9-tetrahydro-5H-benzocyclohepten-1-yloxy}-acetic acid

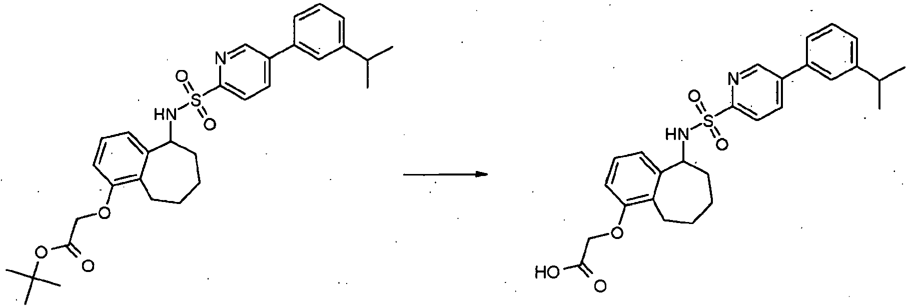

Using the conditions described for the preparation of Example 23, {5-[5-(3-isopropylphenyl)-pyridine-2-sulfonylamino]-6,7,8,9-tetrahydro-5H-benzocyclohepten-1-yloxy}-acetic acid tert-butyl ester (which may be prepared as described for Intermediate 2.18; 100 mg, 0.18 mmol) was hydrolyzed using 2 N NaOH solution (1.8 mL, 3.6 mmol) to give {5-[5-(3-isopropyl-phenyl)-pyridine-2-sulfonylamino]-6,7,8,9-tetrahydro-5H-benzocyclohepten-1-yloxy}-acetic acid (55 mg, 61% yield). ¹H NMR (300 MHz, DMSO-*d₆*) δ: 12.92 (br s, 1 H), 9.00 (d, J = 1.8 Hz, 1 H), 8.43 (d, J = 8.2 Hz, 1 H), 8.26 (dd, J = 2.4, 8.2 Hz, 1 H), 7.90 (d, J = 8.5 Hz, 1 H), 7.64 (s, 1 H), 7.59 (d, J = 7.8 Hz, 1 H), 7.45 (t, J = 7.5 Hz, 1 H), 7.36 (d, J = 7.8 Hz, 1 H), 6.98 (t, J = 7.8 Hz, 1 H), 6.90 (d, J = 7.5 Hz, 1 H), 6.66 (d, J = 7.5 Hz, 1 H), 4.68 (t, J = 7.8 Hz, 1 H), 4.55 (s, 2 H), 3.20-3.28 (m, 1 H), 2.99 (p, J = 6.9 Hz, 1 H), 1.46-1.78 (m, 6 H), 1.26 (d, J = 6.9 Hz, 6 H). HRMS [M+H⁺] observed: 495.1959, calculated for C₂₇H₃₁N₂O₅S: 495.1948.

### Example 32

### (5-{[5-(3-Isopropyl-phenyl)-pyridine-2-sulfonyl]-methyl-amino}-6,7,8,9-tetrahydro-5H-benzocyclohepten-1-yloxy)-acetic acid

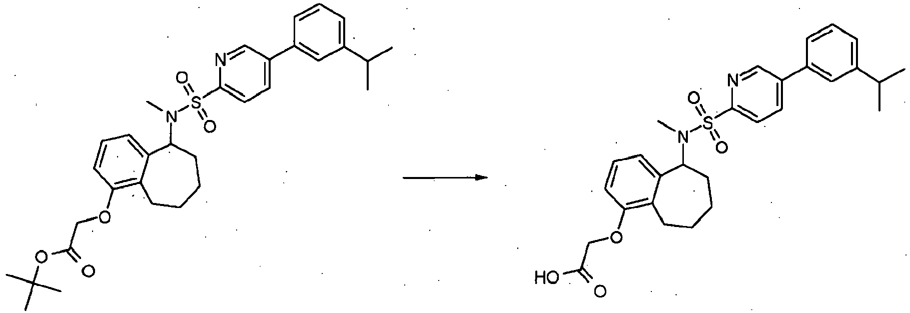

Using the conditions described for the preparation of Example 23, (5-{ [5-(3-isopropyl-phenyl)-pyridine-2-sulfonyl]-methyl-amino}-6,7,8,9-tetrahydro-5H-benzocyclohepten-1-yloxy)-acetic acid tert-butyl ester (which may be prepared as described for Intermediate 3.13; 90 mg, 0.16 mmol) was hydrolyzed using 2 N NaOH solution (1.7 mL, 3.4 mmol) to give (5-{[5-(3-isopropyl-phenyl)-pyridine-2-sulfonyl]-methyl-amino}-6,7,8,9-tetrahydro-5H-benzocyclohepten-1-yloxy)-acetic acid (40 mg, 49% yield). ¹H NMR (DMSO-d₆) δ: 9.13 (d, J = 1.8 Hz, 1 H), 8.39 (dd, J = 8.3, 2.3 Hz, 1 H), 8.03 (d, J = 8.3 Hz, 1 H), 7.71 (s, 1 H), 7.65 (d, J = 7.5 Hz, 1 H), 7.48 (t, J = 7.7 Hz, 1 H), 7.39 (d, J = 7.5 Hz, 1 H), 7.10 (t, J = 8.0 Hz, 1 H), 6.85 (d, J = 7.8 Hz, 1 H), 6.74 (d, J = 8.3 Hz, 1 H), 5.26 (d, J = 10.3 Hz, 1 H), 4.54 (br. s., 2 H), 3.50 (dd, J = 14.2, 6.7 Hz, 1 H), 2.88-3.07 (m, 4 H), 2.26 (br. s., 1 H), 1.37-1.91 (m, 5 H), 1.28 (d, J=7.0 Hz, 6 H), 1.00-1.14 (m, 1 H). HRMS [M+Na] observed: 531.1925, calculated for C₂₈H₃₂N₂NaO₅S: 531.1924.

### Example 33

### {5-[5-(3-Trifluoromethyl-phenyl)-pyridine-2-sulfonylamino]-6,7,8,9-tetrahydro-5H-benzocyclohepten-1-yloxy}-acetic acid

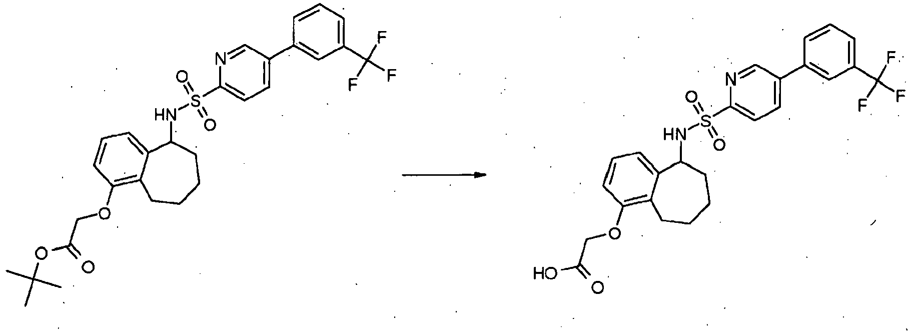

Using the conditions described for the preparation of Example 23, {5-[5-(3-trifluoromethyl-phenyl)-pyridine-2-sulfonylamino]-6,7,8,9-tetrahydro-5H-benzocyclohepten-1-yloxy}-acetic acid tert-butyl ester (which may be prepared as described for Intermediate 2.19; 50 mg, 0.09 mmol) was hydrolyzed using 2 N NaOH solution (0.8 mL, 1.6 mmol) to give {5-[5-(3-trifluoromethyl-phenyl)-pyridine-2-sulfonylamino]-6,7,8,9-tetrahydro-5H-benzocyclohepten-1-yloxy}-acetic acid (30 mg, 66% yield). ¹H NMR (300 MHz, DMSO-*d₆*) δ 12.94 (br s, 1 H), 9.10 (d, J = 2.1 Hz, 1 H), 8.49 (d, J = 7.8 Hz, 1 H), 8.38 (dd, J = 2.3, 8.3 Hz, 1 H), 8.15 (s, 1 H), 8.12 (d, J = 7.8 Hz, 1 H), 7.93 (d, J = 8.2 Hz, 1 H), 7.85 (d, J = 8.1 Hz, 1 H), 7.77 (t, J = 7.5 Hz, 1 H), 6.98 (t, J = 8.0 Hz, 1 H), 6.90 (d, J = 7.5 Hz, 1 H), 6.66 (d, J = 8.1 Hz, 1 H), 4.69 (t, J = 8.5 Hz, 1 H), 4.55 (s, 2 H), 3.20-3.26 (m, 1 H), 1.50-1.78 (m, 5 H), 1.15-1.26 (m, 1 H). HRMS [M+H⁺] observed: 521.1360, calculated for C₂₅H₂₄F₃N₂O₅S: 521.1353:

### Example 34

### (5-{Methyl-[5-(3-trifluoromethyl-phenyl)-pyridine-2-sulfonyl]-amino}-6,7,8,9-tetrahydro-5H-benzocyclohepten-1-yloxy)-acetic acid

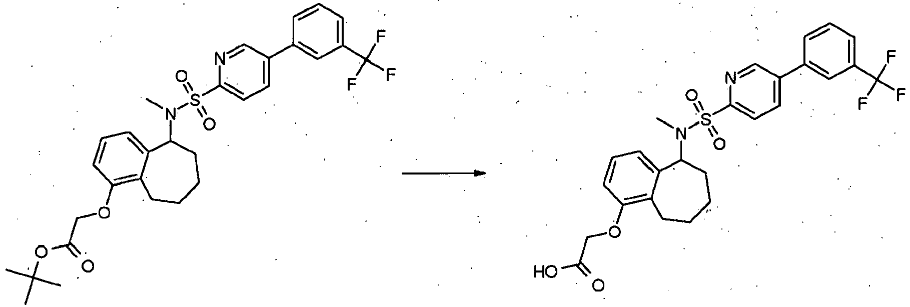

Using the conditions of General Procedure 7, (5-{methyl-[5-(3-trifluoromethyl-phenyl)-pyridine-2-sulfonyl]-amino}-6,7,8,9-tetrahydro-5H-benzocyclohepten-1-yloxy)-acetic acid tert-butyl ester (which may be prepared as described for Intermediate 3.14; 40 mg, 0.07 mmol) was hydrolyzed using 2 N NaOH solution (0.7 mL, 1.4 mmol) to give (5-{methyl-[5-(3-trifluoromethyl-phenyl)-pyridine-2-sulfonyl]-amino}-6,7,8,9-tetrahydro-5H-benzocyclohepten-1-yloxy)-acetic acid (23 mg, 64% yield). ¹H NMR (300 MHz, DMSO-*d₆*) δ 8.20 (d, J = 2.1 Hz, 1 H), 8.48 (dd, J = 2.4, 8.1 Hz, 1 H), 8.20 (s, 1 H), 8.16 (d, J = 8.4 Hz, 1 H), 8.05 (d, J = 8.2 Hz, 1 H), 7.84-7.87 (m, 1 H), 7.78 (t, J = 7.7 Hz, 1 H), 7.07 (t, J = 8.0 Hz, 1 H), 6.82 (d, J = 7.8 Hz, 1 H), 6.71 (d, J = 8.1 Hz, 1 H), 5.25 (d, J = 10.3 Hz, 1 H), 4.48 (s, 2 H), 3.52-3.44 (m, 1 H), 2.21-2.30 (m, 1 H), 1.88-1.44 (m, 5 H), 1.04-1.12 (m, 1 H). HRMS [M+H⁺] observed: 535.1514, calculated for C₂₆H₂₆F₃N₂O₅S: 535.1509.

### Example 35

### {5-[5-(3-tert-Butyl-5-methyl-phenyl)-pyridine-2-sulfonylamino]-6,7,8,9-tetrahydro-5H-benzocyclohepten-1-yloxy}-acetic acid

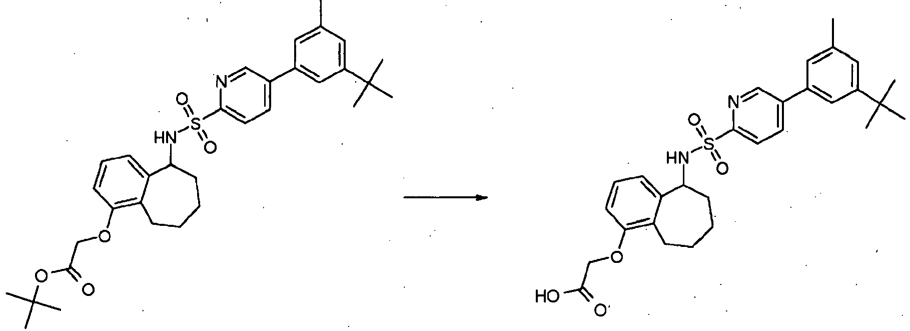

Using the conditions described for the preparation of Example 23, {5-[5-(3-tert-butyl-5-methyl-phenyl)-pyridine-2-sulfonylamino]-6,7,8,9-tetrahydro-5H-benzocyclohepten-1-yloxy}-acetic acid tert-butyl ester (which may be prepared as described for Intermediate 2.20; 100 mg, 0.17 mmol) was hydrolyzed using 2 N NaOH solution (1.7 mL, 3.4 mmol) to give {5-[5-(3-tert-butyl-5-methyl-phenyl)-pyridine-2-sulfonylamino]-6,7,8,9-tetrahydro-5H-benzocyclohepten-1-yloxy}-acetic acid (40 mg, 44% yield). ¹H NMR (300 MHz, DMSO-*d₆*) δ: 12.96 (br s, 1 H), 8.98 (d, J = 1.5 Hz, 1 H), 8.43 (d, J = 7.8 Hz, 1 H), 8.2.5 (dd, J = 2.4, 8.2 Hz, 1 H), 7.89 (d, J = 8.2 Hz, 1 H), 7.52 (s, 1 H), 7.40 (s, 1 H), 7.32 (s, 1 H), 6.97 (t, J = 7.8 Hz, 1 H), 6.90 (d, J = 7.8 Hz, 1 H), 6.65 (d, J = 7.8 Hz, 1 H), 4.68 (t, 1 H), 4.52 (s, 2 H), 3.20-3.28 (m, 1 H), 2.38 (s, 3 H), 1.50-1.80 (m, 5 H), 1.32 (s, 9 H), 1.15-1.25 (m, 1 H). HRMS [M+H⁺] observed: 523.2270, calculated for C₂₉H₃₅N₂O_{S}S: 523.2261.

### Example 36

### (5-{[5-(3-tert-Butyl-5-methyl-phenyl)-pyridine-2-sulfonyl]-methyl-amino}-6,7,8,9-tetrahydro-5H-benzocyclohepten-1-yloxy)-acetic acid

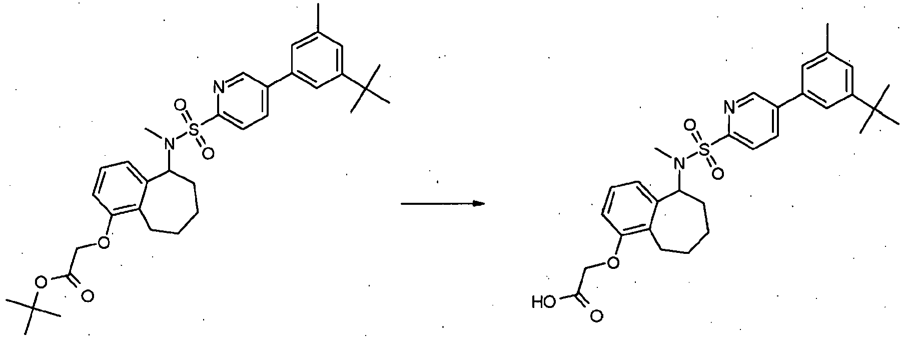

Using the conditions of General Procedure 7, (5-{[5-(3-tert-butyl-5-methyl-phenyl)-pyridine-2-sulfonyl]-methyl-amino}-6,7,8,9-tetrahydro-5H-benzocyclohepten-1-yloxy)-acetic acid tert-butyl ester (which may be prepared as described for Intermediate 3.15; 150 mg, 0.25 mmol) was hydrolyzed using 2 N NaOH solution (2.6 mL, 5.2 mmol) to give (5-{[5-(3-tert-butyl-5-methyl-phenyl)-pyridine-2-sulfonyl]-methyl-amino}-6,7,8,9-tetrahydro-5H-benzocyclohepten-1-yloxy)-acetic acid (50 mg, 37% yield). ¹H NMR (DMSO-d₆) δ: 9.11 (d, J = 1.8 Hz, 1 H), 8.37 (dd, J = 8.2, 2.1 Hz, 1 H), 8.02 (d, J = 8.0 Hz, 1 H), 7.59 (s, 1 H), 7.47 (s, 1 H), 7.34 (s, 1 H), 7.07 (t, J = 8.0 Hz, 1 H), 6.82 (d, J = 7.8 Hz, 1 H), 6.71 (d, J = 8.3 Hz, 1 H), 5.25 (d, J = 10.0 Hz, 1 H), 4.35-4.75 (m, 2 H), 3.50 (dd, J = 13.9, 6.9 Hz, 1 H), 2.96 (s, 3 H), 2.40 (s, 3 H), 2.28 (s, 1 H), 0.84-1.99 (m, 15 H). HRMS [M+H] observed: 537.2415, calculated for C₃₀H₃₇N₂O₅S: 537.2418.

### Example 37

### (5-{5-[3-(2-Hydroxy-ethyl)-phenyl]-pyridine-2-sulfonylamino}-6,7,8,9-tetrahydro-5H-benzocyclohepten-1-yloxy)-acetic acid

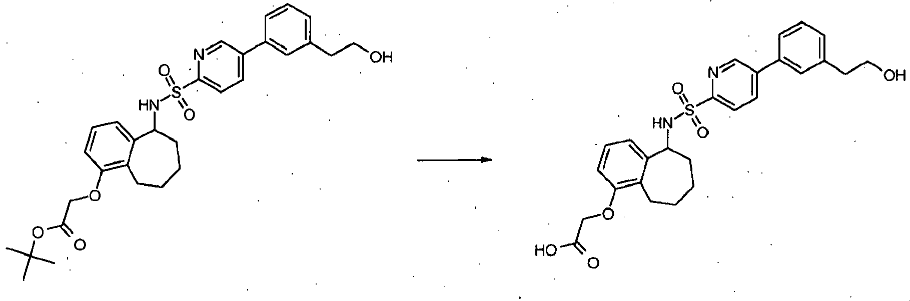

Using the conditions described for the preparation of Example 23, (5-{5-[3-(2-hydroxy-ethyl)-phenyl]-pyridine-2-sulfonylamino}-6,7,8,9-tetrahydro-5H-benzocyclohepten-1-yloxy)-acetic acid tert-butyl ester (which may be prepared as described for Intermediate 2.21; 100 mg, 0.18 mmol) was hydrolyzed using 2 N NaOH solution (1.8 mL, 3.6 mmol) to give (5-{5-[3-(2-hydroxy-ethyl)-phenyl]-pyridine-2-sulfonylamino}-6,7,8,9-tetrahydro-5H-benzocyclohepten-1-yloxy)-acetic acid (52 mg, 58% yield). ¹H NMR (300 MHz, DMSO-*d₆*) δ: 8.99 (d, J = 1.8 Hz, 1 H), 8.43 (d, J = 7.8 Hz, 1 H), 8.25 (dd, J = 2.4, 8.2 Hz, 1 H), 7.90 (d, J = 7.5 Hz, 1 H), 7.64 (s, 1 H), 7.60 (d, J = 7.8 Hz, 1 H), 7.43 (t, J = 7.7 Hz, 1 H), 7.32 (d, J = 7.8 Hz, 1 H), 6.97 (t, J = 7.8 Hz, 1 H), 6.89 (d, J = 7.3 Hz, 1 H), 6.66 (d, J = 7.8 Hz, 1 H), 4.67 (br t, 2 H), 4.54 (s, 2 H), 3.66 (t, J = 6.9 Hz, 2 H), 3.20-3.28 (m, 1 H), 2.81 (t, J = 6.9 Hz, 2 H), 1.78-1.46 (m, 6 H). HRMS [M+H⁺] observed: 497.1748, calculated for C₂₆H₂₉N₂O₆S: 497.1741.

### Example 38

### [5-({5-[3-(2-Hydroxy-ethyl)-phenyl]-pyridine-2-sulfonyl}-methyl-amino)-6,7,8,9-tetrahydro-5H-benzocyclohepten-1-yloxy]-acetic acid

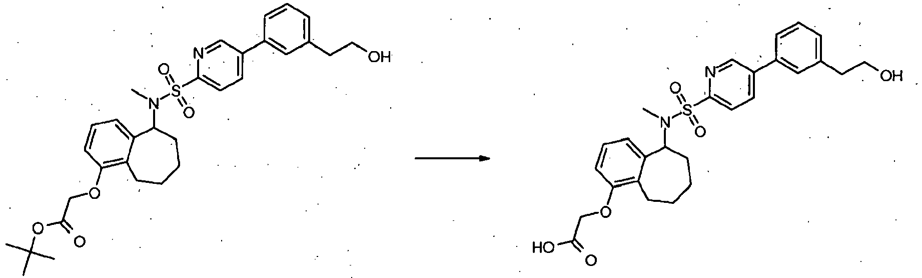

Using the conditions of General Procedure 7, [5-({5-[3-(2-hydroxy-ethyl)-phenyl]-pyridine-2-sulfonyl}-methyl-amino)-6,7,8,9-tetrahydro-5H-benzocyclohepten-1-yloxy]-acetic acid tert-butyl ester (which may be prepared as described for Intermediate 3.16; 100 mg, 0.17 mmol) was hydrolyzed using 2 N NaOH solution (1.8 mL, 3.6 mmol) to give [5-({5-[3-(2-hydroxy-ethyl)-phenyl]-pyridine-2-sulfonyl}-methyl-amino)-6,7,8,9-tetrahydro-5H-benzocyclohepten-1-yloxy]-acetic acid (20 mg, 22% yield). ¹H NMR (DMSO-d₆) δ: 9.12 (d, J = 1.8 Hz, 1 H), 8.37 (dd, J = 8.3, 2.3 Hz, 1 H), 8.04 (d, J = 8.3 Hz, 1 H), 7.63-7.72 (m, 2 H), 7.46 (t, J = 7.7 Hz, 1 H), 7.36 (d, J = 7.8 Hz, 1 H), 7.07-7.16 (m, 1 H), 6.86 (d, J = 7.8 Hz, 1 H), 6.76 (d, J = 8.3 Hz, 1 H), 5.26 (d, J = 10.3 Hz, 1 H), 4.62 (br. s., 2 H), 3.68 (t, J = 6.9 Hz, 2 H), 3.50 (dd, J = 13.9, 6.7 Hz, 1 H), 2.96 (s, 3 H), 2.83 (s, 2 H), 2.21-2.36 (m, 1 H), 0.93-1.92 (m, 6 H). HRMS [M+Na] observed: 533.1717, calculated for C₂₇H₃₀N₂NaO₆S: 533.1717.

### Example 39

### [5-(4'-Methyl-biphenyl-3-sulfonylamino)-6,7,8,9-tetrahydro-5H-benzocyclohepten-1-yloxy]-acetic acid

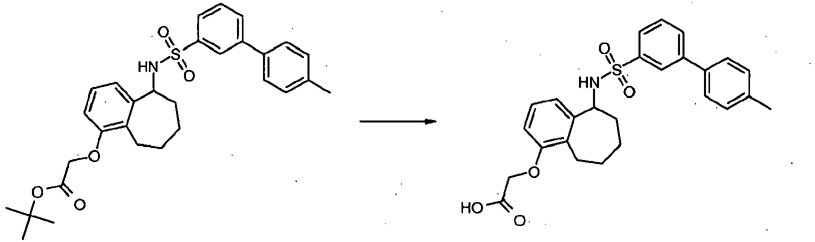

Using the conditions of General Procedure 7, [5-(4'-methyl-biphenyl-3-sulfonylamino)-6,7,8,9-tetrahydro-5H-benzocyclohepten-1-yloxy]-acetic acid tert-butyl ester (which may be prepared as described for Intermediate 2.22; 125 mg, 0.23 mmol) was hydrolyzed using 2 N NaOH solution (2.8 mL, 5.6 mmol). The product was purified by preparative TLC to give [5-(4'-methyl-biphenyl-3-sulfonylamino)-6,7,8,9-tetrahydro-5H-benzocyclohepten-1-yloxy]-acetic acid (15 mg, 13% yield). ¹H NMR (300 MHz, DMSO-*d₆*) δ: 12.93 (br. s., 1 H), 8.21 (d, J=7.8 Hz, 1 H), 7.99 (s, 1 H), 7.88 (d, J=7.8 Hz, 1 H), 7.77 (d, J=7.8 Hz, 1 H), 7.57-7.67 (m, 3 H), 7.46-7.54 (m, 2 H), 7.42 (d, J=7.2 Hz, 1 H), 6.90-7.00 (m, 1 H), 6.81 (d, J=7.5 Hz, 1 H), 6.66 (d, J=8.2 Hz, 1 H), 4.55 (s, 2 H), 4.40-4.52 (m, 1 H), 3.16 (dd, J=13.9, 7.2 Hz, 1 H), 1.67-1.76 (m, 1 H), 1.44-1.56 (m, 3 H), 1.16-1.28 (m, 1 H). HRMS [M+Na] observed: 452.1531, calculated for C₂₆H₂₈NO₅S: 452.1526.

### Example 40

### {5-[Methyl-(4'-methyl-biphenyl-3-sulfonyl)-amino]-6,7,8,9-tetrahydro-5H-benzocyclohepten-1-yloxy}-acetic acid

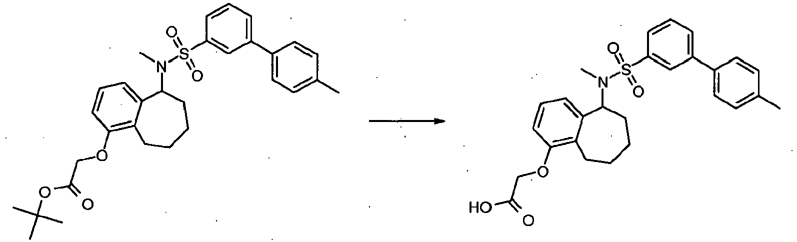

Using the conditions of General Procedure 7, {5-[methyl-(4'-methyl-biphenyl-3-sulfonyl)-amino]-6,7,8,9-tetrahydro-5H-benzocyclohepten-1-yloxy}-acetic acid tert-butyl ester (which may be prepared as described for Intermediate 3.17; 100 mg, 0.19 mmol) was hydrolyzed using 2 N NaOH solution (1.86 mL, 3.7 mmol) to give {5-[methyl-(4'-methyl-biphenyl-3-sulfonyl)-amino]-6,7,8,9-tetrahydro-5H-benzocyclohepten-1-yloxy}-acetic acid (30 mg, 34% yield). ¹H NMR (400 MHz, DMSO-*d*₆) 8: 7.96-8.04 (m, 2 H), 7.87 (d, *J*=7.8 Hz, 1 H), 7.69-7.78 (m, 1 H), 7.63 (d, *J*=7.8 Hz, 2 H), 7.33 (d, *J*=7.3 Hz, 2 H), 7.10 (t, *J*=8.1 Hz, 1 H), 6.83 (d, *J*=8.3 Hz, 1 H), 6.76 (d, *J*=7.8 Hz, 1 H), 5.18 (d, *J*=8.3 Hz, 1 H), 4.63 (br. s., 2 H), 3.43-3.54 (m, 1 H), 2.87 (s, 3 H), 2.37 (s, 3 H), 1.70-1.84 (m, 2 H), 1.14-1.58 (m, 4 H), 0.99-1.09 (m, 1 H). HRMS [M+H⁺] observed: 480.1847, calculated for C₂₇H₃₀NO₅S: 480.1839.

### Example 41

### [5-(3'-Isopropyl-biphenyl-3-sulfonylamino)-6,7,8,9-tetrahydro-5H-benzocyclohepten-1-yloxy]-acetic acid

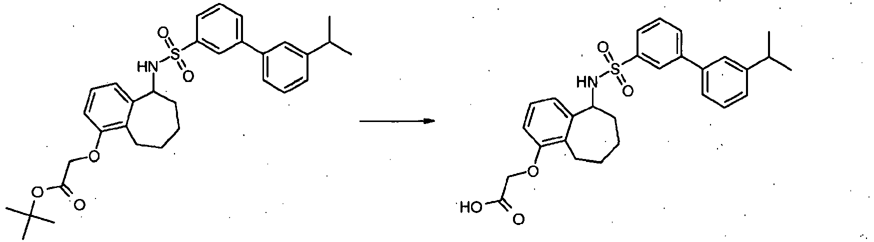

Using the conditions of General Procedure 7, [5-(3'-isopropyl-biphenyl-3-sulfonylamino)-6,7,8,9-tetrahydro-5H-benzocyclohepten-1-yloxy]-acetic acid tert-butyl ester (which may be prepared as described for Intermediate 2.23; 25 mg, 0.05 mmol) was hydrolyzed using 2 N NaOH solution (1 mL, 2 mmol) to give [5-(3'-isopropyl-biphenyl-3-sulfonylamino)-6,7,8,9-tetrahydro-5H-benzocyclohepten-1-yloxy]-acetic acid (18 mg, 80% yield). ¹H NMR (400 MHz, CDCl₃) *J*: 7.97 (s, 1 H), 7.70 (d, *J*=7.9 Hz, 2 H), 7.42-7.48 (m, 1 H), 7.30-7.40 (m, 3 H), 6.85 (t, *J*=7.9 Hz, 1 H), 6.63 (d, *J*=8.5 Hz, 1 H), 6.58 (d, *J*=7.3 Hz, 1 H), 5.00 (d, *J*=6.1 Hz, 1 H), 4.53-4.61 (m, 1 H), 4.38 (br. s., 2 H), 3.47 (q, *J*=7.3 Hz, 1 H), 2.91-2.99 (m, 1 H), 2.80-2.88 (m, 1 H), 1.43-1.93 (m, 5 H), 1.23-1.32 (m, 6 H).

### Example 42

### {5-[(3'-Isopropyl-biphenyl-3-sulfonyl)-methyl-amino]-6,7,8,9-tetrahydro-5H-benzocyclohepten-1-yloxy} -acetic acid

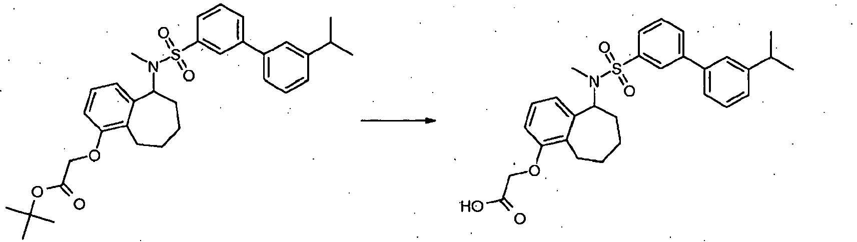

Using the conditions of General Procedure 7, {5-[(3'-isopropyl-biphenyl-3-sulfonyl)-methyl-amino]-6,7,8,9-tetrahydro-5H-benzocyclohepten-1-yloxy}-acetic acid tert-butyl ester (which may be prepared as described for Intermediate 3.18; 80 mg, 0.14 mmol) was hydrolyzed using 2 N NaOH solution (1.4 mL, 2.8 mmol) to give {5-[(3'-isopropyl-biphenyl-3-sulfonyl)-methyl-amino]-6,7,8,9-tetrahydro-5H-benzocyclohepten-1-yloxy}-acetic acid (30 mg, 42% yield). ¹H NMR (400 MHz, DMSO-*d*₆) δ: 12.97 (br. s., 1 H), 7.98-8.06 (m, 2 H), 7.89 (d, *J*=7.8 Hz, 1 H), 7.71-7.78 (m, 1 H), 7.56 (s, 1 H), 7.53 (d, *J*=7.8 Hz, 1 H), 7.44 (t, *J*=7.6 Hz, 1 H), 7.33 (d, *J*=7.3 Hz, 1 H), 7.10 (t, *J*=8.1 Hz, 1 H), 6.83 (d, *J*=7.8 Hz, 1 H), 6.77 (d, *J*=8.3 Hz, 1 H), 5.20 (d, *J*=7.8 Hz, 1 H), 4.66 (s, 2 H), 3.45-3.54 (m, 1 H), 2.96-3.06 (m, 1 H), 2.87 (s, 3 H), 2.31-2.43 (m, 1 H), 1.72-1.85 (m, 2 H), 1.51-1.61 (m, 1 H), 1.33-1.40 (m, 2 H), 1.27 (d, *J*=6.8 Hz, 6 H), 0.98-1.10 (m, 1 H). HRMS [M+H⁺] observed: 508.2159, calculated for C₂₉H₃₄NO₅S: 508.2152.

### Example 43

[5-(3-Isopropyl-5-trifluoromethyl-benzenesulfonylamino)-6,7,8,9-tetrahydro-5H-benzocyclohepten-1-yloxy]-acetic acid

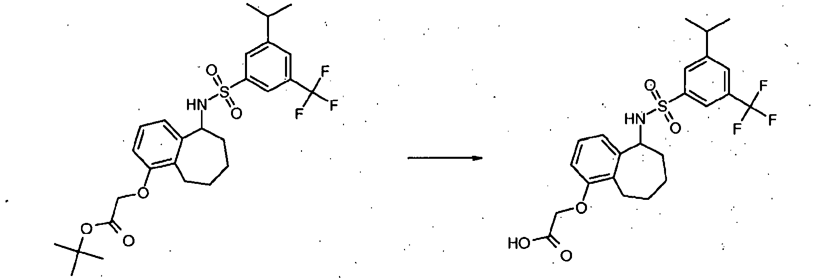

Lithium hydroxide monohydrate (8 mg, 0.19 mmol) was added to a mixture of [5-(3-isopropyl-5-trifluoromethyl-benzenesulfonylamino)-6,7,8,9-tetrahydro-5H-benzocyclohepten-1-yloxy]-acetic acid tert-butyl ester (which may be prepared as described for Intermediate 2.24; 30 mg, 0.06 mmol), THF (4 mL), MeOH (1 mL) and water (1 mL). The mixture was stirred for 16 h, then concentrated and diluted with water (10 mL). The pH of the mixture was adjusted to ∼4 by adding 2N HCl and the mixture was then extracted with EtOAc. The EtOAc was evaporated and the residue was washed with pentane to give [5-(3-isopropyl-5-trifluoromethyl-benzenesulfonylamino)-6,7,8,9-tetrahydro-5H-benzocyclohepten-1-yloxy]-acetic acid (23 mg, 86%) as an off-white solid. ¹H NMR (400 MHz, DMSO-*d*₆) δ: 8.35 (d, *J*=8.0 Hz, 1 H), 7.89 (s, 1 H), 7.79 (s, 2 H), 6.84-6.97 (m, 1 H), 6.70 (d, *J*=7.5 Hz, 1 H), 6.63 (d, *J*=8.3 Hz, 1 H), 4.46-4.54 (m, 3 H), 3.06 (dt, *J*=13.7, 6.7 Hz, 2 H), 2.66 (d, *J*=11.8 Hz, 1 H), 2.54 (s, 5 H), 2.33 (s, 1 H), 1.77 (br. s., 1 H), 1.43-1.62 (m, 4 H), 1.27-1.39 (m, 1 H), 1.20 (dd, *J*=7.0, 4.5 Hz, 6 H).

### Example 44

### {5-[(3-Isopropyl-5-trifluoromethyl-benzenesulfonyl)-methyl-amino]-6,7,8,9-tetrahydro-5H-benzocyclohepten-1-yloxy}-acetic acid

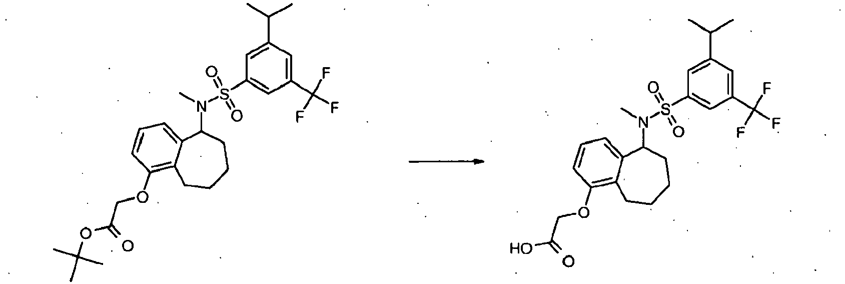

Using conditions similar to those described for Example 43, {5-[(3-isopropyl-5-trifluoromethyl-benzenesulfonyl)-methyl-amino]-6,7,8,9-tetrahydro-5H-benzocyclohepten-1-yloxy}-acetic acid tert-butyl ester (which was prepared as described for Intermediate 3.19; 120 mg of crude material, ∼0.18 mmol) was hydrolyzed using lithium hydroxide monohydrate (0.64 mmol), to give {5-[(3-isopropyl-5-trifluoromethyl-benzenesu]fonyl)-methyl-amino]-6,7,8,9-tetrahydro-5H-benzocyclohepten-1-yloxy}-acetic acid (60 mg, 65%) as a pale yellow solid. ¹H NMR (400 MHz, DMSO-*d*₆) δ: 12.97 (br. s., 1 H), 8.04 (s, 1 H), 7.97 (s, 1 H), 7.94 (s, 1 H), 7.08 (t, *J*=8.0 Hz, 1 H), 6.77 (d, *J*=8.0 Hz, 2 H), 5.20 (d, *J*=10.0 Hz, 1 H), 4.65 (s, 2 H), 3.47 (dd, *J*=14.2, 6.9 Hz, 1 H), 3.19 (dt, *J*=13.8, 6.9 Hz, 1 H), 2.84 (s, 3 H), 2.39 (t, *J*=12.8 Hz, 1 H), 1.72-1.86 (m, 2 H), 1.58 (q, *J*=12.5 Hz, 1 H), 1.32-1.44 (m, 1 H), 1.27 (dd, *J*=6.8, 4.5 Hz, 6 H), 1.03 (q, *J*=11.5 Hz, 1 H). HRMS [M+H⁺] observed: 500.1719, calculated for C₂₄H₂₉F₃NO₅S: 500.1713.

### Examples 45 and 46

### Chiral separation of [5-(3,5-Bis-trifluoromethyl-benzenesulfonylamino)-6,7,8,9-tetrahydro-5H-benzocyclohepten-1-yloxy]-acetic acid to give Enantiomer A and Enantiomer B

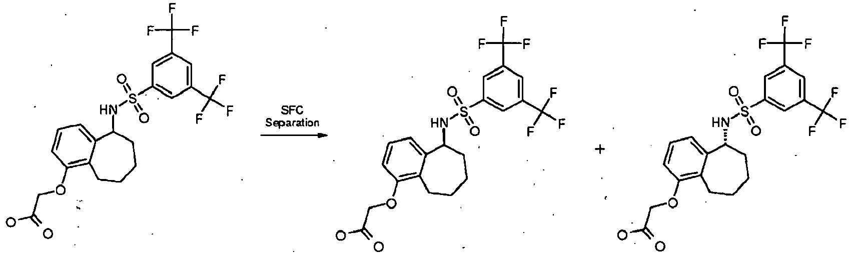

A sample of [5-(3,5-bis-trifluoromethyl-benzenesulfonylamino)-6,7,8,9-tetrahydro-5H-benzocyclohepten-1-yloxy]-acetic acid (which may be prepared as described in Example 1; 50 mg) was separated by supercritical fluid chromatography (SFC) using a Multigram® III instrument (from Thar Technologies, Inc.), using a Daicel OJ 3 x 25 cm column, and eluting with 8% MeOH/CO2 at a flow rate of 70 mL/min at 100 bar back-pressure, with monitoring at 220 nm. 42 mg of this material was purified using 10 injections of 5 mg each to give [5-(3,5-Bis-trifluoromethyl-benzenesulfonylamino)-6,7,8,9-tetrahydro-5H-benzocyclohepten-1-yloxy]-acetic acid Enantiomer A (Example 45, 8.6 mg, Retention Time: 3.46 min) and [5-(3,5-Bis-trifluoromethyl-benzenesulfonylamino)-6,7,8,9-tetrahydro-5H-benzocyclohepten-1-yloxy]-acetic acid Enantiomer B (Example 46, 11.1 mg, Retention Time: 4.26 min).

### Example 47

### Human CRTH2 Receptor Binding Assay

A whole cell receptor binding assay using [³H]ramatroban as the competing radioactive ligand was employed to evaluate the compound binding activity to human CRTH2. The radioactive ligand [³H]ramatroban was synthesized according to Sugimoto et. al. (Eur. J. Pharmacol. 2005, 524, 30-37) to a specific activity of 42 Ci/mmol.

A cell line stably expressing human CRTH2 was established by transfecting CHO-K1 cells with two mammalian expression vectors that harbored human CRTH2 and G-alpha16 cDNAs, respectively, using FuGene^{®} 6 transfection reagent (from Roche). Stable clones expressing CRTH2 were selected by staining each clone with BM16 (BD Pharmingen™ from BD Biosciences, a division of Becton, Dickinson and Company), which is a rat monoclonal antibody to human CRTH2. The cells were maintained as monolayer cultures in Ham's F-12 medium containing 10% fetal bovine serum, 100 units/mL penicillin, 100 µg/mL streptomycin, 2 mM glutamine, 0.5 mg/mL G418 (geneticin) for CRTH2, and 0.2 mg/mL hygromycin-B (for G-alpha 16). For whole cell receptor binding assay, the monolayer cells were rinsed once with PBS (phosphate buffered saline), dissociated using ethylenediaminetetraacetate (Versene™ EDTA from Lonza Inc.), and suspended in PBS containing 10 mM MgCl₂ and 0.06% BSA (bovine serum albumin) at 1.5 x 10⁶ cells/mL.

The binding reactions (0.2 mL) were performed in 96-well plates at room temperature in PBS containing 1.5 x 10⁵ cells, 10 mM MgCl₂, 0.06% BSA, 20 nM [³H]ramatroban, and test compound at various concentrations. After 1 hour of binding reactions, the cells were harvested on GF™/B filter microplates (microtiter plates with embedded glass fiber from PerkinElmer, Inc.) and washed 5 times with PBS using a Filtermate™ Harvester (a cell harvester that harvests and washes cells from microplates from PerkinElmer, Inc.). The radioactivities bound to the cells were determined using a microplate scintillation counter (TopCount^{®} NXT, from PerkinElmer, Inc.) after adding 50 µL of Microscint™ 20 scintillation fluid (from PerkinElmer, Inc.) to each well of the filter plates. The radioactivity from non-specific binding was determined by replacing compound with 10 µM of 15(R)-15-methyl PGD₂ (from Cayman Chemical Company) in the reaction mixtures. The radioactivity bound to the cells in the absence of compound (total binding) was determined by replacing compound with 0.25% of DMSO (dimethyl sulfoxide) in the reaction mixture. Specific binding data were obtained by subtracting the radioactivity of non-specific binding from each binding data.

The IC₅₀ value is defined as the concentration of the tested compound that is required for 50% inhibition of total specific binding. In order to calculate the IC₅₀ value, the percent inhibition data were determined for 7 concentrations for each compound. The percent inhibition for a compound at each concentration was calculated according to the following formula, [1-(specific binding in the presence of compound)/(total specific binding)]x100. The IC₅₀ value was then obtained by fitting the percent inhibition data to a sigmoidal dose-response (4 parameter logistic) model in the XLfit^{®} software Excel add-in program [from ID Business Solutions Ltd., model 205, where F(x) = (A+(B-A)/(1+((C/x)^D)))].

The acid compounds of the foregoing examples were tested using the above Human CRTH2 Receptor Binding Assay. The results of the assay showed that all of these compounds have binding activity exhibiting IC₅₀ values ranging from 0.0033 µM to 2.61 µM. For instance, the following table shows the specific IC₅₀ values for these compounds:

| **Example No.** | **Human CRTH2 Binding IC₅₀ (µM)** |
|---|---|
| 1 | 0.0089 |
| 2 | 0.0584 |
| 3 | 2.6069 |
| 4 | 0.0336 |
| 5 | 0.9525 |
| 6 | 0.1713 |
| 7 | 0.0646 |
| 8 | 0.047 |
| 9 | 0.0574 |
| 10 | 0.0503 |
| 11 | 0.0334 |
| 12 | 0.7502 |
| 13 | 0.1579 |
| 14 | 0.1057 |
| 15 | 0.0033 |
| 16 | 0.0059 |
| 17 | 0.0114 |
| 18 | 0.0114 |
| 19 | 0.013 |
| 20 | 0.1058 |
| 21 | 0.0347 |
| 22 | 0.1894 |
| 23 | 0.1031 |
| 24 | 0.9871 |
| 25 | 0.0337 |
| 26 | 1.4961 |
| 27 | 0.0243 |
| 28 | 0.6035 |
| 29 | 0.0091 |
| 30 | 0.189 |
| 31 | 0.0267 |
| 32 | 0.123 |
| 33 | 0.1775 |
| 34 | 0.4567 |
| 35 | 0.0179 |
| 36 | 0.1643 |
| 37 | 0.017 |
| 38 | 2.1203 |
| 39 | 0.5143 |
| 40 | 0.5548 |
| 41 | 0.2262 |
| 42 | 0.8453 |
| 43 | 0.2978 |
| 44 | 0.1992 |
| 45 | 1.4931 |
| 46 | 0.0245 |

### Example 48

### Calcium Flux Assay Using Fluorometric Imaging Plate Reader Cell Culture Conditions:

CHO-K1 cells previously transfected with G-alpha 16 were subsequently transfected with the human CRTH2 receptor and the neomycin resistance gene. Following selection in 800 µg/mL G418 (geneticin), individual clones were assayed for their receptor expression based on staining with an anti human CRTH2 IgG, followed by assaying for their response to 13,14-dihydro-15-keto Prostaglandin D₂ (DK-PDG₂) (ligand) in the Ca²⁺ Flux assay. Positive clones were then cloned by limiting dilution cloning. The transfected cells were cultured in Ham's F-12 medium supplemented with 10% fetal bovine serum, 2 mM glutamine , 100 U/mL penicillin/100 µg/mL streptomycin, 200 µg/mL hygromycin B, and 800 µg/mL G418 (geneticin). Cells were harvested with trypsin-EDTA (trypsin-ethylenediaminetetraacetic acid) and counted using ViaCount^{®} reagent (from Guava Technologies, Inc. which contains two DNA-binding dyes that enable the reagent user to distinguish between viable and non-viable cells). The cell suspension volume was adjusted to 2.5 x10⁵ cells /mL with complete growth media. Aliquots of 50 µL were dispensed into BD Falcon™ 384 well black/clear microplates (from BD Biosciences, a division of Becton, Dickinson and Company) and the microplates were placed in a 37 °C CO₂ incubator overnight. The following day, the microplates were used in the assay.

### Dye Loading and Assay:

Loading Buffer containing dye (from the FLIPR^{®} Calcium 3 Assay Kit from Molecular Devices, a division of MDS Analytical Technologies and MDS Inc.) was prepared by dissolving the contents of one bottle into 200 mL Hank's Balanced Salt Solution containing 20 mM HEPES (4-(2-hydroxyethyl)-1-piperazineethanesulfonic acid) and 2.5 mM probenecid. Growth media was removed from the cell plates and 25 µL of Hank's Balanced Salt Solution (HBSS) containing 20 mM HEPES, 0.05% BSA and 2.5 mM probenecid was added to each well followed by 25 µL of diluted dye using a Multidrop dispenser. The plates were then incubated for 1 hour at 37°C.

During the incubation, test compound plates were prepared by adding 90 µL of HBSS/20 mM HEPES/0.005% BSA buffer to the 2 µL of serial diluted compounds. To prepare serial diluted compounds, 20 mM stocks of compounds were dissolved in 100% DMSO. The compound dilution plate was set up as follows: well # 1 received 5 µL of compound plus 10 µL of DMSO. Wells 2-10 received 10 µL of DMSO. 5 µL was mixed and transferred from well #1 into well #2. 1:3 serial dilutions were continued out 10 steps. 2 µL of diluted compound was transferred into duplicate wells of a 384 well "assay plate" and then 90 µL of buffer was added.

After incubation, both the cell and "assay plate" plates were brought to the fluorometric imaging plate reader (FLIPR^{®}) and 20 µL of the diluted compounds were transferred to the cell plates by the FLIPR^{®}. Plates were then incubated for 1 hour at room temperature. After the 1 hour incubation, plates were returned to the FLIPR^{®} and 20 µL of 4.5X concentrated ligand was added to the cell plates. During the assay, fluorescence readings Were taken simultaneously from all 384 wells of the cell plate every 1.5 seconds. Five readings were taken to establish a stable baseline, then 20 µL of sample was rapidly (30 µL/sec) and simultaneously added to each well of the cell plate. The fluorescence was continuously monitored before, during and after sample addition for a total elapsed time of 100 seconds. Responses (increase in peak fluorescence) in each well following agonist addition were determined. The initial fluorescence reading from each well, prior to ligand stimulation, was used as a zero baseline value for the data from that well. The responses were expressed as % inhibition of the buffer control. The IC₅₀ value, defined as the concentration of a compound that was required for 50% inhibition of the buffer control, was calculated by fitting the percent inhibition data for 10 concentrations to a sigmoidal dose-response (4 parameter logistic) model using Genedata Screener^{®} Condoseo software program [from Genedata AG, model 205, where F(x) = (A+(B-A)/(1+((C/x)^D)))].

Specific representative compounds tested in the binding assay were tested using the above FLIPR^{®} assay. For instance, the following table shows the specific IC₅₀ values for these compounds:

| **Example No.** | **Human CRTH2 FLIPR^{®} IC₅₀ (µM)** |
|---|---|
| 1 | 0.114 |
| 2 | 0.3397 |
| 4 | 0.2548 |
| 5 | 0.581 |
| 8 | 0.0366 |
| 9 | 0.0061 |
| 23 | 0.0421 |
| 25 | 0.1232 |
| 29 | 0.0606 |
| 31 | 0.476 |
| 33 | 0.0292 |
| 34 | 4.3085 |
| 35 | 0.14 |
| 36 | 0.7835 |
| 37 | 0.214 |
| 41 | >5 |
| 46 | 0.1877 |

### Example 49

### DK-PGD₂-induced IL-13 production assay in Th2 cells

Inhibition of 13,14-dihydro-15-keto Prostaglandin D₂ (DK-PGD₂)-induced IL-13 production in T helper type 2 (Th2) cells was applied to evaluate compound cellular potency.

Cultures of Th2 cells were established from blood of healthy human volunteers according to the following procedure. Peripheral blood mononuclear cells (PBMC) were first isolated from 50 mL of fresh blood by Ficoll-Hypaque density gradient centrifugation, followed by CD4⁺ cell purification using a CD4⁺ T Cell Isolation Kit II (from Miltenyi Biotec Inc.). The CD4⁺ T cells were then differentiated to Th2 cells by culturing the cells in X-VIVO 15^{®} medium (from Cambrex BioScience Walkersville Inc.) containing 10% human AB serum (serum of blood type AB from Invitrogen Corporation), 50 U/mL of recombinant human interleukin-2 (rhIL-2) (from PeproTech Inc.) and 100 ng/mL of recombinant human interleukin-4 (rhIL-4) (from PeproTech Inc.) for 7 days. The Th2 cells were isolated using a CD294 (CRTH2) MicroBead Kit (from Miltenyi Biotec Inc.) and amplified in X-VIVO 15^{®} medium containing 10% human AB serum and 50 U/mL of rhIL-2 for 2 to 5 weeks. In general, 70% to 80% of the Th2 cells used in the assay are CRTH2-positive when analyzed by fluorescence-activated cell sorting using the BM16 antibody (as previously described) conjugated to phycoerythrin (PE).

To determine cellular inhibitory potency, compounds at various concentrations were incubated with 2.5 x 10⁴ Th2 cells and 500 nM DK-PGD₂ for 4 hrs at 37 °C in 200 µL of X-VIVO 15^{®} medium containing 10% human AB serum. IL-13 production to the medium was detected by ELISA (enzyme-linked immunosorbent assay) using an "Instant ELISA™" kit (from Bender MedSystems Inc.) according to the procedure suggested by the vendor. The spontaneous production of IL-13 by Th2 cells was determined in the absence of DK-PGD2 stimulation and the value was subtracted from that in the presence of each compound for percent inhibition and IC₅₀ calculations.

The percent inhibition of interleukin 13 (IL-13) production for a compound at various concentrations was calculated according to the following formula, [1-(IL-13 production in the presence of compound)/(IL-13 production in the presence of 0.15% DMSO)]x100. The IC₅₀ value, defined as the concentration of a compound that is required for 50% inhibition of IL-13 production, was calculated by fitting the percent inhibition data for 7 concentrations to a sigmoidal dose-response (4 parameter logistic) model in the XLfit^{®} software Excel add-in program [ID Business Solutions Ltd., model 205, where F(x) = (A+(B-A)/(1+((C/x)^D)))].

Representative compounds tested in the binding assay were tested using the foregoing DK-PGD₂-induced IL-13 production assay (examples 1-1 to 1-9, 2-1, 3-1 to 3-3, 4-1 to 4-3, 5-1, 6-1, 7-1, 8-1, 9-1 to 9-3, 10-2, 10-5, and 10-6). The results of the DK-PGD₂-induced IL-13 production assay showed that, with the exception of examples 1-8 and 1-9 (which exhibited IC₅₀ values greater than 10), the compounds tested in this assay exhibited activity in inhibiting IL-13 production, with IC₅₀ values ranging from 0.0032 µM to 6.428 µM.

Thus, the compounds of the present invention possess a specific, substantial and credible utility since the compounds tested show some activity in at least one of the above three assays (i.e., binding at the CRTH2 receptor), and therefore may be useful as antagonists in treating diseases and disorders associated with this receptor such as asthma.

### Example 50

### Human Thromboxane A2 Receptor Binding Assay

The thromboxane A2 receptor (TP) plays a key role in hemostasis as its abnormality leads to bleeding disorders. To avoid the potential liability of bleeding disorders, the binding activity of certain compounds of the present invention against TP was monitored by a receptor binding assay using human platelets as the source of the receptor and [³H]SQ29548 (generically named (5Z)-[5,6-³H]-7-[(1S,2R,3R,4R)-3-[[2-[(phenylamino)carbonyl]hydrazinyl]methyl]-7-oxabicyclo[2.2.1]hept-2-yl]-5-heptenoic acid, from PerkinElmer Inc.) as the competing radioactive ligand.

The TP binding reactions (0.2 mL) were performed in 96-well plates at room temperature in PBS containing 5 x 10⁷ platelets, 10 mM MgCl₂, 0.06% BSA, 10 nM [³H]SQ29548, and the test compound at various concentrations. After 1 hour of binding reactions, the platelets were harvested on GF/B filter plates (as previously described from PerkinElmer Inc.) and washed 5 times with PBS using a Filtermate™ Harvester (as previously described from PerkinElmer Inc.). The radioactivities bound to the platelets were determined using a microplate scintillation counter (TopCount^{®} NXT, from PerkinElmer Inc.) after adding 50 µL of Microscint™ 20 scintillation fluid (from PerkinElmer Inc.) to each well of the filter plates. The radioactivity from non-specific binding was determined by replacing the compound with 10 µM of ramatroban (BAY-u3405, from Cayman Chemical Company) in the reaction mixtures. The radioactivity bound to the platelets in the absence of compound (total binding) was determined by replacing the compound with 0.25% of DMSO in the reaction mixture. Specific binding data were obtained by subtracting the radioactivity of non-specific binding from each binding data.

The IC₅₀ value is defined as the concentration of the tested compound that is required for 50% inhibition of total specific binding. In order to calculate the IC₅₀ value, the percent inhibition data were determined for 7 concentrations for each compound. The percent inhibition for a compound at each concentration was calculated according to the following formula, [1-(specific binding in the presence of compound)/(total specific binding)]x100. The IC₅₀ value was then obtained by fitting the percent inhibition data to a sigmoidal dose-response (4 parameter logistic) model in the XLfit^{®} software Excel add-in program [from ID Business Solutions Ltd., model 205, where F(x) = (A+(B-A)/(1+((C/x)^D)))].

The results of the thromboxane A2 receptor binding assay are summarized in the following table:

| **Example No.** | **Thromboxane A2 Receptor Binding IC₅₀ (µM)** |
|---|---|
| 1 | 0.6417 |
| 46 | 3.6135 |

It is to be understood that the invention is not limited to the particular embodiments of the invention described above, as variations of the particular embodiments may be made and still fall within the scope of the appended claims.

## Claims

1. A compound of formula (I): 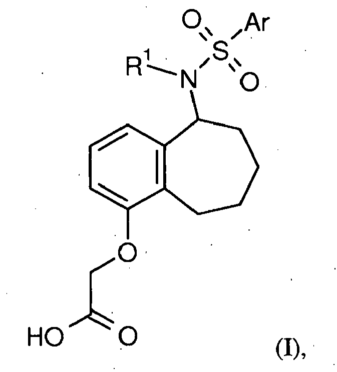 wherein:
Ar is: -phenyl, unsubstituted or mono- or bi-substituted independently with halogen, C₁₋₉ alkyl, -CF₃, -SO₂CH₃, alkoxy, -C(O)CH₃, unsubstituted heteroaryl or heteroaryl substituted with C₁₋₉ alkyl;
- biphenyl, unsubstituted or mono- or bi-substituted independently with -OH, C₁₋₉ alkyl, -SCH₃ or -SO₂CH₃; or
- pyridine, unsubstituted or substituted independently with unsubstituted phenyl or phenyl mono- or bi-substituted independently with C₁₋₉ alkyl, -CF₃ or -
CH₂CH₂OH; and
R¹ is hydrogen or C₁₋₉ alkyl,
or a pharmaceutically acceptable salt thereof.

2. The compound according to claim 1, wherein Ar is phenyl, unsubstituted or mono-or bi-substituted independently with fluorine, chlorine, bromine, -CH(CH₃)₂, -CF₃, - SO₂CH₃, -OCH₃, -C(O)CH₃ or -pyridine-methyl.

3. The compound according to claim 1, wherein Ar is biphenyl, unsubstituted or mono- or bi-substituted independently with -CH₃, -CH(CH₃)₂, -C(CH₃)₃, -OH, -SCH₃ or - SO₂CH₃.

4. The compound according to claim 1, wherein Ar is pyridine substituted independently with unsubstituted phenyl or phenyl mono- or bi-substituted independently with -CH₃, -CH(CH₃)₂, -C(CH₃)₃, -CF₃ or -CH₂CH₂OH.

5. The compound according to any one of claims 1 to 4, wherein R¹ is hydrogen.

6. The compound according to any one of claims 1 to 4, wherein R¹ is methyl.

7. The compound according to claim 1, wherein said compound is:
[5-(3,5-Bis-trifluoromethyl-benzenesulfonylamino)-6,7,8,9-tetrahydro-5H-benzocyclohepten-1-yloxy]-acetic acid;
[5-(3,5-Dichloro-benzenesulfonylamino)-6,7,8,9-tetrahydro-5H-benzocyclohepten-1-yloxy]-acetic acid;
[5-(Biphenyl-3-sulfonylamino)-6,7,8,9-tetrahydro-5H-benzocyclohepten-1-yloxy]-acetic acid;
[5-(Biphenyl-4-sulfonylamino)-6,7,8,9-tetrahydro-5H-benzocyclohepten-1-yloxy]-acetic acid;
[5-(3-Methanesulfonyl-benzenesulfonylamino)-6,7,8,9-tetrahydro-5H-benzocyclohepten-1-yloxy]-acetic acid;
[5-(3-Fluoro-5-trifluoromethyl-benzenesulfonylamino)-6,7,8,9-tetrahydro-5H-benzocyclohepten-1-yloxy]-acetic acid;
{5-[(3-Fluoro-5-trifluoromethyl-benzenesulfonyl)-methyl-amino]-6,7,8,9-tetrahydro-5H-benzocyclohepten-1-yloxy}-acetic acid;
[5-(3-Bromo-5-trifluoromethyl-benzenesulfonylamino)-6,7,8,9-tetrahydro-5H-benzocyclohepten-1-yloxy]-acetic acid;
{5-[(3-Bromo-5-trifluoromethyl-benzenesulfonyl)-methyl-amino]-6,7,8,9-tetrahydro-5H-benzocyclohepten-1-yloxy}-acetic acid;
[5-(3,5-Bis-methanesulfonyl-benzenesulfonylamino)-6,7,8,9-tetrahydro-5H-benzocyclohepten-1-yloxy] -acetic acid;
{5-[(3,5-Bis-methanesulfonyl-benzenesulfonyl)-methyl-amino]-6,7,8,9-tetrahydro-5H-benzocyclohepten-1-yloxy}-acetic acid;
{5-[(Biphenyl-4-sulfonyl)-methyl-amino]-6,7,8,9-tetrahydro-5H-benzocyclohepten-1-yloxy}-acetic acid;
[5-(3-Methoxy-5-trifluoromethyl-benzenesulfonylamino)-6,7,8,9-tetrahydro-5H-benzocyclohepten-1-yloxy]-acetic acid;
{5-[(3-Methoxy-5-trifluoromethyl-benzenesulfonyl)-methyl-amino]-6,7,8,9-tetrahydro-5H-benzocyclohepten-1-yloxy}-acetic acid;
[5-(3-Acetyl-5-trifluoromethyl-benzenesulfonylamino)-6,7,8,9-tetrahydro-5H-benzocyclohepten-1-yloxy] -acetic acid;
{5-[(3-Acetyl-5-trifluoromethyl-benzenesulfonyl)-methyl-amino]-6,7,8,9-tetrahydro-5H-benzocyclohepten-1-yloxy}-acetic acid;
[5-(3-Methanesulfonyl-5-trifluoromethyl-benzenesulfonylamino)-6,7,8,9-tetrahydro-5H-benzocyclohepten-1-yloxy]-acetic acid;
{5-[(3-Methanesulfonyl-5-trifluoromethyl-benzenesulfonyl)-methyl-amino]-6,7,8,9-tetrahydro-5H-benzocyclohepten-1-yloxy}-acetic acid;
[5-(3'-Isopropyl-biphenyl-4-sulfonylamino)-6,7,8,9-tetrahydro-5H-benzocyclohepten-1-yloxy]-acetic acid;
{5-[(3'-Isopropyl-biphenyl-4-sulfonyl)-methyl-amino]-6,7,8,9-tetrahydro-5H-benzocyclohepten-1-yloxy}-acetic acid;
[5-(3'-tert-Butyl-5'-methyl-biphenyl-4-sulfonylamino)-6,7,8,9-tetrahydro-5H-benzocyclohepten-1-yloxy]-acetic acid;
{5-[(3'-tert-Butyl-5`-methyl-biphenyl-4-sulfonyl)-methyl-amino]-6,7,8,9-tetrahydro-5H-benzocyclohepten-1-yloxy}-acetic acid;
[5-(4'-Hydroxy-biphenyl-4-sulfonylamino)-6,7,8,9-tetrahydro-5H-benzocyclohepten-1-yloxy]-acetic acid;
5-[(4'-Hydroxy-biphenyl-4-sulfonyl)-methyl-amino]-6,7,8,9-tetrahydro-5H-benzocyclohepten-1-yloxy}-acetic acid;
{5-[4-(5-Methyl-pyridin-3-yl)-benzenesulfonylamino]-6,7,8,9-tetrahydro-5H-benzocyclohepten-1-yloxy}-acetic acid;
(5-{Methyl-[4-(5-methyl-pyridin-3-yl)-benzenesulfonyl]-amino}-6,7,8,9-tetrahydro-5H-benzocyclohepten-1-yloxy)-acetic acid;
[5-(3'-Methylsulfanyl-biphenyl-4-sulfonylamino)-6,7,8,9-tetrahydro-5H-benzocyclohepten-1-yloxy]-acetic acid;
{5-[Methyl-(3'-methylsulfanyl-biphenyl-4-sulfonyl)-amino]-6,7,8,9-tetrahydro-5H-benzocyclohepten-1-yloxy}-acetic acid;
[5-(3'-Methanesulfonyl-biphenyl-4-sulfonylamino)-6,7,8,9-tetrahydro-5H-benzocyclohepten-1-yloxy]-acetic acid;
{5-[(3'-Methanesulfonyl-biphenyl-4-sulfonyl)-methyl-amino]-6,7,8,9-tetrahydro-5H-benzocyclohepten-1-yloxy}-acetic acid;
{5-[5-(3-Isopropyl-phenyl)-pyridine-2-sulfonylamino]-6,7,8,9-tetrahydro-5H-benzocyclohepten-1-yloxy}-acetic acid;
(5-{[5-(3-Isopropyl-phenyl)-pyridine-2-sulfonyl]-methyl-amino}-6,7,8,9-tetrahydro-5H-benzocyclohepten-1-yloxy)-acetic acid;
{5-[5-(3-Trifluoromethyl-phenyl)-pyridine-2-sulfonylamino]-6,7,8,9-tetrahydro-5H-benzocyclohepten-1-yloxy}-acetic acid;
(5-{Methyl-[5-(3-trifluoromethyl-phenyl)-pyridine-2-sulfonyl]-amino}-6,7,8,9-tetrahydro-5H-benzocyclohepten-1-yloxy)-acetic acid;
{5-[5-(3-tert-Butyl-5-methyl-phenyl)-pyndine-2-sulfonylamino]-6,7,8,9-tetrahydro-5H-benzocycloheptem-1-yloxy}-acetic acid;
(5-{[5-(3-tert-Butyl-5-methyl-phenyl)-pyridine-2-sulfonyl]-methyl-amino}-6,7,8,9-tetrahydro-5H-benzocyclohepten-1-yloxy)-acetic acid;
(5-{5-[3-(2-Hydroxy-ethyl)-phenyl]-pyridine-2-sulfonylamino}-6,7,8,9-tetrahydro-5H-benzocyclohepten-1-yloxy)-acetic acid;
[5-({5-[3-(2-Hydroxy-ethyl)-phenyl]-pyridine-2-sulfonyl}-methyl-amino)-6,7,8,9-tetrahydro-5H-benzocyclohepten-1-yloxy]-acetic acid;
[5-(4'-Methyl-biphenyl-3-sulfonylamino)-6,7,8,9-tetrahydro-5H-benzocyclohepten-1-yloxy]-acetic acid;
{5-[Methyl-(4'-methyl-biphenyl-3-sulfonyl)-amino]-6,7,8,9-tetrahydro-5H-benzocyclohepten-1-yloxy}-acetic acid;
[5-(3'-Isopropyl-biphenyl-3-sulfonylamino)-6,7,8,9-tetrahydro-5H-benzocyclohepten-1-yloxy]-acetic acid;
{5-[(3'-Isopropyl-biphenyl-3-sulfonyl)-methyl-amino]-6,7,8,9-tetrahydro-5H-benzocyclohepten-1-yloxy}-acetic acid;
[5-(3-Isopropyl-5-trifluoromethyl-benzenesulfonylamino)-6,7,8,9-tetrahydro-5H-benzocyclohepten-1-yloxy]-acetic acid; or
{5-[(3-Isopropyl-5-trifluoromethyl-benzenesulfonyl)-methyl-amino]-6,7,8,9-tetrahydro-5H-benzocyclohepten-1-yloxy}-acetic acid.

8. A compound according to any one of claims 1 to 7 for use as a therapeutically active substance.

9. A pharmaceutical composition, comprising a therapeutically effective amount of a compound in accordance with any one of claims 1 to 7 and a therapeutically inert carrier.

10. The use of a compound according to any one of claims 1 to 7 for the preparation of a medicament for the treatment or prophylaxis of asthma or COPD.

11. A compound according to any one of claims 1 to 7 for the treatment or prophylaxis of asthma or COPD.

## Patentansprüche

1. Eine Verbindung der Formel (I): 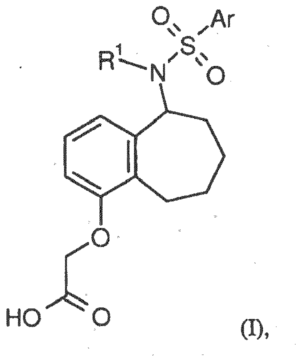 wobei:
Ar: - Phenyl, unsubstituiert oder unabhängig mono- oder disubstituiert mit Halogen, C₁₋₉-Alkyl, -CF₃, -SO₂CH₃, Alkoxy, -C(O)CH₃, unsubstituiertem Heteroaryl oder Heteroaryl, substituiert mit C₁₋₉-Alkyl;
- Biphenyl, unsubstituiert oder unabhängig mono- oder disubstituiert mit -OH, C₁₋₉-Alkyl, -SCH₃ oder -SO₂CH₃; oder
- Pyridin, unsubstituiert oder unabhängig substituiert mit unsubstituiertem Phenyl oder Phenyl, unabhängig mono- oder disubstituiert mit C₁₋₉-Alkyl, -CF₃ oder -CH₂CH₂OH, ist; und
R¹ Wasserstoff oder C₁₋₉-Alkyl ist,
oder ein pharmazeutisch verträgliches Salz davon.

2. Die Verbindung nach Anspruch 1, wobei Ar Phenyl, unsubstituiert oder unabhängig mono- oder disubstituiert mit Fluor, Chlor, Brom, -CH(CH₃)₂, -CF₃, -SO₂CH₃, -OCH₃, -C(O)CH₃ oder -Pyridin-methyl, ist.

3. Die Verbindung nach Anspruch 1, wobei Ar Biphenyl, unsubstituiert oder unabhängig mono- oder disubstituiert mit -CH₃, -CH(CH₃)₂, -C(CH₃)₃, -OH, -SCH₃ oder -SO₂CH₃, ist.

4. Die Verbindung nach Anspruch 1, wobei Ar Pyridin, unabhängig substituiert mit unsubstituiertem Phenyl oder Phenyl, unabhängig mono- oder disubstituiert mit -CH₃, -CH(CH₃)₂, -C(CH₃)₃, -CF₃ oder -CH₂CH₂OH, ist.

5. Die Verbindung nach einem der Ansprüche 1 bis 4, wobei R¹ Wasserstoff ist.

6. Die Verbindung nach einem der Ansprüche 1 bis 4, wobei R¹ Methyl ist.

7. Die Verbindung nach Anspruch 1, wobei die Verbindung:
[5-(3,5-Bis-trifluormethyl-benzolsulfonylamino)-6,7,8,9-tetrahydro-5H-benzocyclohepten-1-yloxy]-essigsäure;
[5-(3,5-Dichlor-benzolsulfonylamino)-6,7,8,9-tetrahydro-5H-benzocyclohepten-1-yloxy]-essigsäure;
[5-(Biphenyl-3-sulfonylamino)-6,7,8,9-tetrahydro-5H-benzocyclohepten-1-yloxy]-essigsäure;
[5-(Biphenyl-4-sulfonylamino)-6,7,8,9-tetrahydro-5H-benzocyclohepten-1-yloxy]-essigsäure;
[5-(3-Methansulfonyl-benzolsulfonylamino)-6,7,8,9-tetrahydro-5H-benzocyclohepten-1-yloxy]-essigsäure;
[5-(3-Fluor-5-trifluormethyl-benzolsulfonylamino)-6,7,8,9-tetrahydro-5H-benzocyclohepten-1-yloxy]-essigsäure;
{5-[(3-Fluor-5-trifluormethyl-benzolsulfonyl)-methyl-amino]-6,7,8,9-tetrahydro-5H-benzocyclohepten-1-yloxy}-essigsäure;
[5-(3-Brom-5-trifluormethyl-benzolsulfonylamino)-6,7,8,9-tetrahydro-5H-benzocyclohepten-1-yloxy]-essigsäure;
{5-[(3-Brom-5-trifluormethyl-benzolsulfonyl)-methyl-amino]-6,7,8,9-tetrahydro-5H-benzocyclohepten-1-yloxy}-essigsäure;
[5-(3,5-Bis-methansulfonyl-benzolsulfonylamino)-6,7,8,9-tetrahydro-5H-benzocyclohepten-1-yloxy]-essigsäure;
{5-[(3,5-Bis-methansulfonyl-benzolsulfonyl)-methyl-amino]-6,7,8,9-tetrahydro-5H-benzocyclohepten-1-yloxy}-essigsäure;
{5-[(Biphenyl-4-sulfonyl)-methyl-amino]-6,7,8,9-tetrahydro-5H-benzocyclohepten-1-yloxy}essigsäure;
[5-(3-Methoxy-5-trifluormethyl-benzolsulfonylamino)-6,7,8,9-tetrahydro-5H-benzocyclohepten-1-yloxy]-essigsäure;
{5-[(3-Methoxy-5-trifluormethyl-benzolsulfonyl)-methyl-amino]-6,7,8,9-tetrahydro-5H-benzocyclohepten-1-yloxy)-essigsäure;
[5-(3-Acetyl-5-trifluormethyl-benzolsulfonylamino)-6,7,8,9-tetrahydro-5H-benzocyclohepten-1-yloxy]-essigsäure;
{5-[(3-Acetyl-5-trifluormethyl-benzolsulfonyl)-methyl-amino]-6,7,8,9-tetrahydro-5H-benzocyclohepten-1-yloxy}-essigsäure;
[5-(3-Methansulfonyl-5-trifluormethyl-benzolsulfonylamino)-6,7,8,9-tetrahydro-5H-benzocyclohepten-1-yloxy]-essigsäure;
{5-[(3-Methansulfonyl-5-trifluormethyl-benzolsulfonyl)-methyl-amino]-6,7,8,9-tetrahydro-5H-benzocyclohepten-1-yloxy}-essigsäure;
[5-(3'-Isopropyl-biphenyl-4-sulfonylamino)-6,7,8,9-tetrahydro-5H-benzocyclohepten-1-yloxy]-essigsäure;
{5-[(3'-Isopropyl-biphenyl-4-sulfonyl)-methyl-amino]-6,7,8,9-tetrahydro-5H-benzocyclohepten-1-yloxy}-essigsäure;
[5-(3'-tert-Butyl-5'-methyl-biphenyl-4-sulfonylamino)-6,7,8,9-tetrahydro-5H-benzocyclohepten-1-yloxy]-essigsäure;
{5-[(3'-tert-Butyl-5'-methyl-biphenyl-4-sulfonyl)-methyl-amino]-6,7,8,9-tetrahydro-5H-benzocyclohepten-1-yloxy}-essigsäure;
[5-(4'-Hydroxy-biphenyl-4-sulfonylamino)-6,7,8,9-tetrahydro-5H-benzocyclohepten-1-yloxy]-essigsäure;
{5-[(4'-Hydroxy-biphenyl-4-sulfonyl)-methyl-amino]-6,7,8,9-tetrahydro-5H-benzocyclohepten-1-yloxy}-essigsäure;
{5-[4-(5-Methyl-pyridin-3-yl)-benzolsulfonylamino]-6,7,8,9-tetrahydro-5H-benzocyclohepten-1-yloxy}-essigsäure;
(5-{Methyl-[4-(5-methyl-pyridin-3-yl)-benzolsulfonyl]-amino}-6,7,8,9-tetrahydro-5H-benzocyclohepten-1-yloxy)-essigsäure;
[5-(3'-Methylsulfanyl-biphenyl-4-sulfonylamino)-6,7,8,9-tetrahydro-5H-benzocyclohepten-1-yloxy]-essigsäure;
{5-[Methyl-(3'-methylsulfanyl-biphenyl-4-sulfonyl)-amino]-6,7,8,9-tetrahydro-5H-benzocyclohepten-1-yloxy}-essigsäure;
[5-(3'-Methansulfonyl-biphenyl-4-sulfonylamino)-6,7,8,9-tetrahydro-5H-benzocyclohepten-1-yloxy]-essigsäure;
{5-[(3'-Methansulfonyl-biphenyl-4-sulfonyl)-methyl-amino]-6,7,8,9-tetrahydro-5H-benzocyclohepten-1-yloxy}-essigsaure;
{5-[5-(3-Isopropyl-phenyl)-pyridin-2-sulfonylamino]-6,7,8,9-tetrahydro-5H-benzocyclohepten-1-yloxy}-essigsäure;
(5-{[5-(3-Isopropyl-phenyl)-pyridin-2-sulfonyl]-methyl-amino}-6,7,8,9-tetrahydro-5H-benzocyclohepten-1-yloxy)-essigsäure;
{5-[5-(3-Trifluormethyl-phenyl)-pyridin-2-sulfonylamino]-6,7,8,9-tetrahydro-5H-benzocyclohepten-1-yloxy}-essigsäure;
(5-{Methyl-[5-(3-trifluormethyl-phenyl)-pyridin-2-sulfonyl]-amino}-6,7,8,9-tetrahydro-5H-benzocyclohepten-1-yloxy)-essigsäure;
{5-[5-(3-tert-Butyl-5-methyl-phenyl)-pyridin-2-sulfonylamino]-6,7,8,9-tetrahydro-5H-benzocyclohepten-1-yloxy}-essigsäure;
(5-{[5-(3-tert-Butyl-5-methyl-phenyl)-pyridin-2-sulfonyl]-methyl-amino}-6,7,8,9-tetrahydro-5H-benzocyclohepten-1-yloxy)-essigsäure;
(5-{5-[3-(2-Hydroxy-ethyl)-phenyl]-pyridin-2-sulfonylamino}-6,7,8,9-tetrahydro-5H-benzocyclohepten-1-yloxy)-essigsäure;
[5-({5-[3-(2-Hydroxy-ethyl)-phenyl]-pyridin-2-sulfonyl}-methyl-amino)-6,7,8,9-tetrahydro-5H-benzocyclohepten-1-yloxy]-essigsäure;
[5-(4'-Methyl-biphenyl-3-sulfonylamino)-6,7,8,9-tetrahydro-5H-benzocyclohepten-1-yloxy]-essigsäure;
{5-[Methyl-(4'-methyl-biphenyl-3-sulfony)-amino]-6,7,8,9-tetrahydro-5H-benzocyclohepten-1-yloxy}-essigsäure;
[5-(3'-Isopropyl-biphenyl-3-sulfonylamino)-6,7,8,9-tetrahydro-5H-benzocyclohepten-1-yloxy]-essigsäure;
{5-[(3'-Isopropyl-biphenyl-3-sulfonyl)-methyl-amino]-6,7,8,9-tetrahydro-5H-benzocyclohepten-1-yloxy}-essigsäure;
[5-(3-Isopropyl-5-trifluormethyl-benzolsulfonylamino)-6,7,8,9-tetrahydro-5H-benzocyclohepten-1-yloxy]-essigsäure; oder
{5-[(3-Isopropyl-5-trifluormethyl-benzolsulfonyl)-methyl-amino]-6,7,8,9-tetrahydro-5H-benzocyclohepten-1-yloxy}-essigsäure ist.

8. Eine Verbindung nach einem der Ansprüche 1 bis 7 zur Verwendung als ein therapeutischer Wirkstoff.

9. Ein Arzneimittel, umfassend eine therapeutisch wirksame Menge einer Verbindung nach einem der Ansprüche 1 bis 7 und einen therapeutisch inerten Träger.

10. Die Verwendung einer Verbindung nach einem der Ansprüche 1 bis 7 zur Herstellung eines Medikaments zur Behandlung oder Prophylaxe von Asthma oder COPD.

11. Eine Verbindung nach einem der Ansprüche 1 bis 7 zur Behandlung oder Prophylaxe von Asthma oder COPD.

## Revendications

1. Composé de formule (I) : 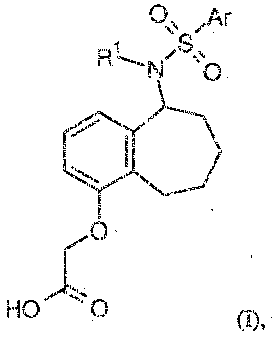 dans laquelle :
Ar représente un groupe :
- phényle, non substitué ou mono- ou bi-substitué indépendamment avec un substituant halogéno, alkyle en C₁ à C₉, -CF₃, -SO₂CH₃, alkoxy, -C(O)CH₃, hétéroaryle non substitué ou hétéroaryle substitué avec un substituant alkyle en C₁ à C₉ ;
- biphényle, non substitué ou mono- ou bi-substitué indépendamment avec un substituant -OH, alkyle en C₁ à C₉, -SCH₃ ou -SO₂CH₃ ; ou
- pyridine, non substitué ou substitué indépendamment avec un substituant phényle non substitué ou phényle mono- ou bi-substitué indépendamment avec un substituant alkyle en C₁ à C₉, -CF₃ ou -CH₂CH₂OH ; et
R¹ représente un atome d'hydrogène ou un groupe alkyle en C₁ à C₉,
ou un de ses sels pharmaceutiquement acceptables.

2. Composé suivant la revendication 1, dans lequel Ar représente un groupe phényle, non substitué ou mono- ou bi-substitué indépendamment avec un substituant fluoro, chloro, bromo, -CH(CH₃)₂, -CF₃, -SO₂CH₃, -OCH₃, -C(O)CH₃ ou pyridine-méthyle.

3. Composé suivant la revendication 1, dans lequel Ar représente un groupe biphényle, non substitué ou mono-ou bi-substitué indépendamment avec un substituant -CH₃, -CH(CH₃)₂, -C(CH₃)₃, -OH, -SCH₃ ou -SO₂CH₃.

4. Composé suivant la revendication 1, dans lequel Ar représente un groupe pyridine substitué indépendamment avec un substituant phényle non substitué ou phényle mono-ou bi-substitué indépendamment avec un substituant -CH₃, -CH(CH₃)₂, -C(CH₃)₃, -CF₃ ou -CH₂CH₂OH.

5. Composé suivant l'une quelconque des revendications 1 à 4, dans lequel R¹ représente un atome d'hydrogène.

6. Composé suivant l'une quelconque des revendications 1 à 4, dans lequel R¹ représente un groupe méthyle.

7. Composé suivant la revendication 1, ledit composé étant :
l'acide [5-(3,5-bis-trifluorométhyl-benzènesulfonylamino)-6,7,8,9-tétrahydro-5H-benzocycloheptène-1-yloxy]-acétique ;
l'acide [5-(3,5-dichlorobenzènesulfonylamino)-6,7,8,9-tétrahydro-5H-benzocyclohepténe-1-yloxy]-acêtique ;
l'acide [5-(biphényl-3-sulfonylamino)-6,7,8,9-tétrahydro-5H-benzocycloheptène-1-yloxy]-acétique ;
l'acide [5-(biphényl-4-sulfonylamino)-6,7,8,9-tétrahydro-5H-benzocycloheptène-1-yloxy]-acétique ;
l'acide [5-(3-méthanesulfonyl-benzènesulfonylamino)-6,7,8,9-têtrahydro-5H-benzocycloheptène-1-yloxy]-acétique ;
l'acide [5-(3-fluoro-5-trifluorométhyl-benzènesulfonyl-amino)-6,7,8,9-tétrahydro-5H-benzocycloheptène-1-yloxy]-acétique ;
l'acide {5-[(3-fluoro-5-trifluorométhyl-benzènesulfonyl)-méthyl-amino]-6,7,8,9-tétrahydro-5H-benzocycloheptène-1-yl-oxy}-acétique ;
l'acide [5-(3-bromo-5-trifluorométhyl-benzènesulfonyl-amino)-6,7,8,9-tétrahydro-5H-benzocycloheptène-1-yloxy]-acétique ;
l'acide {5-[(3-bromo-5-trifluorométhyl-benzènesulfonyl)-méthyl-amino]-6,7,8,9-tétrahydro-5H-benzocycloheptène-1-yl-oxy}-acétique ;
l'acide [5-(3,5-bis-méthanesulfonyl-benzènesulfonyl-amino)-6,7,8,9-tétrahydro-5H-benzocycloheptène-1-yloxyl-acétique ;
l'acide {5-[(3,5-bis-méthanesulfonyl-benzènesulfonyl)-méthyl-amino]-6,7,8,9-tétrahydro-5H-benzocycloheptène-1-yl-oxy}-acétique ;
l'acide (5-[(biphényl-4-sulfonyl)-méthyl-amino]-6,7,8,9-tétrahydro-5H-benzocycloheptène-1-yloxyl-acétique ;
l'acide [5-(3-méthoxy-5-trifluorométhyl-benzènesulfonyl-amino)-6,7,8,9-tétrahydro-5H-benzocycloheptène-1-yloxy]-acétique ;
l'acide {5-[(3-méthoxy-5-trifluorométhyl-benzènesulfonyl)-méthyl-amino]-6,7,8,9-tétrahydro-5H-benzocycloheptène-1-yl-oxy}-acétique ;
l'acide [5-(3-acétyl-5-trifluorométhyl-benzènesulfonyl-amino)-6,7,8,9-tétrahydro-5H-benzocycloheptène-1-yloxyl-acétique ;
l'acide {5-[(3-acétyl-5-trifluorométhyl-benzènesulfonyl)-méthyl-amino]-6,7,8,9-tétrahydro-5H-benzocycloheptène-1-yl-oxy}-acétique ;
l'acide [5-(3-méthanesulfonyl-5-trifluorométhyl-benzène-sulfonylamino)-6,7,8,9-tétrahydro-5H-benzocycloheptène-1-yloxy]-acétique ;
l'acide {5-[(3-méthanesulfonyl-5-trifluorométhyl-benzène-sulfonyl)-méthyl-amino]-6,7,8,9-tétrahydro-5H-benzocycloheptène-1-yloxy}-acétique ;
l'acide [5-(3'-isopropyl-biphényl-4-sulfonylamino)-6,7,8,9-tétrahydro-5H-benzocycloheptène-1-yloxy]-acétique ;
l'acide {5-[(3'-isopropyl-biphényl-4-sulfonyl)-méthyl-amino]-6,7,8,9-tétrahydro-5H-benzocycloheptène-1-yloxy}-acétique ;
l'acide [5-(3'-tertiobutyl-S'-méthyl-biphényl-4-sulfonyl-amino)-6,7,8,9-tétrahydro-5H-benzocycloheptène-1-yloxy]-acétique ;
l'acide {5-[(3'-tertiobutyl-5'-méthyl-biphényl-4-sulfonyl)-méthyl-amino]-6,7,8,9-tétrahydro-5H-benzocycloheptène-1-yl-oxy}-acétique ;
l'acide [5-(4'-hydroxy-biphényl-4-sulfonylamino)-6,7,8,9-tétrahydro-5H-benzocycloheptène-1-yloxy]-acétique ;
l'acide {5-[(4'-hydroxy-biphényl-4-sulfonyl)-méthyl-amino]-6,7,8,9-tétrahydro-5H-benzocycloheptène-1-yloxy}-acétique ;
l'acide {5-[4-(5-méthyl-pyridine-3-yl)-benzènesulfonyl-aminol-6,7,8,9-tétrahydro-5H-benzocycloheptène-1-yloxy}-acétique ;
l'acide (5-{méthyl-[4-(5-méthyl-pyridine-3-yl)-benzène-sulfonyl]-amino}-6,7,8,9-tétrahydro-5H-benzocycloheptène-1-yloxy)-acétique ;
l'acide [5-(3'-méthylsulfanyl-biphényl-4-sulfonylamino)-6,7,8,9-tétrahydro-5H-benzocycloheptène-1-yloxy]-acétique ;
l'acide {5-[méthyl-(3'-méthylsulfanyl-biphényl-4-sulfonyl)-amino]-6,7,8,9-tétrahydro-5H-benzocycloheptène-1-yloxy}-acétique ;
l'acide [5-(3'-méthanesulfonyl-biphényl-4-sulfonylamino)-6,7,8,9-tétrahydro-5H-benzocycloheptène-1-yloxy]-acétique ;
l'acide {5-[(3'-méthanesulfonyl-biphényl-4-sulfonyl)-méthyl-amino]-6,7,8,9-tétrahydro-5H-benzocycloheptène-1-yloxy}-acétique ;
l'acide {5-[5-(3-isopropyl-phényl)-pyridine-2-sulfonylaminol-6,7,8,9-tétrahydro-5H-benzocycloheptène-1-yloxy}-acétique ;
l'acide (5-{[5-(3-isopropyl-phényl)-pyridine-2-sulfonyl]-méthyl-amino}-6,7,8,9-tétrahydro-5H-benzocycloheptène-1-yloxy)-acétique ;
l'acide {5-[5-(3-trifluorométhyl-phényl)-pyridine-2-sulfonylamino]-6,7,8,9-têtrahydro-5H-benzocycloheptène-1-yloxy}-acétique ;
l'acide (5-{méthyl-[5-(3-trifluorométhyl-phényl)-pyridine-2-sulfonyl]-amino}-6,7,8,9-tétrahydro-5H-benzocycloheptène-1-yloxy)-acétique ;
l'acide {5-[5-(3-tertiobutyl-5-méthyl-phényl)-pyridine-2-sulfonylamino]-6,7,8,9-tétrahydro-5H-benzocycloheptène-1-yloxy}-acétique ;
l'acide (5-{[5-(3-tertiobutyl-5-méthyl-phényl)-pyridine-2-sulfonyl]-méthyl-amino}-6,7,8,9-tétrahydro-5H-benzocycloheptène-1-yloxy)-acétique ;
l'acide (5-{5-[3-(2-hydroxy-éthyl)-phényl]-pyridine-2-sulfonylamino}-6,7,8,9-tétrahydro-5H-benzocycloheptène-1-yloxy)-acétique ;
l'acide [5-({5-[3-(2-hydroxy-éthyl)-phényl]-pyridine-2-sulfonyl}-méthyl-amino)-6,7,8,9-tétrahydro-5H-benzocyclo-heptène-1-yloxy]-acétique ;
l'acide [5-(4'-méthyl-biphényl-3-sulfonylamino)-6,7,8,9-tétrahydro-5H-benzocycloheptène-1-yloxy]-acétique ;
l'acide {5-[méthyl-(4'-méthyl-biphényl-3-sulfonyl)-amino]-6,7,8,9-tétrahydro-5H-benzocycloheptène-1-yloxy}-acétique ;
l'acide [5-(3'-isopropyl-biphényl-3-sulfonylamino)-6,7,8,9-tétrahydro-5H-benzocycloheptène-1-yloxy]-acétique ;
l'acide {5-[(3'-isopropyl-biphényl-3-sulfonyl)-méthyl-amino]-6,7,8,9-tétrahydro-5H-benzocycloheptène-1-yloxy}-acétique ;
l'acide [5-(3-isopropyl-5-trifluorométhyl-benzènesulfonylamino)-6,7,8,9-tétrahydro-5H-benzocycloheptène-1-yloxy]-acétique ; ou
l'acide {5-[(3-isopropyl-5-trifluorométhyl-benzène-sulfonyl)-méthyl-amino]-6,7,8,9-tétrahydro-5H-benzocyclo-heptène-1-yloxy}-acétique.

8. Composé suivant l'une quelconque des revendications 1 à 7, pour une utilisation comme substance thérapeutiquement active.

9. Composition pharmaceutique, comprenant une quantité thérapeutiquement efficace d'un composé suivant l'une quelconque des revendications 1 à 7 et un support thérapeutiquement inerte.

10. Utilisation d'un composé suivant l'une quelconque des revendications 1 à 7, pour la préparation d'un médicament pour le traitement ou la prophylaxie de l'asthme ou de la maladie pulmonaire obstructive chronique (COPD).

11. Composé suivant l'une quelconque des revendications 1 à 7, pour le traitement ou la prophylaxie de l'asthme ou de la COPD.
